# EUROPEAN PATENT SPECIFICATION

(11) **EP 1 611 093 B1**
(45) Date of publication and mention of the grant of the patent: **15.07.2015**
(21) Application number: 03749159.4
(22) Date of filing: 28.08.2003
(51) Int. Cl.: A61K 38/40, A61K 38/16, A61K 38/41, C07K 14/79

(54) **MODIFIED TRANSFERRIN FUSION PROTEINS**
FUSIONSPROTEINE MIT MODIFIZIERTEM TRANSFERRIN
PROTEINES DE FUSION DE TRANSFERRINE MODIFIEES

(30) Priority: 30.08.2002 US 406977 P; 04.03.2003 US 378094
(43) Date of publication of application: 04.01.2006
(73) Proprietor: Biorexis Pharmaceutical Corporation, New York, NY 10017-5755 (US)
(72) Inventor: PRIOR, Christopher, P., King of Prussia, Pa 19406-2675 (US); LAI, Char-Huei, King of Prussia, PA 19406-2675 (US); SADEGHI, Homayoun, Philadelphia, PA 19406-2675 (US); TURNER, Andrew, J., Philadelphia, PA 19406-2675 (US)
(74) Representative: Cripps, Joanna Elizabeth
(86) International application number: PCT/US2003/026818
(87) International publication number: WO 2004/020405

(56) References cited:
- WO-A-03/020746
- WO-A-2004/020404
- WO-A-2004/020454
- WO-A-2004/020588
- WO-A2-02/46227
- US-A- 5 817 789
- US-A- 5 986 067
- US-A- 6 027 921
- US-B1- 6 262 026
- ALI S A ET AL: "Transferrin trojan horses as a rational approach for the biological delivery of therapeutic peptide domains" JOURNAL OF BIOLOGICAL CHEMISTRY, AMERICAN SOCIETY OF BIOLOCHEMICAL BIOLOGISTS, BIRMINGHAM,, US, vol. 274, no. 34, 20 August 1999 (1999-08-20), pages 24066-24073, XP008052346 ISSN: 0021-9258
- PARK E ET AL: "Production and characterization of fusion proteins containing transferrin and nerve growth factor" JOURNAL OF DRUG TARGETING, HARWOOD ACADEMIC PUBLISHERS GMBH, DE, vol. 6, no. 1, 1998, pages 53-64, XP002960815 ISSN: 1061-186X
- PARISE F ET AL: "Construction and in vitro functional evaluation of a low-density lipoprotein receptor/transferrin fusion protein as a therapeutic tool for familial hypercholesterolemia" HUMAN GENE THERAPY, MARY ANN LIEBERT, NEW YORK ,NY, US, vol. 10, no. 7, 1 May 1999 (1999-05-01), pages 1219-1228, XP009057500 ISSN: 1043-0342
- WARD P.P. ET AL: 'A system for production of commercial quantities of human lactoferrin: a broad spectrum natural antibiotic' BIOTECHNOLOGY vol. 13, May 1995, NEW YORK, pages 498 - 503, XP002048442
- SALMON V. ET AL: 'Production of human lactoferrin in transgenictobacco plants' PROTEIN EXPR PURIF. vol. 13, June 1998, pages 127 - 135, XP000863470
- ADRIAN G.S. ET AL: 'Human transferrin: Expression and iron modulation of chimeric genes in transgenic mice' J. BIOL. CHEM. vol. 265, no. 22, 05 August 1990, pages 13344 - 13350, XP002988943

## Description

### FIELD OF THE INVENTION

The present invention relates to therapeutic proteins or peptides with extended serum stability or *in vivo* circulatory half-life fused to or inserted in a transferrin molecule modified to reduce or inhibit glycosylation, iron binding and/or transferrin receptor binding. Specifically, the present invention includes GLP-1 fused to or inserted in a modified transferrin molecule.

### BACKGROUND OF THE INVENTION

Therapeutic proteins or peptides in their native state or when recombinantly produced are typically labile molecules exhibiting short periods of serum stability or short *in vivo* circulatory half-lives. In addition, these molecules are often extremely labile when formulated, particularly when formulated in aqueous solutions for diagnostic and therapeutic purposes.

Few practical solutions exist to extend or promote the stability *in vivo* or *in vitro* of proteinaceous therapeutic molecules. Polyethylene glycol (PEG) is a substance that can be attached to a protein, resulting in longer-acting, sustained activity of the protein. If the activity of a protein is prolonged by the attachment to PEG, the frequency that the protein needs to be administered may be decreased. PEG attachment, however, often decreases or destroys the protein's therapeutic activity. While in some instance PEG attachment can reduce immunogenicity of the protein, in other instances it may increase immunogenicity.

Therapeutic proteins or peptides have also been stabilized by fusion to a protein capable of extending the *in vivo* circulatory half-life of the therapeutic protein. For instance, therapeutic proteins fused to albumin or to antibody fragments may exhibit extended *in vivo* circulatory half-life when compared to the therapeutic protein in the unfused state. See U.S. Patents 5,876,969 and 5,766,883.

Another serum protein, glycosylated human transferrin (Tf) has also been used to make fusions with therapeutic proteins to target delivery to the interior of cells or to carry agents across the blood-brain barrier. These fusion proteins comprising glycosylated human Tf have been used to target nerve growth factor (NGF) or ciliary neurotrophic factor (CNTF) across the blood-brain barrier by fusing full-length Tf to the agent. See U.S. Patents 5,672,683 and 5977,307. In these fusion proteins, the Tf portion of the molecule is glycosylated and binds to two atoms of iron, which is required for Tf binding to its receptor on a cell and, according to the inventors of these patents, to target delivery of the NGF or CNTF moiety across the blood-brain barrier. Transferrin fusion proteins have also been produced by inserting an HIV-1 protease target sequence into surface exposed loops of glycosylated transferrin to investigate the ability to produce another form of Tf fusion for targeted delivery to the inside of a cell via the Tf receptor (Ali et al. (1999) J. Biol. Chem. 274(34):24066-24073).

Serum transferrin (Tf) is a monomeric glycoprotein with a molecular weight of 80,000 daltons that binds iron in the circulation and transports it to various tissues via the transferrin receptor (TfR) (Aisen et al. (1980) Ann. Rev. Biochem. 49: 357-393; MacGillivray et al. (1981) J. Biol. Chem. 258: 3543-3553, U.S. Patent 5,026,651). Tf is one of the most common serum molecules, comprising up to about 5-10% of total serum proteins. Carbohydrate deficient transferrin occurs in elevated levels in the blood of alcoholic individuals and exhibits a longer half life (approximately 14-17 days) than that of glycosylated transferrin (approximately 7-10 days). See van Eijk et al. (1983) Clin. Chim. Acta 132:167-171, Stibler (1991) Clin. Chem. 37:2029-2037 (1991), Arndt (2001) Clin. Chem. 47(1):13-27 and Stibler et al. in "Carbohydrate-deficient consumption", Advances in the Biosciences, (Ed Nordmann et al.), Pergamon, 1988, Vol. 71, pages 353-357).

The structure of Tf has been well characterized and the mechanism of receptor binding, iron binding and release and carbonate ion binding have been elucidated (U.S. Patents 5,026,651, 5,986,067 and MacGillivray et al. (1983) J. Biol. Chem. 258(6):3543-3546).

Transferrin and antibodies that bind the transferrin receptor have also been used to deliver or carry toxic agents to tumor cells as cancer therapy (Baselga and Mendelsohn, 1994), and transferrin has been used as a non-viral gene therapy vector to deliver DNA to cells (Frank *et al.,* 1994; Wagner *et al.,* 1992). The ability to deliver proteins to the central nervous system (CNS) using the transferrin receptor as the entry point has been demonstrated with several proteins and peptides including CD4 (Walus *et al.,* 1996), brain derived neurotrophic factor (Pardridge *et al.,* 1994), glial derived neurotrophic factor (Albeck *et al*.), a vasointestinal peptide analogue (Bickel *et al.,* 1993), a beta-amyloid peptide (Saito *et al.,* 1995), and an antisense oligonucleotide (Pardridge *et al.,* 1995).

Transferrin fusion proteins have not, however, been modified or engineered to extend the *in vivo circulatory* half-life of a therapeutic protein nor peptide or to increase bioavailability by reducing or inhibiting glycosylation of the Tf moiety nor to reduce or prevent iron and/or Tf receptor binding.

### SUMMARY OF THE INVENTION

As described in more detail below, the present invention includes modified Tf fusion proteins comprising glucagon-like peptide (GLP-1) , wherein the Tf portion is engineered to extend the *in vivo* circulatory half-life or bioavailability of the molecule. The invention also includes pharmaceutical formulations and compositions comprising the fusion proteins and nucleic acid molecules encoding the modified Tf fusion proteins. The application further describes methods of extending the serum stability, in vivo circulating half-life and bioavailibility of GLP-1 by fusion to a medified transferrin. Also described herein are methods of treating a patient with a modified Tf fusion protein.

Preferably, the modified Tf fusion proteins comprise a human transferrin Tf moiety that has been modified to reduce or prevent glycosylation and/or iron and receptor binding.

The present invention provides fusion proteins comprising GLP-1 fused to or inserted into modified transferrin molecules. Other therapeutic proteins described herein include β-interferon (β-IFN), EPO (erythropoietin) mimetic peptide (EMP1), and T-20.

Also described herein are fusion proteins comprising a soluble toxin receptor fragment that binds a toxin fused to or inserted into the transferrin or modified transferrin molecules. The soluble toxin receptor may be synaptotagmin 1 and the soluble fragment is amino acids 1-53 (SEQ ID NO: 4).

### BRIEF DESCRIPTION OF THE DRAWINGS

**Figure 1** shows an alignment of the N and C Domains of Human (Hu) transferrin (Tf) (SEQ ID NO: 3) with similarities and identities highlighted.

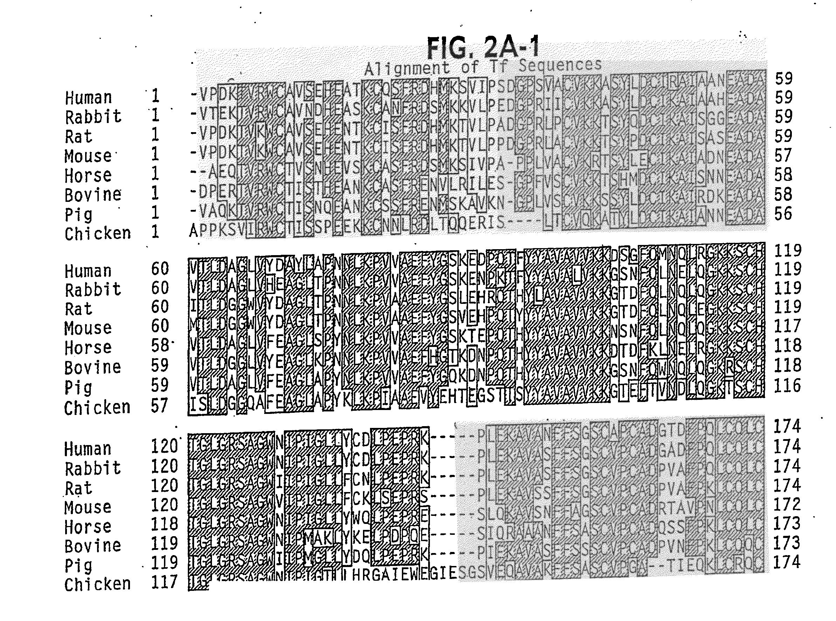

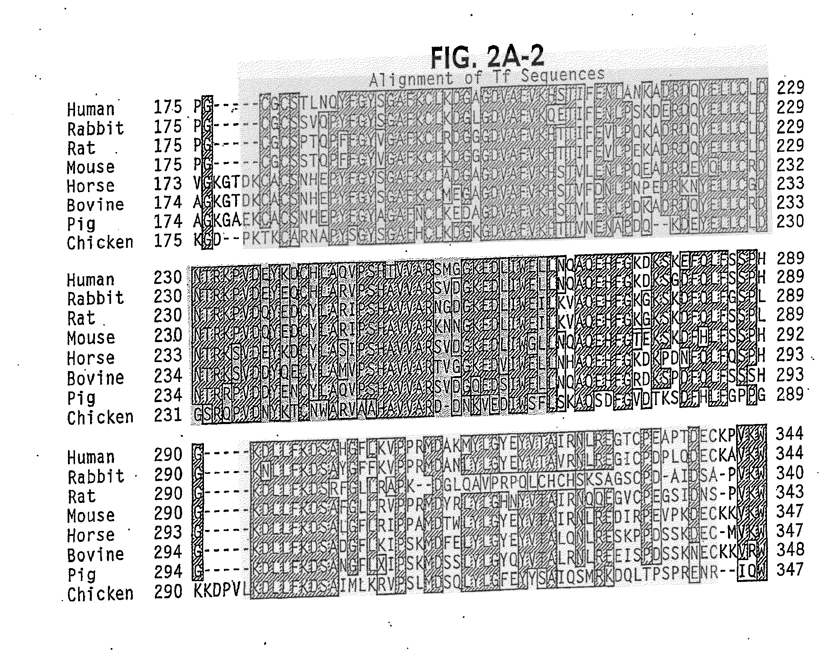

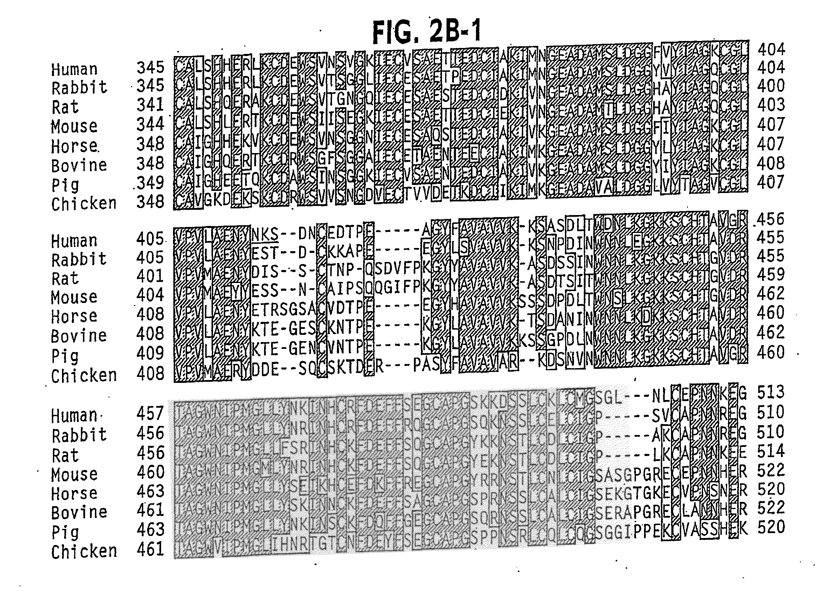

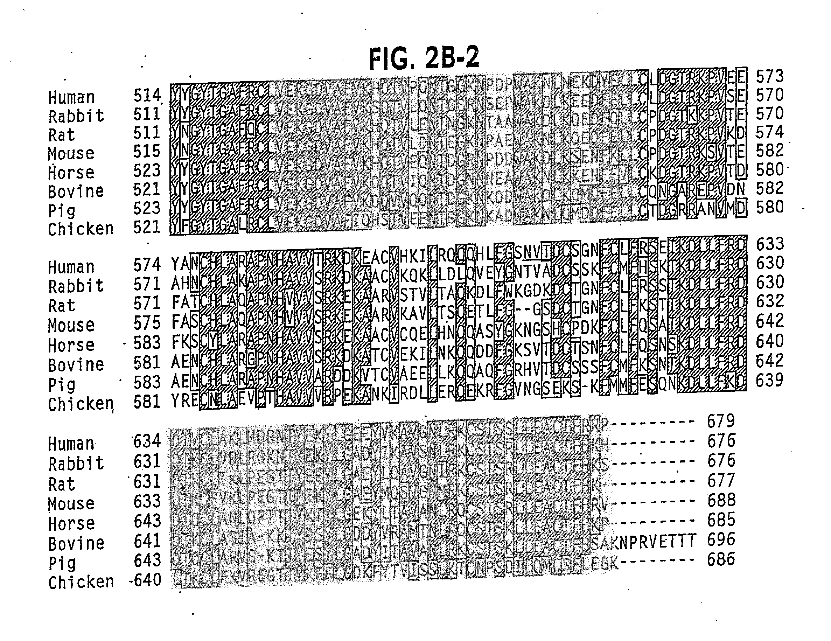

**Figures 2A-2B** show an alignment of transferrin sequences from different species. Light shading: Similarity; Dark shading: Identity (SEQ ID NOS: 84-90).
**Figure 3** shows the location of a number of Tf surface exposed insertion sites for therapeutic proteins, polypeptides or peptides.
**Figure 4** shows the pharmacokinetics of mTf-T20 in two rabbits.

### DETAILED DESCRIPTION

### General Description

The present invention is based in part on the finding by the inventors that therapeutic proteins can be stabilized to extend their serum half-life and/or activity *in vivo* by genetically fusing the therapeutic proteins to transferrin, modified transferrin, or a portion of transferrin or modified transferrin sufficient to extend the half-life of the therapeutic protein in serum. The modified transferrin fusion proteins include a transferrin protein or domain covalently linked to a therapeutic protein or peptide, wherein the transferrin portion is modified to contain one or more amino acid substitutions, insertions or deletions compared to a wild-type transferrin sequence. In one embodiment, Tf fusion proteins are engineered to reduce or prevent glycosylation within the Tf or a Tf domain. In other embodiments, the Tf protein or Tf domain(s) is modified to exhibit reduced or no binding to iron or to have a reduced affinity or not bind to a Tf receptor (TfR).

The therapeutic proteins described herein include, but are not limited to polypeptides, antibodies, peptides, or fragments or variants thereof. The therapeutic proteins described herein include β- interferon, EPO mimetic peptide (EMP1), and T-20. GLP-1 molecules are used in the present invention.

Also described herein are anti-toxin fusion proteins comprising a soluble toxin receptor fragment fused or inserted into transferrin or modified transferrin. Preferably, the soluble toxin receptor fragment binds a specific toxin. In one example, the soluble toxin receptor fragment is amino acids 1-53 (SEQ ID NO: 4) of synaptotagmin 1.

The present invention includes modified transferrin fusion proteins according to the claims and therapeutic compositions comprising the fusion proteins, and such proteins and compositions for use in methods of treating, preventing, or ameliorating diseases or disorders by administering the fusion proteins. A transferrin fusion protein of the invention includes a GLP-1 molecule and a modified transferrin protein, which are associated with one another, preferably by genetic fusion (i.e., the transferrin fusion protein is generated by translation of a nucleic acid in which a polynucleotide encoding all or a portion of GLP-1 is joined in-frame with a polynucleotide encoding all or a portion of modified transferrin) or chemical conjugation to one another. The GLP-1 protein and transferrin protein, once part of the transferrin fusion protein, may be referred to as a "portion", "region" or "moiety" of the transferrin fusion protein (*e.g.,* a "therapeutic protein portion' or a "transferrin protein portion").

In one embodiment, the invention provides a transferrin fusion protein comprising, or alternatively consisting of, a GLP-1 molecule and a modified serum transferrin protein.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

### Definitions

As used herein, an "amino acid corresponding to" or an "equivalent amino acid" in a transferrin sequence is identified by alignment to maximize the identity or similarity between a first transferrin sequence and at least a second transferrin sequence. The number used to identify an equivalent amino acid in a second transferrin sequence is based on the number used to identify the corresponding amino acid in the first transferrin sequence. In certain cases, these phrases may be used to describe the amino acid residues in human transferrin compared to certain residues in rabbit serum transferrin.

As used herein, the term "biological activity" refers to a function or set of activities performed by a therapeutic molecule, protein or peptide in a biological context (*i.e.,* in an organism or an *in vitro* facsimile thereof). Biological activities may include but are not limited to the functions of the therapeutic molecule portion of the claimed fusion proteins, such as, but not limited to, the induction of extracellular matrix secretion from responsive cell lines, the induction of hormone secretion, the induction of chemotaxis, the induction of mitogenesis, the induction of differentiation, or the inhibition of cell division of responsive cells. A fusion protein or peptide of the invention is considered to be biologically active if it exhibits one or more biological activities of its therapeutic protein's native counterpart.

As used herein, "binders" are agents used to impart cohesive qualities to the powdered material. Binders, or "granulators" as they are sometimes known, impart a cohesiveness to the tablet formulation, which insures the tablet remaining intact after compression, as well as improving the free-flowing qualities by the formulation of granules of desired hardness and size. Materials commonly used as binders include starch; gelatin; sugars, such as sucrose, glucose, dextrose, molasses, and lactose; natural and synthetic gums, such as acacia, sodium alginate, extract of Irish moss, panwar gum, ghatti gum, mucilage of isapol husks, carboxymethylcellulose, methylcellulose, polyvinylpyrrolidone, Veegum, microcrystalline cellulose, microcrystalline dextrose, amylose, and larch arabogalactan, and the like.

As used herein, the term "carrier" refers to a diluent, adjuvant, excipient, or vehicle with which a composition is administered. Such pharmaceutical carriers can be sterile liquids, such as water and oils, including those of petroleum, animal, vegetable or synthetic origin, such as peanut oil, soybean oil, mineral oil, sesame oil and the like.

As used herein, "coloring agents" are agents that give tablets a more pleasing appearance, and in addition help the manufacturer to control the product during its preparation and help the user to identify the product. Any of the approved certified water-soluble FD&C dyes, mixtures thereof, or their corresponding lakes may be used to color tablets. A color lake is the combination by adsorption of a water-soluble dye to a hydrous oxide of a heavy metal, resulting in an insoluble form of the dye.

As used herein, "diluents" are inert substances added to increase the bulk of the formulation to make the tablet a practical size for compression. Commonly used diluents include calcium phosphate, calcium sulfate, lactose, kaolin, mannitol, sodium chloride, dry starch, powdered sugar, silica, and the like.

As used herein, "disintegrators" or "disintegrants" are substances that facilitate the breakup or disintegration of tablets after administration. Materials serving as disintegrants have been chemically classified as starches, clays, celluloses, algins, or gums. Other disintegrators include Veegum HV, methylcellulose, agar, bentonite, cellulose and wood products, natural sponge, cation-exchange resins, alginic acid, guar gum, citrus pulp, cross-linked polyvinylpyrrolidone, carboxymethylcellulose, and the like.

The term "dispersibility" or "dispersible" means a dry powder having a moisture content of less than about 10% by weight (%w) water, usually below about 5%w and preferably less than about 3%w; a particle size of about 1.0-5.0 µm mass median diameter (MMD), usually 1.0-4.0 µm MMD, and preferably 1.0-3.0 µm MMD; a delivered dose of about >30%, usually >40%, preferably >50%, and most preferred >60%; and an aerosol particle size distribution of 1.0-5.0 µm mass median aerodynamic diameter (MMAD), usually 1.5-4.5 µm MMAD, and preferably 1.5-4.0 µm MMAD.

The term "dry" means that the composition has a moisture content such that the particles are readily dispersible in an inhalation device to form an aerosol. This moisture content is generally below about 10% by weight (%w) water, usually below about 5%w and preferably less than about 3%w.

As used herein, "effective amount" means an amount of a drug or pharmacologically active agent that is sufficient to provide the desired local or systemic effect and performance at a reasonable benefit/risk ratio attending any medical treatment.

As used herein, "flavoring agents" vary considerably in their chemical structure, ranging from simple esters, alcohols, and aldehydes to carbohydrates and complex volatile oils. Synthetic flavors of almost any desired type are now available.

As used herein, the terms "fragment of a Tf protein" or "Tf protein," or "portion of a Tf protein" refer to an amino acid sequence comprising at least about 5%, 10%, 20%, 30%, 40%, 50%, 60%, 70%, 80%, 90%, 95%, 96%, 97%, 98%, 99% or 100% of a naturally occurring Tf protein or mutant thereof.

As used herein, the term "gene" refers to any segment of DNA associated with a biological function. Thus, genes include, but are not limited to, coding sequences and/or the regulatory sequences required for their expression. Genes can also include non-expressed DNA segments that, for example, form recognition sequences for other proteins. Genes can be obtained from a variety of sources, including cloning from a source of interest or synthesizing from known or predicted sequence information, and may include sequences designed to have desired parameters.

As used herein, a "heterologous polynucleotide" or a "heterologous nucleic acid" or a "heterologous gene" or a "heterologous sequence" or an "exogenous DNA segment" refers to a polynucleotide, nucleic acid or DNA segment that originates from a source foreign to the particular host cell, or, if from the same source, is modified from its original form. A heterologous gene in a host cell includes a gene that is endogenous to the particular host cell, but has been modified. Thus, the terms refer to a DNA segment which is foreign or heterologous to the cell, or homologous to the cell but in a position within the host cell nucleic acid in which the element is not ordinarily found. As an example, a signal sequence native to a yeast cell but attached to a human Tf sequence is heterologous.

As used herein, an "isolated" nucleic acid sequence refers to a nucleic acid sequence which is essentially free of other nucleic acid sequences, e.g., at least about 20% pure, preferably at least about 40% pure, more preferably about 60% pure, even more preferably about 80% pure, most preferably about 90% pure, and even most preferably about 95% pure, as determined by agarose gel electrophoresis. For example, an isolated nucleic acid sequence can be obtained by standard cloning procedures used in genetic engineering to relocate the nucleic acid sequence from its natural location to a different site where it will be reproduced. The cloning procedures may involve excision and isolation of a desired nucleic acid fragment comprising the nucleic acid sequence encoding the polypeptide, insertion of the fragment into a vector molecule, and incorporation of the recombinant vector into a host cell where multiple copies or clones of the nucleic acid sequence will be replicated. The nucleic acid sequence may be of genomic, cDNA, RNA, semi-synthetic, synthetic origin, or any combinations thereof.

As used herein, two or more DNA coding sequences are said to be "joined" or "fused" when, as a result of in-frame fusions between the DNA coding sequences, the DNA coding sequences are translated into a fusion polypeptide. The term "fusion" in reference to Tf fusions includes, but is not limited to, attachment of at least one therapeutic protein, polypeptide or peptide to the N -terminal end of Tf, attachment to the C-terminal end of Tf, and/or insertion between any two amino acids within Tf.

As used herein, "lubricants" are materials that perform a number of functions in tablet manufacture, such as improving the rate of flow of the tablet granulation, preventing adhesion of the tablet material to the surface of the dies and punches, reducing interparticle friction, and facilitating the ejection of the tablets from the die cavity. Commonly used lubricants include talc, magnesium stearate, calcium stearate, stearic acid, and hydrogenated vegetable oils. Typical amounts of lubricants range from about 0.1% by weight to about 5% by weight.

As used herein, "Modified transferrin" as used herein refers to a transferrin molecule that exhibits at least one modification of its amino acid sequence, compared to wild-type transferrin.

As used herein, "Modified transferrin fusion protein" as used herein refers to a protein formed by the fusion of at least one molecule of modified transferrin (or a fragment or variant thereof) to at least one molecule of a therapeutic protein (or fragment or variant thereof).

As used herein, the terms "nucleic acid" or "polynucleotide" refer to deoxyribonucleotides or ribonucleotides and polymers thereof in either single- or double-stranded form. Unless specifically limited, the terms encompass nucleic acids containing analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g. degenerate codon substitutions) and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al. (1991) Nucleic Acid Res. 19:5081; Ohtsuka et al. (1985) J. Biol. Chem. 260:2605-2608; *Cassol et al.* (1992); Rossolini et al. (1994) Mol. Cell. Probes 8:91-98). The term nucleic acid is used interchangeably with gene, cDNA, and mRNA encoded by a gene.

As used herein, a DNA segment is referred to as "operably linked" when it is placed into a functional relationship with another DNA segment. For example, DNA for a signal sequence is operably linked to DNA encoding a fusion protein of the invention if it is expressed as a preprotein that participates in the secretion of the fusion protein; a promoter or enhancer is operably linked to a coding sequence if it stimulates the transcription of the sequence. Generally, DNA sequences that are operably linked are contiguous, and in the case of a signal sequence or fusion protein both contiguous and in reading phase. However, enhancers need not be contiguous with the coding sequences whose transcription they control. Linking, in this context, is accomplished by ligation at convenient restriction sites or at adapters or linkers inserted in lieu thereof.

As used herein, "pharmaceutically acceptable" refers to materials and compositions that are physiologically tolerable and do not typically produce an allergic or similar untoward reaction, such as gastric upset, dizziness and the like, when administered to a human. Typically, as used herein, the term "pharmaceutically acceptable" means approved by a regulatory agency of the Federal or a state government or listed in the U.S. Pharmacopeia or other generally recognized pharmacopeia for use in animals, and more particularly in humans.

As used herein, "physiologically effective amount" is that amount delivered to a subject to give the desired palliative or curative effect. This amount is specific for each drug and its ultimate approved dosage level.

As used herein, the term "powder" means a composition that consists of finely dispersed solid particles that are free flowing and capable of being readily dispersed in an inhalation device and subsequently inhaled by a subject so that the particles reach the lungs to permit penetration into the alveoli. Thus, the powder is said to be "respirable." Preferably the average particle size is less than about 10 microns (µm) in diameter with a relatively uniform spheroidal shape distribution. More preferably the diameter is less than about 7.5 µm and most preferably less than about 5.0 µm. Usually the particle size distribution is between about 0.1 µm and about 5 µm in diameter, particularly about 0.3 µm to about 5 µm.

As used herein, the term "promoter" refers to a region of DNA involved in binding RNA polymerase to initiate transcription.

As used herein, the term "recombinant" refers to a cell, tissue or organism that has undergone transformation with a new combination of genes or DNA.

As used herein, the term "subject" can be a human, a mammal, or an animal. The subject being treated is a patient in need of treatment.

As used herein, a targeting entity, protein, polypeptide or peptide refers to a molecule that binds specifically to a particular cell type [normal (*e.g.,* lymphocytes) or abnormal *e.g.,* (cancer cell)] and therefore may be used to target a Tf fusion protein or compound (drug, or cytotoxic agent) to that cell type specifically.

As used herein, "tablets" are solid pharmaceutical dosage forms containing drug substances with or without suitable diluents and prepared either by compression or molding methods well known in the art. Tablets have been in widespread use since the latter part of the 19^{th} century and their popularity continues. Tablets remain popular as a dosage form because of the advantages afforded both to the manufacturer (*e.g.,* simplicity and economy of preparation, stability, and convenience in packaging, shipping, and dispensing) and the patient (*e.g.,* accuracy of dosage, compactness, portability, blandness of taste, and ease of administration). Although tablets are most frequently discoid in shape, they may also be round, oval, oblong, cylindrical, or triangular. They may differ greatly in size and weight depending on the amount of drug substance present and the intended method of administration. They are divided into two general classes, (1) compressed tablets, and (2) molded tablets or tablet triturates. In addition to the active or therapeutic ingredient or ingredients, tablets contain a number or inert materials or additives. A first group of such additives includes those materials that help to impart satisfactory compression characteristics to the formulation, including diluents, binders, and lubricants. A second group of such additives helps to give additional desirable physical characteristics to the finished tablet, such as disintegrators, colors, flavors, and sweetening agents.

As used herein, the term "therapeutically effective amount" refers to that amount of the transferrin fusion protein comprising a therapeutic molecule which, when administered to a subject in need thereof, is sufficient to effect treatment. The amount of transferrin fusion protein which constitutes a "therapeutically effective amount" will vary depending on the therapeutic protein used, the severity of the condition or disease, and the age and body weight of the subject to be treated, but can be determined routinely by one or ordinary skill in the art having regard to his/her own knowledge and to this disclosure.

As used herein, "therapeutic protein" refers to proteins, polypeptides, peptides or fragments or variants thereof, having one or more therapeutic and/or biological activities. Therapeutic proteins encompassed by the invention include but are not limited to proteins, polypeptides, peptides, antibodies, and biologies. The terms peptides, proteins, and polypeptides are used interchangeably herein. Additionally, the term "therapeutic protein" may refer to the endogenous or naturally occurring correlate of a therapeutic protein. By a polypeptide displaying a "therapeutic activity" or a protein that is "therapeutically active" is meant a polypeptide that possesses one or more known biological and/or therapeutic activities associated with a therapeutic protein such as one or more of the therapeutic proteins described herein or otherwise known in the art. As a non-limiting example, a "therapeutic protein" is a protein that is useful to treat, prevent or ameliorate a disease, condition or disorder. Such a disease, condition or disorder may be in humans or in a non-human animal, *e.g.,* veterinary use.

As used herein, the term "toxin" refers to a poisonous substance of biological origin.

As used herein, the term "transformation" refers to the transfer of nucleic acid (*i.e.,* a nucleotide polymer) into a cell. As used herein, the term "genetic transformation" refers to the transfer and incorporation of DNA, especially recombinant DNA, into a cell.

As used herein, the term "transformant" refers to a cell, tissue or organism that has undergone transformation.

As used herein, the term "transgene" refers to a nucleic acid that is inserted into an organism, host cell or vector in a manner that ensures its function.

As used herein, the term "transgenic" refers to cells, cell cultures, organisms, bacteria, fungi, animals, plants, and progeny of any of the preceding, which have received a foreign or modified gene and in particular a gene encoding a modified Tf fusion protein by one of the various methods of transformation, wherein the foreign or modified gene is from the same or different species than the species of the organism receiving the foreign or modified gene.

"Variants or variant" refers to a polynucleotide or nucleic acid differing from a reference nucleic acid or polypeptide, but retaining essential properties thereof. Generally, variants are overall closely similar, and, in many regions, identical to the reference nucleic acid or polypeptide. As used herein, "variant" refers to a therapeutic protein portion of a transferrin fusion protein of the invention, differing in sequence from a native therapeutic protein but retaining at least one functional and/or therapeutic property thereof as described elsewhere herein or otherwise known in the art.

As used herein, the term "vector" refers broadly to any plasmid, phagemid or virus encoding an exogenous nucleic acid. The term is also be construed to include non-plasmid, non-phagemid and non-viral compounds which facilitate the transfer of nucleic acid into virions or cells, such as, for example, polylysine compounds and the like. The vector may be a viral vector that is suitable as a delivery vehicle for delivery of the nucleic acid, or mutant thereof, to a cell, or the vector may be a non-viral vector which is suitable for the same purpose. Examples of viral and non-viral vectors for delivery of DNA to cells and tissues are well known in the art and are described, for example, in Ma et al. (1997, Proc. Natl. Acad. Sci. U.S.A. 94:12744-12746). Examples of viral vectors include, but are not limited to, a recombinant vaccinia virus, a recombinant adenovirus, a recombinant retrovirus, a recombinant adeno-associated virus, a recombinant avian pox virus, and the like (Cranage et al., 1986, EMBO J. 5:3057-3063; International Patent Application No. WO 94/17810, published August 18, 1994; International Patent Application No. WO 94/23744, published October 27, 1994). Examples of non-viral vectors include, but are not limited to, liposomes, polyamine derivatives of DNA, and the like.

As used herein, the term "wild type" refers to a polynucleotide or polypeptide sequence that is naturally occurring.

### Transferrin and Transferrin Modifications

The present invention provides fusion proteins comprising a GLP-1 protein and modified transferrin. Other therapeutic proteins described herein include β-IFN, EMP1, and T-20. In some aspects of the invention, the transferrin protein which is modified for use in the present invention is human transferrin protein of SEQ ID NO: 3. In other aspects of the invention, the transferrin protein which is modified may be selected from the group consisting of human Tf (SE ID NO: 3), rabbit Tf (SEQ ID NO: 84), rat Tf (SEQ ID NO: 85), mouse Tf (SEQ ID NO: 86), horse Tf (SEQ ID NO: 87), bovine Tf 9SEQ ID NO: 88), pig Tf (SEQ ID NO: 89) and chicken Tf (SEQ ID NO: 90). The modified transferrin protein is modified by amino acid substitution, deletion or insertion of between 1 and 30 amino acids.

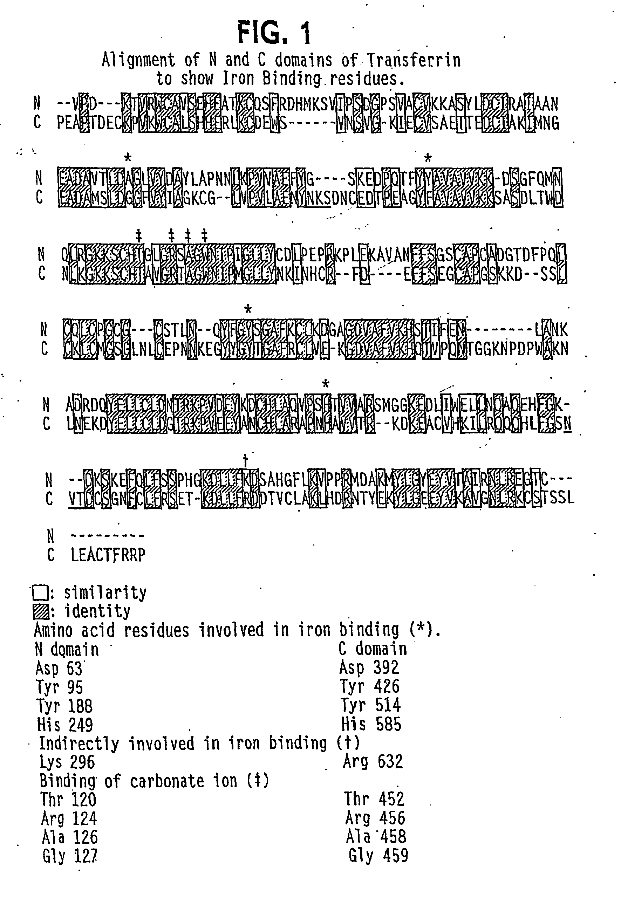

The wild-type human Tf (Tf) is a 679 amino acid protein of approximately 75kDa (not accounting for glycosylation), with two main domains, N (about 330 amino acids) and C (about 340 amino acids), which appear to originate from a gene duplication. See GenBank accession numbers NM_001063, XM_002793, M12530, XM_039845, XM_039847 and S95936 (www.ncbi.nlm.nih.gov/), as well as SEQ ID NOS 1, 2 and 3. The two domains have diverged over time but retain a large degree of identity/similarity (Fig. 1).

Each of the N and C domains is further divided into two subdomains, N1 and N2, C1 and C2. The function of Tf is to transport iron to the cells of the body. This process is mediated by the Tf receptor (TfR), which is expressed on all cells, particularly actively growing cells. TfR recognizes the iron bound form of Tf (two molecules of which are bound per receptor), endocytosis then occurs whereby the TfR/Tf complex is transported to the endosome, at which point the localized drop in pH results in release of bound iron and the recycling of the TfR/Tf complex to the cell surface and release of Tf (known as apoTf in its iron-unbound form). Receptor binding is through the C domain of Tf. The two glycosylation sites in the C domain do not appear to be involved in receptor binding as unglycosylated iron bound Tf does bind the receptor.

Each Tf molecule can carry two iron ions (Fe³⁺). These are complexed in the space between the N1 and N2, C1 and C2 sub domains resulting in a conformational change in the molecule. Tf crosses the blood brain barrier (BBB) via the Tf receptor.

In human transferrin, the iron binding sites comprise at least amino acids Asp 63 (Asp 82 of SEQ ID NO: 2 which includes the native Tf signal sequence), Asp 392 (Asp 411 of SEQ ID NO: 2), Tyr 95 (Tyr 114 of SEQ ID NO: 2), Tyr 426 (Tyr 445 of SEQ ID NO: 2), Tyr 188 (Tyr 207 of SEQ ID NO: 2), Tyr 514 or 517 (Tyr 533 or Tyr 536 SEQ ID NO: 2), His 249 (His 268 of SEQ ID NO: 2), and His 585 (His 604 of SEQ ID NO: 2) of SEQ ID NO: 3. The hinge regions comprise at least N domain amino acid residues 94-96, 245- 247 and/or 316-318 as well as C domain amino acid residues 425-427, 581-582 and/or 652-658 of SEQ ID NO: 3. The carbonate binding sites comprise at least amino acids Thr 120 (Thr 139 of SEQ ID NO: 2), Thr 452 (Thr 471 of SEQ ID NO: 2), Arg 124 (Arg 143 of SEQ ID NO: 2), Arg 456 (Arg 475 of SEQ ID NO: 2), Ala 126 (Ala 145 of SEQ ID NO: 2), Ala 458 (Ala 477 of SEQ ID NO: 2), Gly 127 (Gly 146 of SEQ ID NO: 2), and Gly 459 (Gly 478 of SEQ ID NO: 2) of SEQ ID NO: 3.

The modified transferrin fusion protein of the invention includes a modified human transferrin, although in other aspects other animal Tf molecule may be used to produce the fusion proteins of the invention, including cow, pig, rabbit, rat, mouse, and chicken Tf. All of these Tf sequences are readily available in GenBank and other public databases. The human Tf nucleotide sequence is available (see SEQ ID NOS 1, 2 and 3 and the accession numbers described above and available at www.ncbi.nlm.nih.gov/) and can be used to make genetic fusions between Tf or a domain of Tf and the therapeutic molecule of choice. Fusions may also be made from related molecules such as lacto transferrin (lactoferrin) GenBank Acc: NM_002343) or melanotransferrin (GenBank Acc. NM_013900, murine melanotransferrin).

Melanotransferrin is a glycosylated protein found at high levels in malignant melanoma cells and was originally named human melanoma antigen p97 (Brown et al., 1982, Nature, 296: 171-173). It possesses high sequence homology with human serum transferrin, human lactoferrin, and chicken transferrin (Brown et al., 1982, Nature, 296: 171-173; Rose et al., Proc. Natl. Acad. Sci. USA, 1986, 83: 1261-1265). However, unlike these receptors, no cellular receptor has been identified for melanotransferrin. Melanotransferrin reversibly binds iron and it exists in two forms, one of which is bound to cell membranes by a glycosyl phosphatidylinositol anchor while the other form is both soluble and actively secreted (Baker et al., 1992, FEBS Lett, 298: 215-218; Alemany et al., 1993, J. Cell Sci., 104: 1155-1162; Food et al., 1994, J. Biol. Chem. 274: 7011-7017).

Lactoferrin (Lf), a natural defense iron-binding protein, has been found to possess antibacterial, antimycotic, antiviral, antineoplastic and anti-inflammatory activity. The protein is present in exocrine secretions that are commonly exposed to normal flora: milk, tears, nasal exudate, saliva, bronchial mucus, gastrointestinal fluids, cervico-vaginal mucus and seminal fluid. Additionally, Lf is a major constituent of the secondary specific granules of circulating polymorphonuclear neutrophils (PMNs). The apoprotein is released on degranulation of the PMNs in septic areas. A principal function of Lf is that of scavenging free iron in fluids and inflamed areas so as to suppress free radical-mediated damage and decrease the availability of the metal to invading microbial and neoplastic cells. In a study that examined the turnover rate of ¹²⁵I Lf in adults, it was shown that Lf is rapidly taken up by the liver and spleen, and the radioactivity persisted for several weeks in the liver and spleen (Bennett et al (1979), Clin. Sci. (Lond.) 57: 453-460).

The transferrin portion of the transferrin fusion protein described herein includes a transferrin splice variant. In one example, a transferrin splice variant can be a splice variant of human transferrin. In one example, the human transferrin splice variant can be that of Genbank Accession AAA61140.

The transferrin portion of the transferrin fusion protein described herein includes a lactoferrin splice variant. In one example, a human serum lactoferrin splice variant can be a novel splice variant of a neutrophil lactoferrin. The neutrophil lactoferrin splice variant can be that of Genbank Accession AAA59479. The neutrophil lactoferrin splice variant can comprise the following amino acid sequence EDCIALKGEADA (SEQ ID NO: 8), which includes the novel region of splice-variance.

The transferrin portion of the transferrin fusion protein described herein may include a melanotransferrin variant.

Modified Tf fusions may be made with any Tf protein, fragment, domain, or engineered domain. For instance, fusion proteins may be produced using the full-length Tf sequence, with or without the native Tf signal sequence. Tf fusion proteins may also be made using a single Tf domain, such as an individual N or C domain or a modified form of Tf comprising 2N or 2C domains (see U.S. Provisional Application 60/406,977, filed August 30,2002).

In some examples, fusions of a therapeutic protein to a single C domain may be produced, wherein the C domain is altered to reduce, inhibit or prevent glycosylation. In other examples , the use of a single N domain is advantageous as the Tf glycosylation sites reside in the C domain and the N domain, on its own. A preferred example is the Tf fusion protein having a single N domain which is expressed at a high level.

As used herein, a C terminal domain or lobe modified to function as an N-like domain is modified to exhibit glycosylation patterns or iron binding properties substantially like that of a native or wild-type N domain or lobe. In a preferred example, the C domain or lobe is modified so that it is not glycosylated and does not bind iron by substitution of the relevant C domain regions or amino acids to those present in the corresponding regions or sites of a native or wild-type N domain.

As used herein, a Tf moiety comprising "two N domains or lobes" includes a Tf molecule that is modified to replace the native C domain or lobe with a native or wild-type N domain or lobe or a modified N domain or lobe or contains a C domain that has been modified to function substantially like a wild-type or modified N domain.

Analysis of the two domains by overlay of the two domains (Swiss PDB Viewer 3.7b2, Iterative Magic Fit) and by direct amino acid alignment (ClustalW multiple alignment) reveals that the two domains have diverged over time. Amino acid alignment shows 42% identity and 59% similarity between the two domains. However, approximately 80% of the N domain matches the C domain for structural equivalence. The C domain also has several extra disulfide bonds compared to the N domain.

Alignment of molecular models for the N and C domain reveals the following structural equivalents:

| | | | | | | | | | | | | | | |
|---|---|---|---|---|---|---|---|---|---|---|---|---|---|---|
| **N domain (1-330)** | 4-24 | 36-72 | 94-136 | 138-139 | 149-164 | 168-173 | 178-198 | 219-255 | 259-260 | 263-268 | 271-275 | 279-280 | 283-288 | 309-327 |
| | | 75-88 | | | | | 200-214 | | | | | | 290-304 | |
| **C domain (340-679)** | 340-361 | 365-415 | 425-437 | 470-471 | 475-490 | 492-497 | 507-542 | 555-591 | 593-594 | 597-602 | 605-609 | 614-615 | 620-640 | 645-663 |
| | | | 439-468 | | | | | | | | | | | |

The disulfide bonds for the two domains align as follows:

| N | **C** | | | |
|---|---|---|---|---|
| | *C339-C596* | | | |
| **C9-C48** | **C345-C377** | | | |
| **C19-C39** | **C355-C368** | | Bold | aligned disulfide bonds |
| | C402-C674 | | *Italics* | bridging peptide |
| | C418-C637 | | | |
| **C118-C194** | **C450-C523** | | | |
| *C137-C331* | | | | |
| | C474-C665 | | | |
| **C158-C174** | **C484-C498** | | | |
| C161-C179 | | | | |
| **C171-C177** | **C495-C506** | | | |
| **C227-C241** | **C563-C577** | | | |
| | C615-C620 | | | |

The transferrin portion of the transferrin fusion protein may include at least two N terminal lobes of transferrin. The transferrin portion of the transferrin fusion protein may include at least two N terminal lobes of transferrin derived from human serum transferrin.

The transferrin portion of the transferrin fusion protein may include, comprise, or consist of at least two N terminal lobes of transferrin having a mutation in at least one amino acid residue selected from the group consisting of Asp63, Gly65, Tyr95, Tyr188, and His249 of SEQ ID NO: 3.

In one embodiment, the transferrin portion of the modified transferrin fusion protein includes a recombinant human serum transferrin N-terminal lobe mutant having a mutation at Lys206 or His207 of SEQ ID NO: 3.

The transferrin portion of the transferrin fusion protein may include, comprise, or consist of at least two C terminal lobes of transferrin.

The transferrin portion of the transferrin fusion protein may include; at least two C terminal lobes of transferrin derived from human serum transferrin.

The C terminal lobe mutant may further include a mutation of at least one of Asn413 and Asn611 of SEQ ID NO: 3 which does not allow glycosylation.

The transferrin portion of the transferrin fusion protein may include at least two C terminal lobes of transferrin having a mutation in at least one amino acid residue selected from the group consisting of Asp392, Tyr426, Tyr514, Tyr517 and His585 of SEQ ID NO: 3, wherein the mutant retains the ability to bind metal. Alternatively, the transferrin portion of the transferrin fusion protein may include at least two C terminal lobes of transferrin having a mutation in at least one amino acid residue selected from the group consisting of Tyr426, Tyr514, Tyr517 and His585 of SEQ ID NO: 3, wherein the mutant has a reduced ability to bind metal.

The transferrin portion of the transferrin fusion protein may include at least two C terminal lobes of transferrin having a mutation in at least one amino acid residue selected from the group consisting of Asp392, Tyr426, Tyr517 and His585 of SEQ ID NO:3, wherein the mutant does not retain the ability to bind metal and functions substantially like an N domain.

The Tf or Tf portion will be of sufficient length to increase the *in vivo* circulatory half-life, serum stability, *in vitro* solution stability or bioavailability of the therapeutic protein or peptide or soluble toxin receptor compared to the *in vivo* circulatory half-life, serum stability, *in vitro* solution stability or bioavailability of the therapeutic protein or peptide or soluble toxin receptor in an unfused state. Such an increase in stability, serum half-life or bioavailability may be about a 30%, 50%, 70%, 80%, 90% or more increase over the unfused therapeutic protein or peptide or soluble toxin receptor. In some cases, the transferrin fusion proteins comprising modified transferrin exhibit a serum half-life of about 10-20 or more days, about 12-18 days or about 14-17 days.

When the C domain of Tf is part of the fusion protein, the two N-linked glycosylation sites, amino acid residues corresponding to N413 and N611 of SEQ ID NO: 3 may be mutated for expression in a yeast system to prevent glycosylation or hypermannosylationn and extend the serum half-life of the fusion protein and/or therapeutic protein (to produce asialo-, or in some instances, monosialo-Tf or disialo-Tf). In addition to Tf amino acids corresponding to N413 and N611, mutations may be to the adjacent residues within the N-X-S/T glycosylation site to prevent or substantially reduce glycosylation. See U.S. Patent 5,986,067 of Funk *et al.* It has also been reported that the N domain of Tf expressed in *Pichia pastoris* becomes O-linked glycosylated with a single hexose at S32 which also may be mutated or modified to prevent such glycosylation.

Accordingly, in one embodiment of the invention, the transferrin fusion protein includes a modified transferrin molecule wherein the transferrin exhibits reduced glycosylation, including but not limited to asialo- monosialo- and disialo- forms of Tf. In another embodiment, the transferrin portion of the transferrin fusion protein includes a recombinant transferrin mutant that is mutated to prevent glycosylation. In another embodiment, the transferrin portion of the transferrin fusion protein includes a recombinant transferrin mutant that is fully glycosylated. In a further embodiment, the transferrin portion of the transferrin fusion protein incudes a recombinant human serum transferrin mutant that is mutated to prevent glycosylation, wherein at least one of Asn413 and Asn611 of SEQ ID NO: 3 are mutated to an amino acid which does not allow glycosylation. In another embodiment, the transferrin portion of the transferrin fusion protein includes a recombinant human serum transferrin mutant that is mutated to prevent or substantially reduce glycosylation, wherein mutations may be to the adjacent residues within the N-X-S/T glycosylation site. Moreover, glycosylation may be reduced or prevented by mutating the serine or threonine residue. Further, changing the X to proline is known to inhibit glycosylation.

As discussed below in more detail, modified Tf fusion proteins of the invention may also be engineered to not bind iron and/or bind the Tf receptor. In other embodiments of the invention, the iron binding is retained and the iron binding ability of Tf may be used to deliver a GLP-1 protein or peptide(s) to the inside of a cell, across an epithelial or endothelial cell membrane and/or across the BBB. These embodiments that bind iron and the Tf receptor will be engineered to reduce or prevent glycosylation to extend the serum half-life of the GLP-1 protein. The N domain alone will not bind to TfR when loaded with iron, and the iron bound C domain will bind TfR but not with the same affinity as the whole molecule.

In another embodiment, the transferrin portion of the transferrin fusion protein includes a recombinant transferrin mutant having a mutation wherein the mutant does not retain the ability to bind metal ions. In an alternate embodiment, the transferrin portion of the transferrin fusion protein includes a recombinant transferrin mutant having a mutation wherein the mutant has a weaker binding avidity for metal ions than wild-type serum transferrin. In an alternate embodiment, the transferrin portion of the transferrin fusion protein includes a recombinant transferrin mutant having a mutation wherein the mutant has a stronger binding avidity for metal ions than wild-type serum transferrin.

In another embodiment, the transferrin portion of the transferrin fusion protein includes a recombinant transferrin mutant having a mutation wherein the mutant does not retain the ability to bind to the transferrin receptor. In an alternate embodiment, the transferrin portion of the transferrin fusion protein includes a recombinant transferrin mutant having a mutation wherein the mutant has a weaker binding avidity for the transferrin receptor than wild-type serum transferrin. In an alternate embodiment, the transferrin portion of the transferrin fusion protein includes a recombinant transferrin mutant having a mutation wherein the mutant has a stronger binding avidity for the transferrin receptor than wild-type serum transferrin.

Also described herein is a recombinant transferrin mutant having a mutation wherein the mutant does not retain the ability to bind to carbonate ions. In an alternate example, the transferrin portion of the transferrin fusion protein includes a recombinant transferrin mutant having a mutation wherein the mutant has a weaker binding avidity for carbonate ions than wild-type serum transferrin. In an alternate example, the transferrin portion of the transferrin fusion protein includes a recombinant transferrin mutant having a mutation wherein the mutant has a stronger binding avidity for carbonate ions than wild-type serum transferrin.

In another embodiment, the transferrin portion of the transferrin fusion protein includes a recombinant human serum transferrin mutant having a mutation in at least one amino acid residue selected from the group consisting of Asp63, Gly65, Tyr95, Tyr188, His249, Asp392, Tyr426, Tyr514, Tyr517 and His585 of SEQ ID NO: 3, wherein the mutant retains the ability to bind metal ions. In an alternate embodiment, a recombinant human serum transferrin mutant having a mutation in at least one amino acid residue selected from the group consisting of Asp63, Gly65, Tyr95, Tyr188, His249, Asp392, Tyr426, Tyr514, Tyr517 and His585 of SEQ ID NO: 3, wherein the mutant has a reduced ability to bind metal ions. In another embodiment, a recombinant human serum transferrin mutant having a mutation in at least one amino acid residue selected from the group consisting of Asp63, Gly65, Tyr95, Tyr188, His249, Asp392, Tyr426, Tyr517 and His585 of SEQ ID NO: 3, wherein the mutant does not retain the ability to bind metal ions.

In another embodiment, the transferrin portion of the transferrin fusion protein includes a recombinant human serum transferrin mutant having a mutation at Lys206 or His207 of SEQ ID NO:3, wherein the mutant has a stronger binding avidity for metal ions than wild-type human serum transferrin (see U.S. Patent 5,986,067).

In an alternate embodiment, the transferrin portion of the transferrin fusion protein includes a recombinant human serum transferrin mutant having a mutation at Lys206 or His207 of SEQ ID NO:3, wherein the mutant has a weaker binding avidity for metal ions than wild-type human serum transferrin. In a further embodiment, the transferrin portion of the transferrin fusion protein includes a recombinant human serum transferrin mutant having a mutation at Lys206 or His207 of SEQ ID NO:3, wherein the mutant does not bind metal ions.

Any available technique may be used to produce the transferrin fusion proteins of the invention, including but not limited to molecular techniques commonly available, for instance, those disclosed in Sambrook et al. Molecular Cloning: A Laboratory Manual, 2nd Ed., Cold Spring Harbor Laboratory Press, 1989. When carrying out nucleotide substitutions using techniques for accomplishing site-specific mutagenesis that are well known in the art, the encoded amino acid changes are preferably of a minor nature, that is, conservative amino acid substitutions, although other, non-conservative, substitutions are contemplated as well, particularly when producing a modified transferrin portion of a Tf fusion protein, e.g., a modified Tf protein exhibiting reduced glycosylation, reduced iron binding and the like. Specifically contemplated are amino acid substitutions, small deletions or insertions, typically of one to about 30 amino acids; insertions between transferrin domains; small amino- or carboxyl-terminal extensions, such as an amino-terminal methionine residue, or small linker peptides of less than 50, 40, 30, 20 or 10 residues between transferrin domains or linking a transferrin protein and therapeutic protein or peptide or soluble toxin receptor or a small extension that facilitates purification, such as a poly-histidine tract, an antigenic epitope or a binding domain.

Examples of conservative amino acid substitutions are substitutions made within the same group such as within the group of basic amino acids (such as arginine, lysine, histidine), acidic amino acids (such as glutamic acid and aspartic acid), polar amino acids (such as glutamine and asparagine), hydrophobic amino acids (such as leucine, isoleucine, valine), aromatic amino acids (such as phenylalanine, tryptophan, tyrosine) and small amino acids (such as glycine, alanine, serine, threonine, methionine).

Non-conservative substitutions encompass substitutions of amino acids in one group by amino acids in another group. For example, a non-conservative substitution would include the substitution of a polar amino acid for a hydrophobic amino acid. For a general description of nucleotide substitution, see *e.g.* Ford et al. (1991), Prot. Exp. Pur. 2: 95-107. Non-conservative substitutions, deletions and insertions are particularly useful to produce Tf fusion proteins of the invention that exhibit no or reduced binding of iron, no or reduced binding of the fusion protein to the Tf receptor and/or no or reduced glycosylation.

Iron binding and/or receptor binding may be reduced or disrupted by mutation, including deletion, substitution or insertion into, amino acid residues corresponding to one or more of Tf N domain residues Asp63, Tyr95, Tyr188, His249 and/or C domain residues Asp 392, Tyr 426, Tyr 514 and/or His 585 of SEQ ID NO: 3. Iron binding may also be affected by mutation to amino acids Lys206, His207 or Arg632 of SEQ ID NO: 3. Carbonate binding may be reduced or disrupted by mutation, including deletion, substitution or insertion into, amino acid residues corresponding to one or more of Tf N domain residues Thr120, Arg124, Ala126, Gly 127 and/or C domain residues Thr 452, Arg 456, Ala 458 and/or Gly 459 of SEQ ID NO: 3. A reduction or disruption of carbonate binding may adversely affect iron and/or receptor binding.

Binding to the Tf receptor may be reduced or disrupted by mutation, including deletion, substitution or insertion into, amino acid residues corresponding to one or more of Tf N domain residues described above for iron binding.

As discussed above, glycosylation may be reduced or prevented by mutation, including deletion, substitution or insertion into, amino acid residues corresponding to one or more of Tf C domain residues around the N-X-S/T sites corresponding to C domain residues N413 and/or N611 (See U.S. Patent No. 5,986,067). For instance, the N413 and/or N611 may be mutated to Glu residues.

In instances where the Tf fusion proteins described herein are not modified to prevent glycosylation, iron binding, carbonate binding and/or receptor binding, glycosylation, iron and/or carbonate ions may be stripped from or cleaved off of the fusion protein. For instance, available deglycosylases may be used to cleave glycosylation residues from the fusion protein, in particular the sugar residues attached to the Tf portion, yeast deficient in glycosylation enzymes may be used to prevent glycosylation and/or recombinant cells may be grown in the presence of an agent that prevents glycosylation, *e.g.,* tunicamycin.

The carbohydrates on the fusion protein may also be reduced or completely removed enzymatically by treating the fusion protein with deglycosylases. Deglycosylases are well known in the art. Examples of deglycosylases include but are not limited to galactosidase, PNGase A, PNGase F, glucosidase, mannosidase, fucosidase, and Endo H deglycosylase.

Nevertheless, in certain circumstances, it may be preferable for oral delivery that the Tf portion of the fusion protein be fully glycosylated

Additional mutations may be made with Tf to alter the three dimensional structure of Tf, such as modifications to the hinge region to prevent the conformational change needed for iron biding and Tf receptor recognition. For instance, mutations may be made in or around N domain amino acid residues 94-96, 245-247 and/or 316-318 as well as C domain amino acid residues 425-427, 581-582 and/or 652-658. In addition, mutations may be made in or around the flanKing regions of these sites to alter Tf structure and function.

In one aspect of the invention, the transferrin fusion protein can function as a carrier protein to extend the half life or bioavailability of the therapeutic protein as well as, in some instances, delivering the therapeutic protein inside a cell and/or across the blood brain barrier. In an alternate embodiment, the transferrin fusion protein includes a modified transferrin molecule wherein the transferrin does not retain the ability to cross the blood brain barrier.

In another embodiment, the transferrin fusion protein includes a modified transferrin molecule wherein the transferrin molecule retains the ability to bind to the transferrin receptor and transport the therapeutic peptide inside cells. In an alternate embodiment, the transferrin fusion protein includes a modified transferrin molecule wherein the transferrin molecule does not retain the ability to bind to the transferrin receptor and transport the therapeutic peptide inside cells.

In further embodiments, the transferrin fusion protein includes a modified transferrin molecule wherein the transferrin molecule retains the ability to bind to the transferrin receptor and transport the therapeutic peptide inside cells and retains the ability to cross the blood brain barrier. In an alternate embodiment, the transferrin fusion protein includes a modified transferrin molecule wherein the transferrin molecule retains the ability to cross the blood brain barrier, but does not retain the ability to bind to the transferrin receptor and transport the therapeutic peptide inside cells.

### Modified Transferrin Fusion Proteins

The fusion of proteins of the invention may contain one or more copies of the GLP-1 protein or polypeptide attached to the N-terminus and/or the C-terminus of the Tf protein. In some embodiments, the GLP-1 protein or polypeptide is attached to both the N- and C-terminus of the Tf protein and the fusion protein may contain one or more GLP-1 molecules on either or both ends of Tf. In other embodiments, the GLP-1 protein or polypeptide is inserted into known domains of the Tf protein, for instance, into one or more of the loops of Tf (see Ali et al. (1999) J. Biol. Chem. 274(34):24066-24073). In fact, the GLP-1 protein or polypeptide may be inserted into all five loops of transferrin to create a pentavalent molecule with increased avidity for the antigen, receptor, or targeting molecule, which the GLP-1 protein binds. In other embodiments, the GLP-1 protein or polypeptide is inserted between the N and C domains of Tf. Alternatively, the therapeutic protein or polypeptide is inserted anywhere in the transferrin molecule.

Generally, the transferrin fusion protein of the invention may have one modified transferrin-derived region and one therapeutic protein region. Multiple regions of each protein, however, may be used to make a transferrin fusion protein of the invention. Similarly, more than one therapeutic protein may be used to make a transferrin fusion protein of the invention, thereby producing a multi-functional modified Tf fusion protein.

In one embodiment, the transferrin fusion protein of the invention contains a GLP-1 molecule fused to a modified transferrin protein. In another embodiment, the transferrin fusion protein of the inventions contains a GLP-1 protein or polypeptide fused to the N terminus of a modified transferrin molecule. In an alternate embodiment, the transferrin fusion protein of the invention contains aGLP-1 protein or polypeptide fused to the C terminus of a modified transferrin molecule. In a further embodiment, the transferrin fusion protein of the invention contains a modified transferrin molecule fused to the N terminus of a GLP-1 protein or polypeptide. In an alternate embodiment, the transferrin fusion protein of the invention contains a modified transferrin molecule fused to the C terminus of a GLP-1 protein or polypeptide.

In other embodiments, the transferrin fusion protein of the inventions contains a GLP-1 protein fused to both the N-terminus and the C-terminus of modified transferrin. Also described herein are therapeutic proteins fused at the N- and C-termini which bind the same therapeutic proteins. The therapeutic proteins fused at the N- and C- termini may be different therapeutic proteins. The therapeutic proteins fused to the N- and C- termini may bind different therapeutic proteins which may be used to treat or prevent the same disease, disorder, or condition. The therapeutic proteins fused at the N-and C- termini may be different therapeutic proteins which may be used to treat or prevent diseases or disorders which are known in the art to commonly occur in patients simultaneously.

In addition to modified transferrin fusion protein of the invention in which the modified transferrin portion is fused to the N terminal and/or C-terminal of the GLP-1 protein portion, transferrin fusion protein of the invention may also be produced by inserting the GLP-1 molecule into an internal region of the modified transferrin. Internal regions of modified transferrin include, but are not limited to, the iron binding sites, the hinge regions, the bicarbonate binding sites, or the receptor binding domain.

Within the protein sequence of the modified transferrin molecule a number of loops or turns exist, which are stabilized by disulfide bonds. These loops are useful for the insertion, or internal fusion, of therapeutically active peptides particularly those requiring a secondary structure to be functional, or therapeutic proteins to generate a modified transferrin molecule with specific biological activity.

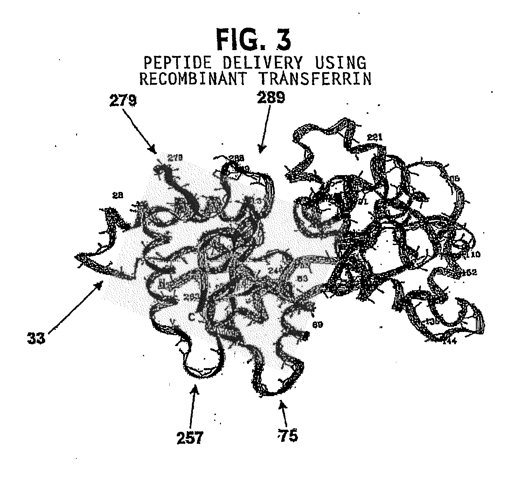

When therapeutic proteins are inserted into or replace at least one loop of a Tf molecule, insertions may be made within any of the surface exposed loop regions, in addition to other areas of Tf. For instance, insertions may be made within the loops comprising Tf amino acids 32-33, 74-75, 256-257, 279-280 and 288-289 (Ali *et al., supra*) (See Figure 3). As previously described, insertions may also be made within other regions of Tf such as the sites for iron and bicarbonate binding, hinge regions, and the receptor binding domain as described in more detail below. The loops in the Tf protein sequence that are amenable to modification/replacement for the insertion of proteins or peptides may also be used for the development of a screenable library of random peptide inserts. Any procedures may be used to produce nucleic acid inserts for the generation of peptide libraries, including available phage and bacterial display systems, prior to cloning into a Tf domain and/or fusion to the ends of Tf.

The N-terminus of Tf is free and points away from the body of the molecule. Fusions of proteins or peptides on the N-terminus may therefore be a preferred embodiment. Such fusions may include a linker region, such as but not limited to a poly-glycine stretch, to separate the therapeutic protein from Tf. Attention to the junction between the leader sequence, the choice of leader sequence, and the structure of the mRNA by codon manipulation/optimization (no major stem loops to inhibit ribosome progress) will increase secretion and can be readily accomplished using standard recombinant protein techniques.

The C-terminus of Tf appears to be more buried and secured by a disulfide bond 6 amino acids from the C-terminus. In human Tf, the C-terminal amino acid is a proline which, depending on the way that it is orientated, will either point a fusion away or into the body of the molecule. A linker or spacer moiety at the C-terminus may be used in some embodiments of the invention. There is also a proline near the N-terminus. In one aspect of the invention, the proline at the N- and/or the C- termini may be changed out. In another aspect of the invention, the C-terminal disulfide bond may be eliminated to untether the C-terminus.

Small molecule therapeutics may be complexed with iron and loaded on a modified Tf protein fusion for delivery to the inside of cells and across the BBB. The addition of a targeting peptide or, for example, a single chain antibody (SCA) can be used to target the payload to a particular cell type, e.g., a cancer cell.

### Therapeutic Proteins and Peptides

Although the invention concerns GLP-1 as the therapeutic protein,

any therapeutic molecule may be used as the fusion partner to Tf. As used herein, a therapeutic molecule is typically a protein or peptide capable of exerting a beneficial biological effect *in vitro* or *in vivo* and includes proteins or peptides that exert a beneficial effect in relation to normal homeostasis, physiology or a disease state. Therapeutic molecules do not include fusion partners commonly used as markers or protein purification aids, such as bacterial galactosidases (see for example, U.S. Patent 5, 986, 067 and Aldred et al. (1984) Biochem. Biophys. Res. Commun. 122: 960-965). For instance, a beneficial effect as related to a disease state includes any effect that is advantageous to the treated subject, including disease prevention, disease stabilization, the lessening or alleviation of disease symptoms or a modulation, alleviation or cure of the underlying defect to produce an effect beneficial to the treated subject.

A modified transferrin fusion protein of the invention includes at least a GLP-1 molecule and at least a fragment or variant of modified serum transferrin, as set out in the claims, which are associated with one another, preferably by genetic fusion.

The transferrin fusion protein may include a modified transferrin molecule linked to a neuropharmaceutical agent. The modified transferrin fusion protein may include transferrin at the carboxyl terminus linked to a neuropharmaceutical agent at the amino terminus. Alternatively, the modified transferrin fusion protein may include transferrin at the amino terminus linked to a neuropharmaceutical agent at the carboxy terminus. Preferably, the neuropharmaceutical agent is either nerve growth factor or ciliary neurotrophic factor.

As described herein, a modified transferrin fusion protein may contain at least a fragment or variant of a therapeutic protein. In a further example, the transferrin fusion proteins can contain peptide fragments or peptide variants of proteins or antibodies wherein the variant or fragment retains at least one biological or therapeutic activity. The transferrin fusion proteins can contain therapeutic proteins that can be peptide fragments or peptide variants at least about 4, at least 5, at least 6, at least 7, at least 8, at least 9, at least 10, at least 11, at least 12, at least 13, at least 14, at least 15, at least 20, at least 25, at least 30, at least 35, or at least about 40, at least about 50, at least about 55, at least about 60 or at least about 70 or more amino acids in length fused to the N and/or C termini, inserted within, or inserted into a loop of a modified transferrin.

The modified transferrin fusion proteins may contain one or more peptides. Increasing the number of peptides enhances the function of the peptides fused to transferrin and the function of the entire transferrin fusion protein. The peptides may be used to make a bi- or multi-functional fusion protein by including peptide or protein domains with multiple functions. For instance, a multi-functional fusion protein can be made with a therapeutic protein and a second protein to target the fusion protein to a specific target. Other peptides may be used to induce the immune response of a cellular system, or induce an antiviral, antibacterial, or anti-pathogenic response.

The modified transferrin fusion molecules may contain a therapeutic protein portion that can be fragments of a therapeutic protein that include the full length protein as well as polypeptides having one or more residues deleted from the amino terminus of the amino acid sequence.

The modified transferrin fusion molecules may contain a therapeutic protein portion that can be fragments of a therapeutic protein that include the full length protein as well as polypeptides having one or more residues deleted from the carboxy terminus of the amino acid sequence.

The modified transferrin fusion molecules may contain a therapeutic protein portion that can have one or more amino acids deleted from both the amino and the carboxy termini.

The modified transferrin fusion molecules may contain a therapeutic protein portion that is at least about 80%, 85%, 90%, 95%, 96%, 97%, 98% or 99% identical to a reference therapeutic protein set forth herein, or fragments thereof. The transferrin fusion molecules may contain a therapeutic protein portion that is at least about 80%, 85%, 90%, 95%, 96%, 97%, 98% or 99% identical to reference polypeptides having the amino acid sequence of N- and C-terminal deletions as described above.

The modified transferrin fusion molecules may contain the therapeutic protein portion that is at least about 80%, 85%, 90%, 95%, 96%, 97%, 98%, 99% or 100%, identical to, for example, the native or wild-type amino acid sequence of a therapeutic protein. Fragments, of these polypeptides are also provided.

The therapeutic proteins corresponding to a therapeutic protein portion of a modified transferrin fusion protein such as cell surface and secretory proteins, can be modified by the attachment of one or more oligosaccharide groups. The modification referred to as glycosylation can significantly affect the physical properties of proteins and can be important in protein stability, secretion, and localization. Glycosylation occurs at specific locations along the polypeptide backbone. There are usually two major types of glycosylation: glycosylation characterized by O-linked oligosaccharides, which are attached to serine or threonine residues; and glycosylation characterized by N-linked oligosaccharides, which are attached to asparagine residues in an Asn-X-Ser/Thr sequence, where X can be an amino acid except proline. Variables such as protein structure and cell type influence the number and nature of the carbohydrate units within the chains at different glycosylation sites. Glycosylation isomers are also common at the same site within a given cell type. For example, several types of human interferon are glycosylated.

Therapeutic proteins corresponding to a therapeutic protein portion of a transferrin fusion protein , as well as analogs and variants thereof, may be modified so that glycosylation at one or more sites is altered as a result of manipulation(s) of their nucleic acid sequence by the host cell in which they are expressed, or due to other conditions of their expression. For example, glycosylation isomers may be produced by abolishing or introducing glycosylation sites, e.g., by substitution or deletion of amino acid residues, such as substitution of glutamine for asparagine, or unglycosylated recombinant proteins may be produced by expressing the proteins in host cells that will not glycosylate them, *e.g.* in glycosylation-deficient yeast. These approaches are known in the art.

Therapeutic proteins and their nucleic acid sequences are well known in the art and available in public databases such as Chemical Abstracts Services Databases (*e.g.,* the CAS Registry), GenBank, and GenSeq.

In other embodiments, the transferrin fusion proteins of the invention are capable of a therapeutic activity and/or biologic activity, corresponding to the therapeutic activity and/or biologic activity of the therapeutic protein described elsewhere in this application.

The present disclosure is further directed to modified Tf fusion proteins comprising fragments of the GLP-1 proteins herein described. Even if deletion of one or more amino acids from the N-terminus of a protein results in modification or loss of one or more biological functions of the therapeutic protein portion, other therapeutic activities and/or functional activities (*e.g.,* biological activities, ability to multimerize, ability to bind a ligand) may still be retained. For example, the ability of polypeptides with N-terminal deletions to induce and/or bind to antibodies which recognize the complete or mature forms of the polypeptides generally will be retained with less than the majority of the residues of the complete polypeptide removed from the N-terminus. Whether a particular polypeptide lacking N-terminal residues of a complete polypeptide retains such immunologic activities can be assayed by routine methods described herein and otherwise known in the art. It is not unlikely that a mutant with a large number of deleted N-terminal amino acid residues may retain some biological or immunogenic activities. In fact, peptides composed of as few as six amino acid residues may often evoke an immune response.

Also as mentioned above, even if deletion of one or more amino acids from the N-terminus or C-terminus of a therapeutic protein results in modification or loss of one or more biological functions of the protein, other functional activities (*e.g.,* biological activities, ability to multimerize, ability to bind a ligand) and/or therapeutic activities may still be retained. For example the ability of polypeptides with C-terminal deletions to induce and/or bind to antibodies which recognize the complete or mature forms of the polypeptide generally will be retained when less than the majority of the residues of the complete or mature polypeptide are removed from the C-terminus. Whether a particular polypeptide lacking the N-terminal and/or, C-terminal residues of a reference polypeptide retains therapeutic activity can readily be determined by routine methods described herein and/or otherwise known in the art.

Peptide fragments of the therapeutic proteins can be fragments comprising, or alternatively, consisting of, an amino acid sequence that displays a therapeutic activity and/or functional activity (*e.g*. biological activity) of the polypeptide sequence of the therapeutic protein of which the amino acid sequence is a fragment.

The peptide fragments of the therapeutic protein may comprise only the N-and C- termini of the protein, *i.e.,* the central portion of the therapeutic protein has been deleted. Alternatively, the peptide fragments may comprise non-adjacent and/or adjacent portions of the central part of the therapeutic protein.

Other polypeptide fragments are biologically active fragments. Biologically active fragments are those exhibiting activity similar, but not necessarily identical, to an activity of a therapeutic protein described herein. The biological activity of the fragments may include an improved desired activity, or a decreased undesirable activity.

Generally, variants of proteins are overall very similar, and, in many regions, identical to the amino acid sequence of the therapeutic protein corresponding to a therapeutic protein portion of a transferrin fusion protein described herein. Nucleic acids encoding these variants are also described herein.

Further therapeutic polypeptides that may be used are polypeptides encoded by polynucleotides which hybridize to the complement of a nucleic acid molecule encoding an amino acid sequence of a therapeutic protein under stringent hybridization conditions which are known to those of skill in the art. (see, for example, Ausubel, F.M. et al., eds., 1989 Current protocol in Molecular Biology, Green Publishing Associates, Inc., and John Wiley & Sons Inc., New. York).

By a polypeptide-having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, or substituted with another amino acid. These alterations of the reference sequence may occur at the amino- or carboxy-terminal positions of the reference amino acid sequence or anywhere between those terminal positions, interspersed either individually among residues in the reference sequence, or in one or more contiguous groups within the reference sequence.

As a practical matter, whether any particular polypeptide is at least about 80%, 85%, 90%, 95%, 96%, 97%, 98% or 99% identical to, for instance, the amino acid sequence of a transferrin fusion protein of the invention or a fragment thereof (such, as the therapeutic protein portion of the transferrin fusion protein or the transferrin portion of the transferrin fusion protein), can be determined conventionally using known computer programs. A preferred method for determining the best overall match between a query sequence (a sequence of the present invention) and a subject sequence, also referred to as a global sequence alignment, can be determined using the FASTDB computer program based on the algorithm of Brufiag et al. (Comp. App. Biosci 245 (1990)).

The polynucleotide variants may contain alterations in the coding regions, non-coding regions, or both. Polynucleotide variants containing alterations which produce silent substitutions, additions, or deletions, but do not alter the properties or activities of the encoded polypeptide may be used to produce modified Tf fusion proteins. Nucleotide variants produced by silent substitutions due to the degeneracy of the genetic code can be utilized. Moreover, polypeptide variants in which less than about 50, less than 40, less than 30, less than 20, less than 10, or 5-50, 5-25, 5-10, 1-5, or 1-2 amino acids are substituted, deleted, or added in any combination can also be utilized. Polynucleotide variants can be produced for a variety of reasons, e.g., to optimize codon expression for a particular host (change codons in the human mRNA to those preferred by a host, such as, yeast or *E. coli* as described above).

In other examples, the therapeutic protein moiety has conservative substitutions compared to the wild-type sequence. By "conservative substitutions" is intended swaps within groups such as replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly. Guidance concerning how to make phenotypically silent amino acid substitutions is provided, for example, in Bowie et al., "Deciphering the Message in Protein Sequences: Tolerance to Amino Acid Substitutions," Science 247:1306-1310 (1990). In specific examples , the polypeptides described herein comprise, or alternatively, consist of, fragments or variants of the amino acid sequence of a therapeutic protein described herein and/or serum transferrin, and/ modified transferrin protein, wherein the fragments or variants have 1-5, 5-10, 5-25, 5-50, 10-50 or 50-150 amino acid residue additions, substitutions, and/or deletions when compared to the reference amino acid sequence. In further examples, the amino acid substitutions are conservative.

The modified fusion proteins of the present invention can be composed of amino- acids joined to each other by peptide bonds or modified peptide bonds and may contain amino acids other than the 20 gene-encoded amino acids. The polypeptides may be modified by either natural processes, such as post-translational processing, or by chemical modification techniques which are well known in the art. Such modifications are well described in basic texts and in more detailed monographs, as well as in a voluminous research literature.

Modifications can occur anywhere in a polypeptide, including the peptide backbone, the amino acid side-chains and the amino or carboxy termini. It will be appreciated that the same type of modification may be present in the same or varying degrees at several sites in a given polypeptide. Also, a given polypeptide may contain many types of modifications. Polypeptides may be branched, for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, glycosylation, GPI anchor formation, hydroxylation, iodination, methylation, myristylation, oxidation, pegylation, proteolytic processing, phosphorylation, prenylation, racemization, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York(1993); POST-TRANSLATIONAL COVALENT MODIFICATION OF PROTEINS, B. C. Johnson, Ed., Academic Press, New York, pgs. 1-12 (1983); Seifter et al. (1990) Meth. Enzymol. 182:626-646; Rattan et al., Ann. N.Y. Acad. Sci. 663:48-62.

The therapeutic protein of the present invention is glucagon-like peptide 1. Other therapeutic proteins described herein include β-interferon (β-IFN), EPO mimetic peptide (EMP-1), T-20, and soluble toxin receptor, such as synaptotagmin I.

### β-interferon

Most cytokines, including β-IFN, have relatively short circulation half-lives since they are produced *in vivo* to act locally and transiently. To use β-IFN as an effective systemic therapeutic, one needs relatively large doses and frequent administrations. Such frequent parenteral administrations are inconvenient and painful. Further, toxic side effects are associated with β-IFN administration which are so severe that some multiple sclerosis patients cannot tolerate the treatment. These side effects are probably associated with administration of a high dosage.

Described herein are β-IFN/transferrin fusion proteins with increased half-lives and pharmaceutical compositions comprising such fusion proteins with increased stability. Such fusion proteins can be administered to patients at lower doses, thus reducing the toxic side effects associated with β-IFN. Described herein is the use of the β-IFN/transferrin fusion proteins to treat various diseases and conditions associated with β-IFN, such as but not limited to multiple sclerosis, cancer including brain tumors and skin cancer, and viral infections such as hepatitis B and C. Preferably, the β-IFN/transferrin fusion proteins are used to treat subjects suffering from multiple sclerosis.

β-interferon (β-IFN) is a glycoprotein with an apparent molecular weight (MW) of 23 kilodaltons. The gene encoding β-IFN is located on chromosome 9. Its amino acid sequence containing 166 residues was determined by K. Hosoi et al. (J. Interferon Res., 8, pp 375-384 (1988)), and its glucoside sequence was reported by Y. Kagawa et al. (J. Biol. Chem., 263, pp 17508-17515 (1988)).

β-IFN is secreted by fibroblasts in response to a viral or bacterial infection, or exposure to foreign cells, macromolecules, or RNA. In particular, β-IFN inhibits the proliferation of infected cells and stimulates the immune system. The specific antiviral activity of homogeneous Hu-β-IFN is considered to be between 3 x 10⁸ and 1 x 10⁹ iu/mg (international units per milligram of total protein) inclusive (see U.S. Pat. No. 4,289,689 and EP-A-94 672).

"Interferon-beta" (IFN-β) or "beta-interferon" (β-IFN) includes native and recombinant Type I interferons exhibiting the same or similar pharmaceutical characteristics as the Type I interferons commonly known as IFN-β-1a and IFN-β-1b.

Any β-IFN sequence may be used to prepare Tf fusion proteins.
For instance, U.S. Patent 4,738,931 discloses the human β-IFN gene derived from human chromosomal DNA. A 1.8 kb EcoRI fragment, containing the nucleic acid encoding the human β-IFN, introduced into *Escherichia coli* has been deposited with the American Type Culture Collection in U.S.A. as *Escherichia coli* CI4 under accession number ATCC 31905. The GenBank accession number for the amino acid sequence of Human β-IFN amino acid sequence is AAA72588. The β-IFN could also be a mutein as described in U.S. Pat. No. 4,588,585, in which the cysteine (Cys) normally occurring at position 17 of the wild-type or native molecule has been replaced by a neutral amino acid, such as serine or alanine. Mark et al. (Proc. Natl. Acad. Sci. USA 81: 5662-5666 (1984)) showed that when Cys 17 was changed for serine, the IFN exhibited the same spectrum of biological activities as β-IFN, such as anticellular and antiproliferative activity, activation of NK cells and neutralization of anti-human IFN antibodies. The mutein also exhibited greater stability than natural human (Hu) β-IFN when incubated at 70°C.

Because of its activity, β-IFN is regarded as an active principle not only in the treatment and prophylaxis of viral diseases such as herpes, influenza *etc,* but also in the treatment of tumoral conditions such as encephaloma and leukemia. β-IFN is used to treat multiple sclerosis, brain tumor, skin cancer and hepatitis B and C. β-IFN fusion proteins may be used to treat any of these diseases.

Human β-IFN is also effective in treating coronary restenosis in humans by selectively inhibiting the proliferation of coronary smooth muscle cell at the site of vascular injury following a surgical procedure while having no inhibitory effect on the normal proliferation of coronary endothelial cells following the procedure. U.S. Patent 5,681,558 discloses a method of treating restenosis comprising administering β-IFN to the patient. Accordingly, β-IFN fusion proteins may be used to treat restenosis.

β-IFN has an erythropoietic effect on the growth of progenitor cells from individuals suffering from several diseases with a very low production of red blood cells. Additionally, β-IFN increases burst formation as well as promotes a more rapid maturation toward normoblasts and even late reticulocytes. U.S. Patent 5,104,653 discloses a method for the stimulation of erythropoiesis in a patient suffering from a disorder characterized by lack of maturation of progenitor blood cells to red blood cells comprising administering to said patient an erythropoietic effective amount of human β-IFN. Therefore, β-IFN fusion proteins may be used to stimulate erythropoiesis.

β-IFN, acting via STAT1 and STAT2, is known to upregulate and downregulate a wide variety of genes, most of which are involved in the antiviral immune response. Although most IFN responses are induced by the presence of dsRNA, both DNA and RNA viruses are sensitive to the effects of β-IFN (Biron, Seminars in Immunology, 10: 383-390 (1998)).

β-IFN is generally produced in response to a viral infection. Interferon β-IFN exerts its biological effects by binding to specific receptors on the surface of human cells. This binding initiates a complex cascade of intracellular events that leads to the expression of numerous interferon-induced gene products and markers, for example, 2', 5'-oligoadenylate synthetase, b₂-microglobulin, and neopterin.

(2'-5')-Oligoadenylate synthetase and dsRNA dependent protein kinase are the two best-known IFN-β-induced proteins (Biron,1998, supra). (2'-5')-oligoadenylate synthetase polymerizes ATP in a unique 2'-5' fashion (Janeway et al., Immunobiology: The Immune System in Health and Disease, 4th Edition, New York, Elsevier Science/Garland Publishing pp 385-386(1999)); the resultant oligomers activate RNase L, which cleaves mRNA (Biron, 1998, supra). dsRNA dependent protein kinase phosphorylates and inactivates elF2, a transcriptional initiator. Both (2'-5')-oligoadenylate synthetase and dsRNA dependent protein kinase act only in the presence of dsRNA, *i*.*e.* in virally infected cells. The net result of the action of these two proteins is to inhibit protein translation, which will retard viral replication (Biron, 1998, supra).

β-IFN dependent upregulation of TAP (transporter associated with antigen processing), Lmp2, Lmp7 serves to increase presentation of viral peptides by MHC class I molecules in order to facilitate CD8 T cell recognition and destruction of infected cells. TAP is the molecule responsible for loading peptide fragments onto MHC class I molecules in the ER; the Lmp proteins are components of the proteasome which cleave proteins specifically for MHC class I presentation (Janeway *et al.,* 1999, supra).

β-IFN is known to both activate and induce some proliferation in natural killer (NK) cells (Janeway *et al.,* 1999, supra). However, interferons themselves are not mitogens. The proliferation of NK cells is probably caused by an intermediary cytokine which is induced by IFN-β (Biron, 1998, supra). NK cells can kill cells which exhibit atypical patterns of MHC class I expression; such cells are generally virally infected (Janeway *et al.,* 1999, supra).

Although at the end of a successfully defeated infection, T cells die by apoptosis as the immune system returns to a homeostatic balance, some T cells must avoid apoptosis and enter a G₀/G₁ memory state to preserve immunological memory. These memory T cells are rescued from apoptosis by interacting with stromal cells, which secrete β-IFN and some IFN-α (Pilling et al., European Journal of Immunology 29:1041-1050 (1999)). T cell apoptosis may be induced by either cytokine deprivation or ligation of Fas on the cell surface, but β-IFN is able to block both apoptotic pathways. The former apoptotic pathway is blocked by β-IFN dependent upregulation of Bcl-x, an apoptotic inhibitor. Fas ligation-induced apoptosis occurs much too quickly to be blocked by upregulation of a gene, so β-IFN must block that apoptotic pathway by different means (Scheel-Toellner et al., European Journal of Immunology 29:2603-2612 (1999)). The existence of a second blocking mechanism is supported by the results of Marrack et al. (Journal of Experimental Medicine 189:521-529(1999)), who found that β-IFN prevented T cell apoptosis without increased production of Bcl-x.

Der et al. (Proc. Nat. Acad. Sci., USA 95: 15623-15628 (1998)) found that β-IFN increased transcription of well over 100 proteins in human fibrosarcoma cells. Induced proteins ranged in function from cytochromes and cell scaffolding proteins to immunologically active proteins such as Complement components and dsRNA adenosine deaminase. These results indicate that β-IFN has truly pleiotropic effects, many of which are not fully understood.

Much clinical research on β-IFN is currently focused on its use as a treatment for multiple sclerosis (MS). MS is an autoimmune disease in which T cells mount an immune response against self myelin antigens in the glial cells of the central nervous system (Goodkin, 1999. Multiple sclerosis: Treatment options for patients with relapsing-remitting and secondary progressive multiple sclerosis. <http://www.msnews.org/goodkin1_99.htm>). In 1993, the FDA approved subcutaneous injections of IFN-β1b for treatment of MS (Revelle M., 1993, FDA licenses interferon beta-1b. (<http://www.fda.gov/bbs/topics/NEWS/NEW00424.html>). β-IFN 1b is a non-glycosylated form of IFN-β produced in *E. coli* (Arduini et al., Protein Science 8: 1867-1877 (1999)). Adverse experiences associated with β-IFN 1b therapy include: injection site reactions (inflammation, pain, hypersensitivity and necrosis), and a flu-like symptom complex (fever, chills, anxiety and confusion). These adverse side effects may be, in fact, reduced or alleviated by fusing β-IFN 1b to transferrin as described above.

Currently, β-IFN 1a (a eukaryotic, glycosylated form) is also available (Goodkin, 1999, supra). β-IFN 1a is produced by recombinant DNA technology. Interferon beta-1a is a 166 amino acid glycoprotein with a predicted molecular weight of approximately 22,500 daltons. It is produced by mammalian cells (Chinese Hamster Ovary cells) into which the human IFN-β gene has been introduced. The amino acid sequence of β-IFN 1a is identical to that of natural human β-IFN and may be used to make Tf fusion proteins.

β-IFN/transferrin fusion proteins treatment may also ameliorate autoimmune attacks by restoring suppressor T cell function; cotreatment with all-trans-retinoic acid seems to increase this restorative action for unknown reasons (Qu *et al.,* 1998. All-trans retinoic acid potentiates the ability of interferon beta-
1b. <http://members.tripod.com/-ThJuland/ra-beta1b.html>). β-IFN may also inhibit the induction of inducible nitric oxide synthase (INOS) expression by IL-1 and IFN-γ. Production of nitric oxide by INOS in astrocytes has been implicated as a factor in the parthenogenesis of MS (Hua *et al.* 1998. Beta inteferon prevents nitric oxide/peroxynitrate from damaging the central nervous system.
(<http://members.tripod.com/-ThJuland/nitric-oxide beta.html>).

Described herein is the use of β-IFN analogs that are therapeutically effective for treating various diseases associated with β-IFN for generating β-IFN/transferrin fusion proteins.

Also described herein is the use of the β-IFN/transferrin fusion protein in the methods described above to inhibit or stimulate various cellular processes and for the treatment and prevention of the various disease and conditions described above. In particular, the β-IFN/transferrin fusion protein may be used to treat multiple sclerosis, herpes, influenza, brain tumor, and skin cancer.

The β-IFN/transferrin fusion protein can be formulated into pharmaceutical compositions by well known methods. See, *e.g.,* Remington's Pharmaceutical Sciences by E. W. Martin which describes suitable formulations. The pharmaceutical composition of the (3-IFN/transferrin fusion protein may be formulated in a variety of forms, including liquid, gel, lyophilized, or any other suitable form. The preferred form will depend upon the particular indication being treated and will be apparent to one of skill in the art.

The β-IFN/transferrin fusion protein can be administered in pure form or in an appropriate pharmaceutical composition. Administration can be carried out via any of the accepted modes. Thus, administration can be, for example, orally, nasally, parenterally, topically, transdermally, or rectally, in the form of solid, semi-solid, lyophilized powder, or liquid dosage forms, such as for example, tablets, suppositories, pills, soft elastic and hard gelatin capsules, powders, solutions, suspensions, or aerosols, or the like, preferably in unit dosage forms suitable for simple administration of precise dosages. The compositions will include a conventional pharmaceutical carrier or excipient and the β-IFN/transferrin fusion protein as the active agent, and, in addition, may include other medicinal agents, pharmaceutical agents, carriers, adjuvants, *etc.*

Generally, depending on the intended mode of administration, the pharmaceutically acceptable compositions will contain about 1% to about 99% by weight of the β-IFN /transferrin fusion protein, and 99% to 1% by weight of a suitable pharmaceutical excipient. The composition could be about 5% to 75% by weight of the β-IFN/transferrin fusion protein with the rest being suitable pharmaceutical excipients.

The route of administration could be parenterally, using a convenient daily dosage regimen which can be adjusted according to the degree of severity of the disease, preferably multiple sclerosis, to be treated. For such parenteral administration, a pharmaceutically acceptable composition containing the β-IFN/transferrin fusion protein may be formed by the methods disclosed in U.S. Pat. Nos. 4,462,940,4,588,585 and 4,992,271.

Alternatively, the β-IFN/transferrin fusion protein pharmaceutical compositions may be administered orally, intravenously, intramuscularly, intraperitoneally, intradermally or subcutaneously or in any other acceptable manner. The preferred mode of administration will depend upon the particular indication being treated and will be apparent to one of skill in the art.

U.S. Patent 6,333,032 describes effective methods of using β-IFN to treat diseases in warm-blooded vertebrates, such as multiple sclerosis. Treatment of multiple sclerosis comprises administering β-IFN at a dosage of 0.01 to about 5 IU/1b per day in a dosage form adapted to promote contact of said dosage of interferon with the oral and pharyngeal mucosa of said animal. The dosage of interferon could be from 0.1 to about 4.0 IU/lb per day, or from 0.5 to about 1.5 IU/1b of body weight per day.

Administering the β-IFN in a dosage form adapted to assure maximum contact of the interferon in said dosage form with the oral and pharyngeal mucosa of the human or animal undergoing treatment is contemplated. Contact of interferon with the mucosa can be enhanced by maximizing residence time of the treatment solution in the oral or pharyngeal cavity. Thus, best results seem to be achieved in human patients when the patient is requested to hold said solution of interferon in the mouth for a period of time. Contact of interferon with the oral and pharyngeal mucosa and thereafter with the lymphatic system of the treated human or animal is unquestionably the most efficient method administering immunotherapeutic amounts of interferon.

Further, the use of the β-IFN/transferrin protein for the manufacture of a medicament which is useful for the treatment of diseases associated with β-IFN is contemplated. The diseases contemplated include but are not limited to those described above.

### Glucagon-Like Peptide-1 (GLP-1)

Glucagon-Like Peptide-1 (GLP-1) is a gastrointestinal hormone that regulates insulin secretion belonging to the so-called enteroinsular axis. The enteroinsular axis designates a group of hormones, released from the gastrointestinal mucosa in response to the presence and absorption of nutrients in the gut, which promote an early and potentiated release of insulin. The incretin effect which is the enhancing effect on insulin secretion is probably essential for a normal glucose tolerance. GLP-1 is a physiologically important insulinotropic hormone because it is responsible for the incretin effect.

GLP-1 is a product of proglucagon (Bell, et al., Nature, 1983, 304: 368-371). It is synthesized in intestinal endocrine cells in two principal major molecular forms, as GLP-1(7-36)amide and GLP-1(7-37). The peptide was first identified following the cloning of cDNAs and genes for proglucagon in the early 1980s.

Initial studies done on the full length peptide GLP-1(1-37) and GLP-1(1-36^{amide}) concluded that the larger GLP-1 molecules are devoid of biological activity. In 1987, three independent research groups demonstrated that removal of the first six amino acids resulted in a GLP-1 molecule with enhanced biological activity.

The amino acid sequence of GLP-1 is disclosed by Schmidt et al. (1985 Diabetologia 28 704-707). Human GLP-1 is a 37 amino acid residue peptide originating from preproglucagon which is synthesized in the L-cells in the distal ileum, in the pancreas, and in the brain. Processing of preproglucagon to GLP-1 (7-36^{amide}), GLP-1(7-37) and GLP-2 occurs mainly in the L-cells. The amino acid sequence of GLP-1(7-36 ^{amide}) and GLP-1(7-37) is (SEQ ID NO: 6): wherein X is NH₂ for GLP-1(7-36 ^{amide}) and X is Gly for GLP-1(7-37).

GLP-1 like molecules possesses anti-diabetic activity in human subjects suffering from Type II (non-insulin-dependent diabetes mellitus (NIDDM)) and, in some cases, even Type I diabetes. Treatment with GLP-1 elicits activity, such as increased insulin secretion and biosynthesis, reduced glucagon secretion, delayed gastric emptying, only at elevated glucose levels, and thus provides a potentially much safer therapy than insulin or sulfonylureas. Post-prandial and glucose levels in patients can be moved toward normal levels with proper GLP-1 therapy. There are also reports suggesting GLP-1-like molecules possess the ability to preserve and even restore pancreatic beta cell function in Type-II patients.

Any GLP-1 sequence may be used to make Tf fusion proteins of the present invention, including GLP-1(7-35), GLP-1(7-36), and GLP-1(7-37). GLP-1 also has powerful actions on the gastrointestinal tract. Infused in physiological amounts, GLP-1 potently inhibits pentagastrin-induced as well as meal-induced gastric acid secretion (Schjoldager et al., Dig. Dis. Sci. 1989, 35:703-708; Wettergren et al., Dig Dis Sci 1993; 38:665-673). It also inhibits gastric emptying rate and pancreatic enzyme secretion (Wettergren et al., Dig Dis Sci 1993; 38:665-673). Similar inhibitory effects on gastric and pancreatic secretion and motility may be elicited in humans upon perfusion of the ileum with carbohydrate- or lipid-containing solutions (Layer et al., Dig Dis Sci 1995, 40:1074-1082; Layer et al., Digestion 1993, 54: 385-38). Concomitantly, GLP-1 secretion is greatly stimulated, and it has been speculated that GLP-1 may be at least partly responsible for this so-called "ileal-brake" effect (Layer et al., Digestion 1993; 54: 385-38). In fact, recent studies suggest that, physiologically, the ileal-brake effects of GLP-1 may be more important than its effects on the pancreatic islets. Thus, in dose response studies GLP-1 influences gastric emptying rate at infusion rates at least as low as those required to influence islet secretion (Nauck et al., Gut 1995; 37 (suppl. 2): A124).

GLP-1 seems to have an effect on food intake. Intraventricular administration of GLP-1 profoundly inhibits food intake in rats (Schick et al. in Ditschuneit et al. (eds.), Obesity in Europe, John Libbey & Company ltd, 1994; pp. 363-367; Turton et al., Nature 1996, 379: 69-72). This effect seems to be highly specific. Thus, N-terminally extended GLP-1(PG 72-107) amide is inactive and appropriate doses of the GLP-1 antagonist, exendin 9-39, abolish the effects of GLP-1(Tang-Christensen et al., Am. J. Physiol., 1996,271(4 Pt 2):R848-56). Acute, peripheral administration of GLP-1 does not inhibit food intake acutely in rats (Tang-Christensen et al., Am. J. Physiol., 1996,271(4 Pt 2):R848-56; Turton et al., Nature 1996, 379: 69-72). However, it remains possible that GLP-1 secreted from the intestinal L-cells may also act as a satiety signal.

In diabetic patients, GLP's insulinotropic effects and the effects of GLP-1 on the gastrointestinal tract are preserved (Willms et al, Diabetologia 1994; 37, suppl.1: A118), which may help curtail meal-induced glucose excursions, but, more importantly, may also influence food intake. Administered intravenously, continuously for one week, GLP-1 at 4 ng/kg/min has been demonstrated to dramatically improve glycaemic control in NIDDM patients without significant side effects (Larsen et al., Diabetes 1996; 45, suppl. 2: 233A.).

GLP-1/transferrin fusion proteins comprising at least one analog of GLP-1 and fragments thereof are useful in the treatment of Type 1 and Type 2 diabetes and obesity.

As used herein, the term "GLP-1 molecule" means GLP-1, a GLP-1 analog, or GLP-1 derivative.

As used herein, the term "GLP-1 analog" is defined as a molecule having one or more amino acid substitutions, deletions, inversions, or additions compared with GLP-1. Many GLP-1 analogs are known in the art and include, for example, GLP-1(7-34), GLP-1(7-35), GLP-1(7-36), Val⁸ -GLP-1(7-37), Gly⁸-GLP-1(7-37), Ser⁸-GLP-1(7-37), Gln⁹-GLP1(7-37), D-Gln⁹-GLP-1(7-37), Thr¹⁶-Lys¹⁸-GLP-1(7-37), and Lys¹⁸-GLP-1(7-37). Other analogs include dipeptidyl-peptidase resistant versions of GLP-1, wherein the N-terminal end of the peptide is protected. Such analogs include, but are not limited to GLP-1 with additional amino acids, such as histidine residue added to the N-terminal end or substituted into the N-terminal amino acids (amino acid 7 or 8 in GLP-1(7-36) or GLP-1(7-37). In these analogs, the N-terminal end may comprise the residues His-His-Ala, Gly-His-Ala, His-Gly-Glu, His-Ser-Glu, His-Ala-Glu, His-Gly-Glu, His-Ser-Glu, His-His-Ala-Glu, His-His-Gly-Glu, His-His-Ser-Glu, Gly-His-Ala-Glu, Gly-His-Gly-Glu, Gly-His-Ser-Glu, His-X-Ala-Glu, His-X-Gly-Glu, His-X-Ser-Glu, wherein X is any amino acid. U.S. Patent 5,118,666 discloses examples of GLP-1 analogs such as GLP-1(7-34) and GLP-1(7-35).

The term "GLP-1 derivative" is defined as a molecule having the amino acid sequence of GLP-1 or a GLP-1 analog, but additionally having chemical modification of one or more of its amino acid side groups, α-carbon atoms, terminal amino group, or terminal carboxylic acid group. A chemical modification includes, but is not limited to, adding chemical moieties, creating new bonds, and removing chemical moieties.

As used herein, the term "GLP-1 related compound" refers to any compound falling within the GLP-1, GLP-1 analog, or GLP-1 derivative definition.

WO 91/11457 discloses analogs of the active GLP-1 peptides 7-34, 7-35, 7-36, and 7-37 which can also be useful as GLP-1 moieties.

EP 0708179-A2 (Eli Lilly & Co.) discloses GLP-1 analogs and derivatives that include an N-terminal imidazole group and optionally an unbranched C₆ -C₁₀ acyl group in attached to the lysine residue in position 34.

EP 0699686-A2 (Eli Lilly & Co.) discloses certain N-terminal truncated fragments of GLP-1 that are reported to be biologically active.

U.S. Patent 5,545,618 discloses GLP-1 molecules consisting essentially of GLP-1(7-34), GLP1(7-35), GLP-1(7-36), or GLP-1(7-37), or the amide forms thereof, and pharmaceutically-acceptable salts thereof, having at least one modification selected from the group consisting of: (a) substitution of glycine,serine, cysteine, threonine, asparagine, glutamine, tyrosine, alanine, valine, isoleucine, leucine, methionine, phenylalanine, arginine, or D-lysine for lysine at position 26 and/or position 34; or substitution of glycine, serine, cysteine, threonine, asparagine, glutamine, tyrosine, alanine, valine, isoleucine, leucine, methionine, phenylalanine, lysine, or a D-arginine for arginine at position 36; (b) substitution of an oxidation-resistant amino acid for tryptophan at position 31; (c) substitution of at least one of: tyrosine for valine at position 16; lysine for serine at position 18; aspartic acid for glutamic acid at position 21; serine for glycine at position 22; arginine for glutamine at position 23; arginine for alanine at position 24; and glutamine for lysine at position 26; and (d) substitution of at least one of: glycine, serine, or cysteine for alanine at position 8; aspartic acid, glycine, serine, cysteine, threonine, asparagine, glutamine, tyrosine, alanine, valine, isoleucine, leucine, methionine, or phenylalanine for glutamic acid at position 9; serine, cysteine, threonine, asparagine, glutamine, tyrosine, alanine, valine, isoleucine, leucine, methionine, or phenylalanine for glycine at position 10; and glutamic acid for aspartic acid at position 15; and (e) substitution of glycine, serine, cysteine, threonine, asparagine, glutamine, tyrosine, alanine, valine, isoleucine, leucine, methionine, or phenylalanine, or the D- or N-acylated or alkylated form of histidine for histidine at position 7; wherein, in the substitutions is (a), (b), (d), and (e), the substituted amino acids can optionally be in the D-form and the amino acids substituted at position 7 can optionally be in the N-acylated or N-alkylated form.

U.S. Pat. No. 5,118,666 discloses a GLP-1 molecule having insulinotropic activity. Such molecule is selected from the group consisting of a peptide having the amino acid sequence His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys (SEQ ID NO: 7) or His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly (SEQ ID NO: 8); and a derivative of said peptide and wherein said peptide is selected from the group consisting of: a pharmaceutically-acceptable acid addition salt of said peptide; a pharmaceutically-acceptable carboxylate salt of said peptide; a pharmaceutically-acceptable lower alkylester of said peptide; and a pharmaceutically-acceptable amide of said peptide selected from the group consisting of amide, lower alkyl amide, and lower dialkyl amide.

U.S. Patent 6,277,819 teaches a method of reducing mortality and morbidity after myocardial infarction comprising administering GLP-1, GLP-1 analogs, and GLP-1 derivatives to the patient. The GLP-1 analog being represented by the following structural formula (SEQ ID NO: 9): R₁-X₁-Glu-Gly-Thr-Phe-Thr-Ser-Asp-Val-Ser-Ser-Tyr-Leu-X₂-Gly-Gln-Ala-Ala-Lys- X₃-Phe-Ile-Ala-Trp-Leu-Val-Lys-Gly-Arg-R₂ and pharmaceutically-acceptable salts thereof, wherein: R₁ is selected from the group consisting of L-histidine, D-histidine, desamino-histidine, 2-amino-histidine, .beta.-hydroxy-histidine, homohistidine, alpha-fluoromethyl-histidine, and alpha-methyl-histidine; X₁ is selected from the group consisting of Ala, Gly, Val, Thr, Ile, and alphamethyl-Ala; X₂ is selected from the group consisting of Glu, Gin, Ala, Thr, Ser, and Gly; X₃ is selected from the group consisting of Glu, Gln, Ala, Thr, Ser, and Gly; R₂ is selected from the group consisting of NH₂, and Gly--OH; provided that the GLP-1 analog has an isoelectric point in the range from about 6.0 to about 9.0 and further providing that when R₁ is His, X₁ is Ala, X₂ is Glu, and X₃ is Glu, R₂ must be NH₂.

Ritzel et al. (Journal of Endocrinology, 1998, 159: 93-102) disclose a GLP-1 analog, [Ser⁸]GLP-1, in which the N-terminal second amino acid, alanine, is replaced with serine. The modification did not impair the insulinotropic action of the peptide but produced an analog with increased plasma stability as compared to GLP-1.

U.S. Patent 6,429,197 teaches that GLP-1 treatment after acute stroke or hemorrhage, preferably intravenous administration, can be an ideal treatment because it provides a means for optimizing insulin secretion, increasing brain anabolism, enhancing insulin effectiveness by suppressing glucagon, and maintaining euglycemia or mild hypoglycemia with no risk of severe hypoglycemia or other adverse side effects. The present invention provides a method of treating the ischemic or reperfused brain with GLP-1 or its biologically active analogues after acute stroke or hemorrhage to optimize insulin secretion, to enhance insulin effectiveness by suppressing glucagon antagonism, and to maintain euglycemia or mild hypoglycemia with no risk of severe hypoglycemia.

U.S. Patent 6,277,819 provides a method of reducing mortality and morbidity after myocardial infarction, comprising administering to a patient in need thereof, a compound selected from the group consisting of GLP-1, GLP-1 analogs, GLP-1 derivatives and pharmaceutically-acceptable salts thereof, at a dose effective to normalize blood glucose.

U.S. Patent 6,191,102 discloses a method of reducing body weight in a subject in need of body weight reduction by administering to the subject a composition comprising a glucagon-like peptide-1 (GLP-1), a glucagon-like peptide analog (GLP-1 analog), a glucagon-like peptide derivative (GLP-1 derivative) or a pharmaceutically acceptable salt thereof in a dose sufficient to cause reduction in body weight for a period of time effective to produce weight loss, said time being at least 4 weeks.

GLP-1 is fully active after subcutaneous administration (Ritzel et al., Diabetologia 1995; 38: 720-725), but is rapidly degraded mainly due to degradation by dipeptidyl peptidase IV-like enzymes (Deacon et al., J Clin Endocrinol Metab 1995, 80: 952-957; Deacon et al., 1995, Diabetes 44: 1126-1131). Thus, unfortunately, GLP-1 and many of its analogues have a short plasma half-life in humans (Orskov et al., Diabetes 1993; 42:658-661). Accordingly, it is an objective of the present invention to provide transferrin fusion proteins comprising GLP-1 or analogues thereof which have a protracted profile of action relative to GLP-1(7-37). It is a further object of the disclosure to provide derivatives of GLP-1 and analogues thereof which have a lower clearance than GLP-1(7-37). Moreover, it is an object of the invention to provide pharmaceutical compositions comprising GLP-1/transferrin fusion proteins or GLP-1 analog/transferrin fusion proteins with improved stability. Additionally, the present invention includes GLP-1/transferrin fusion proteins or GLP-1 analog/transferrin fusion proteins for use to treat diseases associated with GLP-1 such as but not limited to those described above.

In one aspect of the present invention, the pharmaceutical compositions comprising the GLP-1 peptide/transferrin fusion proteins and GLP-1 analog/transferrin fusion proteins may be formulated by any of the established methods of formulating pharmaceutical compositions, *e.g.* as described in Remington's Pharmaceutical Sciences, 1985. The composition may be in a form suited for systemic injection or infusion and may, as such, be formulated with a suitable liquid vehicle such as sterile water or an isotonic saline or glucose solution. The compositions may be sterilized by conventional sterilization techniques which are well known in the art. The resulting aqueous solutions may be packaged for use or filtered under aseptic conditions and lyophilized, the lyophilized preparation being combined with the sterile aqueous solution prior to administration. The composition may contain pharmaceutically acceptable auxiliary substances as required to approximate physiological conditions, such as buffering agents, tonicity adjusting agents and the like, for instance sodium acetate, sodium lactate, sodium chloride, potassium chloride, calcium chloride, *etc.*

The GLP-1/transferrin fusion proteins and GLP-1 analog/transferrin fusion proteins of the present invention may also be adapted for nasal, transdermal, pulmonal or rectal administration. The pharmaceutically acceptable carrier or diluent employed in the composition may be any conventional solid carrier. Examples of solid carriers are lactose, terra alba, sucrose, talc, gelatin, agar, pectin, acacia, magnesium stearate and stearic acid. Similarly, the carrier or diluent may include any sustained release material known in the art, such as glyceryl monostearate or glyceryl distearate, alone or mixed with a wax.

It may be of particular advantage to provide the composition of the invention in the form of a sustained release formulation. As such, the composition may be formulated as microcapsules or microparticles containing the GLP-1/transferrin fusion protein or GLP-1 analog/transferrin fusion protein encapsulated by or dispersed in a suitable pharmaceutically acceptable biodegradable polymer such as polylactic acid, polyglycolic acid or a lactic acid/glycolic acid copolymer.

For nasal administration, the preparation may contain GLP-1/transferrin fusion proteins or GLP-1 analog/transferrin fusion proteins dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

Generally, the compounds of the present invention are dispensed in unit dosage form comprising 0.5-500 mg of the fusion protein together with a pharmaceutically acceptable carrier per unit dosage.

Moreover, the present invention contemplates the use of the GLP-1/transferrin and GLP-1 analog/transferrin fusion proteins for the manufacture of a medicinal product which can be used in the treatment of diseases associated with elevated glucose level, such as but not to limited to those described above. Specifically, the present invention contemplates the GLP-1/transferrin fusion protein for use in the treatment of diabetes including type II diabetes, obesity, severe burns, and heart failure, including congestive heart failure and acute coronary syndrome.

The N-terminus of GLP-1 is normally amidated. In yeast, amidation does not occur. In one embodiment of the invention, in order to compensate for amidation on the N-terminus which does not occur in yeast, an extra amino acid is added on the N-terminus of GLP-1. The addition of an amino acid to the N-terminus of GLP-1 may prevent dipeptidyl peptidase from cleaving at the second amino acid of GLP-1 due to steric hindrance. Therefore, GLP-1 will remain functionally active. Any one of the 20 amino acids may be added to the N-terminus of GLP-1. In some instances, an uncharged or positively charged amino acid may be used and preferably, a smaller amino acid such as Glycine is added. The GLP-1 with the extra amino acid is then fused to transferrin. Accordingly, the GLP-1 with the added amino acid will be fused at the N-terminus of the GLP-1/transferrin fusion protein.

In one embodiment of making the GLP-1(7-36) or GLP-1(7-37) peptide more resistant to cleavage by dipeptidyl peptidase, a His residue is added at the N-terminus of GLP-1 or is inserted after the His residue at the N-terminus of GLP-1, so that the N-terminus of GLP-1 begins with His-His.

In another embodiment of the invention, the second residue from the N-terminus in the GLP-1(7-36) or GLP-1(7-37) peptide (SEQ ID NO: 6) is substituted with another amino acid. For example, the Ala residue at the second residue from the N-terminus in the GLP-1(7-36) or GLP-1(7-37) peptide may be substituted with Ser, Gly, Val, or another amino acid.

### GLP-mTf Fusion Protein for Treating Type 2 Diabetes

As discussed above, GLP-1 activates and regulates important endocrine hormone systems in the body and plays a critical management role in the metabolism of glucose. Unlike all other diabetic treatments on the market GLP-1 has the potential to be restorative by acting as a growth factor for B-cells thus improving the ability of the pancreas to secrete insulin and also, to make the existing insulin levels act more efficiently by improving sensitivity and better stabilizing glucose levels. This reduces the burden on daily monitoring of glucose levels and potentially offers a delay in the serious long term side effects caused by fluctuations in blood glucose due to diabetes. Furthermore, GLP-1 can reduce appetite and reduce weight. Obesity is an inherent consequence of poor control of glucose metabolism and this only serves to aggravate the diabetic condition.

Clinical application of natural GLP-1 is limited because it is rapidly degraded in the circulation (half-life is several minutes). To maintain therapeutic levels in the circulation requires constant administration of high doses using pumps or patch devices which adds to the cost of treatment. This is inconvenient for long term chronic use especially in conjunction with all the other medications for treating diabetes and monitoring of glucose levels. The GLP-1 fusion proteins retain the activity of GLP-1 but have the long half-life (14-17 days), solubility, and biodistribution properties of transferrin (mTf). These properties could provide for a low cost, small volume, monthly s.c. (subcutaneous) injection and this type of product is absolutely needed for long term chronic use.

### Insulin

Human insulin contains two peptide chains, known as the A and B chains, which are 21 and 30 amino acids in length, respectively, and which are connected by two cystine disulphide bridges. This peptide has a molecular weight of approximately 6 kDa. The immediate precursor of insulin is proinsulin, a single chain peptide composed of the B and A chains linked to a connecting peptide of approximately 31 amino acids, known as the C-peptide, by adjacent pairs of basic residues. The arrangement of these three peptides in the proinsulin molecule, beginning with the amino-terminal end, is as follows: B chain-Arg-Arg-C-peptide-Lys-Arg-A chain. When translated into mRNA, however, preproinsulin is produced, which contains proinsulin joined at its amino-terminal end to a largely hydrophobic signal peptide 24 amino acids in length.

Preproinsulin is synthesized in pancreatic beta cells located within the islets of Langerhans, which are dispersed throughout the pancreas. Removal of the signal peptide occurs in the rough endoplasmic reticulum, and the resulting proinsulin is then transported to the Golgi apparatus for packaging into secretion granules. The folded proinsulin is stabilized by disulfide bonds. During processing of the secretion granules, the folded proinsulin molecule is cleaved by specific proteases at the paired basic residues to liberate insulin and the C-peptide.

Diabetes mellitus is a disease that affects approximately 17 million people in the United States, or 6.2% of the population. About one million people over the age of 20 are diagnosed with diabetes annually, and diabetes is the sixth leading cause of death in the United States
(http://www.niddk.nih.gov/health/diabetes/pubs/dmstats/dmstats.htm#7).
Approximately 90-95% of diabetes cases are Type 2, formerly called adult-onset diabetes, which begins as insulin resistance (failure of the body's cells to use insulin properly) as progresses to an inability of the pancreas to produce insulin. Type 1 diabetes, formerly called juvenile diabetes or insulin-dependent diabetes, accounts for the remaining 5-10% of diabetes cases. In type 1 diabetes, the body's immune system destroys the beta cells in the pancreas, which manufacture insulin.

Because human insulin contains only 51 amino acid residues, it is readily made by recombinant techniques, and a large number of insulin analogues and variants have been prepared. Any of these analogues or variants can be used to make mTf fusion proteins as described herein.

Diabetics typically require insulin replacement therapy, which involves one or more doses of the drug per day by subcutaneous injection. Treatment by injection, however, is both psychologically and physically painful, as well as demanding of technical expertise, and many diabetics require assistance in administering injections. Oral formulations of insulin have not been successful, however, because the peptide is rapidly degraded in the acidic environment of the GI tract, particularly in the stomach. Nevertheless, alternatives to injection, such as oral, nasal and topical formulations have been attempted. U.S. Patent 5,824,638, Burnside et al.*,* describes oral emulsion preparations in which insulin is dissolved in a hydrophilic phase, such as water, saline or a water-miscible alcohol, and dispersed with a surfactant in a hydrophobic phase, such as a long chain fatty acid or fatty acid ester. Although an emulsion keeps insulin dispersed, it cannot protect the peptide from the harsh conditions of the stomach. Nasal preparations, which deliver insulin in an aerosol to the lungs, are disclosed in U.S. Patent 6,427,681, Gonda et al.*,* while topical preparations are disclosed in U.S. Patent 6,399,566, Dardai et al.

Modified insulins for injection, containing amino acid substitutions or glycosylated residues, to enhance activity, inhibit degradation or inhibit peptide aggregation have also been developed (see U.S. Patent 4,478,746, Kim et al.*,* glycosylated insulin derivatives; U.S. Patent 4,992,418, Katsoyanis et al.*,* Asp₁₀-containing insulin (B chain) for increased activity; U.S. Patent 5,716,927, Balschmidt et al.*,* Lys or Arg at position 28 in the B chain, or A18, A21 or B3 modified from Asn, or other modifications at the C-terminal end of the B chain, to prevent aggregation and reduced activity) Additional amino acid substitutions that confer a longer active phase, because they can be acylated, are disclosed in U.S. Patent 5,750,497, Havelund et al. A21 B3 and B30 can be replaced by any amino acid except Lys, Arg or Cys. B1 may be deleted, and B30 may be replaced by a lipophilic chain of 10-24 carbon atoms. Fusion proteins for improved recombinant production of insulin (higher yields, soluble, allowing correct folding) are described in U.S. Patent 6,534,288, Habermann et al. These peptides contain a fusion portion at the amino terminal end of the B chain, followed by amino acids RDVP-Yₙ-A chain, where Y is a peptide 2-50 amino acids in length, terminating with a basic amino acid.

Described herein are fusion proteins comprising transferrin and an insulin protein or peptide. The fusion proteins may be formulated for oral delivery. Methods of orally administering insulin fusion proteins to a patient in need thereof, in particular, a diabetic patient are contemplated.

Also described are transferrin fusion protein comprising single chain insulin analog (Lee et al., 2000, Nature, 408: 483). The insulin in the transferrin fusion protein may contain a protease cleavage site specific to the gastrointestinal (GI) tract, or a specific part of the gastrointestinal tract, such that the site would be recognized by one or more enzymes in the GI tract. The proinsulin could be activated in this manner. The cleavage site could reside in the peptide linking the A and B chain.

### EPO Mimetic Peptide (EMP)

Erythropoietin (EPO) is a glycoprotein hormone that is synthesized in the kidneys of mammals for stimulating mitotic cell division and differentiation of erythrocyte precursor cells. Accordingly, EPO acts to stimulate and regulate the production of erythrocytes. Because of its role in red blood cell formation, EPO is useful in both the diagnosis and the treatment of blood disorders characterized by low or defective red blood cell production.

Studies have shown the efficacy of EPO therapy in a variety of disease states, disorders, and states of hematologic irregularity, for example, beta-thalassemia (Vedovato et al. (1984) Acta. Haematol. 71:211-213); cystic fibrosis (Vichinsky et al. (1984) J. Pediatric 105:15-21); pregnancy and menstrual disorders (Cotes et al. (1983) Brit. J. Ostet. Gyneacol. 90:304-311); early anemia of prematurity (Haga et al. (1983) Acta Pediatr. Scand. 72:827-831); spinal cord injury (Claus-Walker et al. (1984) Arch. Phys. Med. Rehabil. 65:370-374); space flight (Dunn et al. (1984) Eur. J. Appl. Physiol. 52:178-182); acute blood loss (Miller et al. (1982) Brit. J. Haematol. 52:545-590); aging (Udupa et al. (1984) J. Lab. Clin. Med. 103:574-588); various neoplastic disease states accompanied by abnormal erythropoiesis (Dainiak et al. (1983) Cancer 5:1101-1106); and renal insufficiency (Eschbach et al. (1987) N. Eng. J. Med. 316:73-78). During the last fifteen years, EPO has been used for the treatment of the anemia of renal failure, anemia of chronic disease associated with rheumatoid arthritis, inflammatory bowel disease, AIDS, and cancer, as well as for the treatment of anemia in hematopoietic malignancies, post-bone marrow transplantation, and autologous blood donation.

The activity of EPO is mediated by its receptor. The EPO-receptor (EPO-R) belongs to the class of growth-factor-type receptors which are activated by a ligand-induced protein dimerization. Other hormones and cytokines such as human growth hormone (hGH), granulocyte colony stimulating factor (G-CSF), epidermal growth factor (EGF) and insulin can cross-link two receptors resulting in juxtaposition of two cytoplasmic tails. Many of these dimerization-activated receptors have protein kinase domains within the cytoplasmic tails that phosphorylate the neighboring tail upon dimerization. While some cytoplasmic tails lack intrinsic kinase activity, these function by association with protein kinases. The EPO receptor is of the latter type. In each case, phosphorylation results in the activation of a signaling pathway.

There has been an increasing interest in molecular mimicry with EPO potency. For example, dimerization of the erythropoietin receptor (EPOR) in the presence of either natural EPO or synthetic EPO mimetic peptides (EMPs) is the extracellular event that leads to activation of the receptor and downstream signal transduction events. In general, there is an interest in obtaining mimetics with equivalent potency to EPO.

Wrighton et al (1996, Science, 273:458-463) employed phage display where random peptides are to be exposed on coat proteins of filamentous phage. A library of random peptide-phage was allowed to bind to and subsequently eluted from the extracellular domain of EPO receptor in the screening system. They used weak-binding system to first fish out EPO domain-weak-binding (Kd 10 mM) CRIGPITWVC (SEQ ID NO: 10) as the consensus sequence. Consequently, a 20-amino acid peptide, EMP1, (GGTYSCHFGPLTWVCKPQGG, SEQ ID NO: 11) with an affinity (Kd) of 200 nM, compared to 200 pM for EPO was isolated, the sequence of which does not actually exist in the native EPO. The crystal structure at 2.8 Å resolution of a complex of this mimetic agonist peptide with the extracellular domain of EPO receptor revealed that a peptide dimer induces an almost perfect twofold dimerization of the receptor (Livnah et al., 1996 Science, 273 (274): 464-471). This 20-amino acid peptide has a b-sheet structure and is stabilized by the C-C disulfide bond.

The biological activity of EMP1 indicates that EMP1 can act as an EPO mimetic. For example, EMP1 competes with EPO in receptor binding assays to cause cellular proliferation of cell lines engineered to be responsive to EPO (Wrighton et al., 1996, Science, 273:458-463). Both EPO and EMP1 induce a similar cascade of phosphorylation events and cell cycle progression in EPO responsive cells (Wrighton et al., 1996, Science, 273:458-463). Further, EMP1 demonstrates significant erythropoietic effects in mice as monitored by two different *in vivo* assays of nascent red blood cell production (Wrighton et al., 1996, Science, 273:458-463).

Johnson et al. (1998, Biochemistry, 37:3699-3710) identified the minimal peptide that retained activity in the assays for EPO mimetic action. Using N- and C-terminal deletions, they found that the minimal active peptide is EMP20 having the sequence, YSCHFGPLTWVCK (SEQ ID NO: 12), namely amino acids 4 through 16 of EMP1. They also found Tyr4 and Trpl3 of EMP1 are critical for mimetic action.

Described herein are EMP1/transferrin fusion proteins with increased half-life and pharmaceutical compositions comprising such fusion proteins. Any EMP1 sequence may be used to make EMP1/transfernn fusion proteins, including EMP1 sequences wherein one or more C residues is deleted or replaced. These sequences can then be inserted into a mTf loop to provide three dimensional structure to the EMP1 region of the fusion protein. The use of the fusion protein to treat various diseases and conditions associated with EPO such as but not limited to those described above is contemplated.

The pharmaceutical compositions comprising the EMP1/transferrin fusion protein may be formulated by any of the established methods of formulating pharmaceutical compositions, *e.g*. as described in Remington's Pharmaceutical Sciences, 1985. The composition may be in a form suited for systemic injection or infusion and may, as such, be formulated with a suitable liquid vehicle such as sterile water or an isotonic saline or glucose solution. These pharmaceutical compositions may contain buffers, salts and other excipients to stabilize the composition or assist in the delivery of the transferrin fusion proteins.

Described herein is a method for treating disorders associated with EPO. The method is accomplished by administering a EMP1/transferrin fusion protein provided herein for a time and under conditions sufficient to alleviate the symptoms of the disorder, *i.e*. sufficient to effect dimerization or biological activation of EPO receptors. In the case of EPO such methodology is useful in the treatment of end-stage renal failure/dialysis; anemia, especially associated with AIDS or chronic inflammatory diseases such as rheumatoid arthritis and chronic bowel inflammation; auto-immune disease; and for boosting the red blood cell count of patient when necessary, e.g. prior to surgery or as pretreatment to transfusion. The EMP1/transferrin fusion protein described herein which behave as EPO agonists can be used to activate megakaryocytes.

Since EPO has been shown to have a mitogenic and chemotactic effect on vascular endothelial cells as well as an effect on central cholinergic neurons (Amagnostou et al. (1990) Proc. Natl. Acad. Sci. USA 87:597805982; Konishi et al. (1993) Brain Res. 609:29-35), the compounds described herein can also be used to treat a variety of vascular disorders, such as promoting wound healing, growth of collateral coronary blood vessels (such as those that may occur after myocardial infarction), trauma, and post vascular graft treatment, and a variety of neurological disorders, generally characterized by low absolute levels of acetyl choline or low relative levels of acetyl choline as compared to other neuroactive substances *e.g*., neurotransmitters.

Also described are pharmaceutical compositions comprising, as an active ingredient, the EMP1/transferrin fusion protein described herein in association with a pharmaceutical carrier or diluent. The EMP1/transferrin fusion protein described herein can be administered by oral, parenteral (intramuscular, intraperitoneal, intravenous (IV) or subcutaneous injection), transdermal (either passively or using iontophoresis or electroporation) or transmucosal (nasal, vaginal, rectal, or sublingual) routes of administration in dosage forms appropriate for each route of administration.

Solid dosage forms for oral administration include capsules, tablets, pill, powders, and granules. In such solid dosage forms, the active compound is admixed with at least one inert pharmaceutically acceptable carrier such as sucrose, lactose, or starch. Such dosage forms can also comprise, as it normal practice, additional substances other than inert diluents, *e.g.,* lubricating, agents such as magnesium stearate. In the case of capsules, tablets and pills, the dosage forms may also comprise buffering, agents. Tablets and pills can additionally be prepared with enteric coatings.

Liquid dosage forms for oral administration include pharmaceutically acceptable emulsions, solutions, suspensions, syrups, with the elixirs containing inert diluents commonly used in the art, such as water. Besides such inert diluents, compositions can also include adjuvants, such as wetting agents, emulsifying and suspending agents, and sweetening, flavoring and perfuming agents.

Preparations for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, filtration through a bacteria retaining filter, by incorporating sterilizing agents into the compositions, by irradiating the compositions, or by heating the compositions. They can also be manufactured using sterile water, or some other sterile injectable medium, immediately before use.

Compositions for rectal or vaginal administration are preferably suppositories which may contain, in addition to the active substance, excipients such as cocoa butter or a suppository wax. Compositions for nasal or sublingual administration are also prepared with standard excipients well known in the art.

The dosage of active ingredient in the compositions may be varied; however, it is necessary that the amount of the active ingredient shall be such that a suitable dosage form is obtained. The selected dosage depends upon the desired therapeutic effect, on the route of administration, and on the duration of the treatment desired. Generally dosage levels of between 0.001 to 10 mg/kg of body weight daily are administered to mammals.

Moreover, the use of the transferrin fusion protein comprising EMP1 or analogs thereof for the manufacture of a medicinal product which can be used in the treatment of diseases associated with low or defective red blood cell production are contemplated. Examples of such diseases are not limited to those described above.

### T-20 and T-1249

HIV infection is pandemic and HIV associated diseases represent a major world health problem. Although considerable effort is being put into the successful design of effective therapeutics, currently no curative anti-retroviral drugs against AIDS exist. In attempts to develop such drugs, several stages of the HIV life cycle have been considered as targets for therapeutic intervention (Mitsuya, H. et al., 1991, FASEB J. 5:2369-2381). For example, virally encoded reverse transcriptase has been one focus of drug development. A number of reverse-transcriptase-targeted drugs, including 2',3'-dideoxynucleoside analogs such as AZT, ddI, ddc, and d4T have been developed which have been shown to been active against HIV (Mitsuya, H. et al., 1991, Science 249:1533-1544). While beneficial, these nucleoside analogs are not curative, probably due to the rapid appearance of drug resistant HIV mutants (Lander, B. et al., 1989, Science 243:1731-1734). In addition, the drugs often exhibit toxic side effects, such as bone marrow suppression, vomiting, and liver function abnormalities.

Entry inhibitors are distinct from the existing classes of drugs that fight HIV. Other drugs work inside the infected cell. Nucleoside reverse transcriptase inhibitors such as AZT and abacavir and non-nucleoside reverse transcriptase inhibitors like nevirapine and efavirenz all act by shutting down the reverse transcriptase enzyme that HIV uses to replicate itself once it is inside the cell. Protease inhibitors shut down the viral protease enzyme HIV uses to package itself up for export. By contrast, entry inhibitors are drugs that interfere with the processes involved in the virus' initial assault on the cell's outer membrane.

T-20 is the most studied of all the entry inhibitors and is the first member of the fusion inhibitor class. Unlike existing AIDS drugs that work inside the cell and target viral enzymes involved in the replication of the virus, T-20 inhibits fusion of HIV with host cells before the virus enters the cell and begins its replication process. T-20 binds to one of the two helical domains of gp41. Gp41 is a spring-loaded HIV-1 protein that is activated when CD4 binds to HIV gp-120. The fusion action of gp41 is inhibited if its two helical domains cannot fold together. T-20 binds to gp41, effectively keeping the protein from functioning. It has been shown in early, single-arm clinical studies to be about as potent as a protease inhibitor by itself-giving greater than 10 fold reductions in viral load-and to be safe in combination with other antiretrovirals.

U.S. Patent 5,464,933 discloses T-20 (pentafuside, DP-178) as a 36 amino acid synthetic peptide. Since this drug is a peptide, it cannot be given orally because it is readily broken down by the digestive system. When administered by subcutaneous injection, T-20 achieves sufficient levels in the blood to have anti-HIV activity. It is administered by subcutaneous injection twice daily. However, patients develop skin reactions at the injection site. The most frequently reported treatment related adverse events were mild to moderate local injection site reactions. These consist of mild pain, temporary swelling and redness at the site of injection.

U.S. Patent 6,479,055 discloses peptide analogs of the DP-178 (peptides corresponding to amino acid residues 638 to 673 of transmembrane protein gp41 of HTV-1_{LAI}, which exhibit anti-membrane fusion capability, antiviral activity, such as the ability to inhibit HIV transmission to uninfected CD-4⁺ cells, or an ability to modulate intracellular processes involving coiled-coil peptide structures. Further, the patent relates to the use of DP-178 and DP-178 portions and/or analogs as antifusogenic or antiviral compounds or as inhibitors of intracellular events involving coiled-coil peptide structures. Further, the patent teaches the use of the peptides as diagnostic agents. For example, a DP178 peptide may be used as an HIV subtype-specific diagnostic.

T-1249 is a sister compound of T-20. Like T-20, T-1249 targets the HIV glycoprotein known as gp41 which HIV uses to bind onto CD4 cells. T-1249 has shown potent anti-HIV effects in animal and laboratory studies. Preliminary safety, dosing and efficacy studies in humans have provided support for ongoing research.

T-1249 is currently administered by subcutaneous (under the skin) injection once or twice daily. The first safety study of T-1249 conducted in humans found two serious adverse events: hypersensitivity reaction (oral ulcers, maculopapular rash, fever) and severe neutropenia. Forty percent of recipients developed injection site reactions but these were deemed to be mild. Dizziness, diarrhea, headache and fever have also been reported by recipients. No dose-limiting toxicity was identified and experiments with higher doses are likely.

T-1249 has completed phase I/II safety and dosing studies. Initial results indicated that higher doses produced an average viral load drop of 1.3 log.

Dose-dependent decreases in HIV RNA have been reported. In the study of T-1249, the average reduction from baseline ranged from 0.29 to 1.96 log copies/ml (Gulick 2002).

Described herein are transferrin fusion proteins comprising T-20, T-1249, or analogs thereof with increased half-life and Pharmaceutical compositions comprising such fusion proteins. Also described are pharmaceutical compositions comprising these transferrin fusion proteins for therapeutic purposes. The use of such fusion proteins as inhibitors of human and non-human retroviral, especially HIV, transmission to uninfected cells are contemplated. The human retroviruses whose transmission may be inhibited by the peptides include, but are not limited to all strains of HIV-1 and HIV-2 and the human T-lymphocyte viruses (HTLV-I, II, III). The non-human retroviruses whose transmission may be inhibited by the peptides of the invention include, but are not limited to bovine leukosis virus, feline sarcoma and leukemia viruses, simian sarcoma and leukemia viruses, and sheep progress pneumonia viruses.

With respect to HIV, the transferrin fusion protein comprising T-20, T-1249 or analogs thereof may be used as a therapeutic in the treatment of AIDS. These transferrin fusion proteins may be administered using techniques well known to those in the art. Preferably, the pharmaceutical compositions comprising these transferrin fusion proteins are formulated and administered systemically. Techniques for formulation and administration may be found in "Remington's Pharmaceutical Sciences" 18th ed., 1990 Mack Publishing Co., Easton, Pa. Suitable routes may include oral, rectal, transmucosal, or intestinal administration; parenteral delivery, including intramuscular, subcutaneous, intramedullary injections, as well as intrathecal, direct intraventricular, intravenous, intraperitoneal, intranasal, or intraocular injections, just to name a few. Most preferably, administration is intravenous. For injection, the transferrin fusion proteins comprising T-20, T1249, or analogs thereof may be formulated in aqueous solutions, preferably in physiologically compatible buffers such as Hanks' solution, Ringer's solution, or physiological saline buffer. For such transmucosal administration, penetrants appropriate to the barrier to be permeated are used in the formulation. Such penetrants are generally known in the art.

In addition, the transferrin fusion protein comprising T-20, T1249, or analogs thereof may be used as a prophylactic measure in previously uninfected individuals after acute exposure to an HIV virus. Examples of such prophylactic use of the peptides may include, but are not limited to, prevention of virus transmission from mother to infant and other settings where the likelihood of HIV transmission exists, such as, for example, accidents in health care settings wherein workers are exposed to HIV-containing blood products. The transferrin fusion proteins comprising T-20, T-1249, or analogs thereof in such cases may serve the role of a prophylactic vaccine, wherein the host raises antibodies against the fusion proteins, which then serve to neutralize HIV viruses by, for example, inhibiting further HIV infection. Administration of the transferrin fusion proteins as a prophylactic vaccine, therefore, would comprise administering to a host a concentration of transferrin fusion protein effective in raising an immune response which is sufficient to neutralize HIV, by, for example, inhibiting HIV ability to infect cells. The exact concentration will depend upon the specific peptide in the transferrin fusion protein to be administered, but may be determined by using standard techniques for assaying the development of an immune response which are well known to those of ordinary skill in the art. The transferrin fusions protein to be used as vaccines are usually administered intramuscularly.

Effective dosages of the transferrin fusion proteins comprising T-20, T-1249, or analogs thereof to be administered may be determined through procedures well known to those in the art which address such parameters as biological half-life, bioavailability, and toxicity. Given the data presented below in Section 6, DP-178, for example, may prove efficacious in vivo at doses required achieve circulating levels of 10 ng per ml of peptide.

Furthermore, use of the transferrin fusion proteins comprising T-20, T-1249, or analogs thereof for the manufacture of a medicinal product for the treatment of diseases associated with the transmission of a virus is contemplated.

### Soluble Toxin Receptors

Also described herein are fusion proteins comprising soluble toxin receptor and transferrin or modified transferrin. As used herein, the term "toxin" refers to a poisonous substance of biological origin. The fusion proteins comprising a soluble toxin receptor may be used to treat patients suffering from diseases associated with toxins. Such fusion proteins may also be used for diagnostic purposes.

Examples of toxins include, but are not limited to, Pseudomonas exotoxins (PE), Diphtheria toxins (DT), ricin toxin, abrin toxin, anthrax toxins, shiga toxin, botulism toxin, tetanus toxin, cholera toxin, maitotoxin, palytoxin, ciguatoxin, textilotoxin, batrachotoxin, alpha conotoxin, taipoxin, tetrodotoxin, alpha tityustoxin, saxitoxin, anatoxin, microcystin, aconitine, exfoliatin toxins A and B, enterotoxins, toxic shock syndrome toxin (TSST-1), *Y. pestis* toxin, gas gangrene toxin, and others. Because of the seriousness of the diseases that some of these toxins cause and the ease of obtaining some of them for biological warfare, there is a need to develop methods to obtain large quantities of potent anti-toxins at a low cost.

The use of soluble toxin receptors as anti- toxins for treatment and prevention of diseases associated with various toxins is contemplated. Toxin receptors are molecules that bind to a specific toxin. A soluble toxin receptor is one that is capable of being dissolved. Usually peptides or fragments of a receptor are soluble.

Similar to other peptides discussed above, these peptides have a short half-life. Described herein are fusion proteins comprising a soluble peptide of a toxin receptor fused to a transferrin or modified transferrin molecule. The resulting fusion protein has an increased half-life as compared to the soluble toxin receptor peptide. The fusion protein is also easy to produce in large quantities by recombinant means. Since the binding properties of the soluble peptide has not been altered, it will bind the toxin in circulation and prevent the toxin from binding to the target receptor, thus inactivating the toxin. Accordingly, the fusion protein is a potent anti-toxin.

A pharmaceutical composition comprising soluble toxin receptor fused to transferrin or modified transferrin and a pharmaceutically acceptable carrier is described. Also described is the use of transferrin fusion protein comprising soluble toxin receptor for the manufacture of a medicament for the treatment or prevention of diseases or conditions associated with a toxin.

Unlike antibodies which are difficult and expensive to produce in large quantities, the present transferrin/anti-toxin fusion protein is highly potent and less costly to manufacture. Additionally, immunization is not expected to maintain the antibody titers required to protect in instances of mass exposure following an act of bioterrorism. It is unrealistic for the population to be immunized on a mass scale in anticipation of an exposure.

### Bacillus Anthracis Toxin Receptor

Anthrax toxin is a well-known agent of biological warfare derived from *Bacillus anthracis. Bacillus anthracis* produces three proteins which when combined appropriately form two potent toxins, collectively designated anthrax toxin. Protective antigen (PA, 82,684 Da (Dalton)) and edema factor (EF, 89,840 Da) combine to form edema toxin (ET), while PA and lethal factor (LF, 90,237 Da) combine to form lethal toxin (LT) (Leppla, S. H. Alouf, J. E. and Freer, J. H., eds. Academic Press, London 277-302, 1991). ET and LT each conform to the AB toxin model, with PA providing the target cell binding (B) function and EF or LF acting as the effector or catalytic (A) moieties. A unique feature of these toxins is that LF and EF have no toxicity in the absence of PA, apparently because they cannot gain access to the cytosol of eukaryotic cells.

Recently, two of the targets of Lethal factor (LF) were identified in cells. LF is a rnetalloprotease that specifically cleaves Mek1 and Mek2 proteins, kinases that are part of the MAP-kinase signaling pathway. LF's proteolytic activity inactivates the MAP-kinase signaling cascade through cleavage of mitogen activated protein kinase kinases 1 or 2 (MEK1 or MEK2). (Leppla, S. A. In The Comprehensive Sourcebook of Bacterial Protein Toxins. J. E. Alouf and J. H. Freer, Eds. 2nd edition, San Diego, Academic Press, 1999; pp243-263.).

PA is capable of binding to the surface of many types of cells. After PA binds to a specific receptor (Leppla, supra, 1991) on the surface of susceptible cells, it is cleaved at a single site by a cell surface protease, probably furin, to produce an amino-terminal 19-kDa fragment that is released from the receptor/PA complex (Singh et al., J. Biol. Chem. 264:19103-19107, 1989). Removal of this fragment from PA exposes a high-affinity binding site for LF and EF on the receptor-bound 63-kDa carboxyl-terminal fragment (PA63). The complex of PA63 and LF or EF enters cells and probably passes through acidified endosomes to reach the cytosol.

PA, the non-toxic, cell-binding component of the toxin, is the essential component of the currently available human vaccine. The vaccine is usually produced from batch cultures of the Sterne strain of B. anthracis, which although avirulent, is still required to be handled as a Class III pathogen. In addition to PA, the vaccine contains small amounts of the anthrax toxin moieties, edema factor and lethal factor, and a range of culture derived proteins. All these factors contribute to the recorded reactogenicity of the vaccine in some individuals. The vaccine is expensive and requires a six month course of four vaccinations. Futhermore, present evidence suggests that this vaccine may not be effective against inhalation challenge with certain strains (M. G. Broster et al., Proceedings of the International Workshop on Anthrax, Apr. 11-13, 1989, Winchester UK. Salisbury med Bull Suppl No 68, (1990) 91-92).

Bradley et al. (Nature, 2001,414: 225-229) disclose cloning of the human anthrax receptor that binds to PA. The receptor, ATR (anthrax toxin receptor) is a type I membrane protein consisting of 368 amino acids. The protein has a predicted signal peptide of 27 amino acids, an extracellular domain of 293 amino acids containing three putative N-linked glycosylation sites, a putative transmembrane region of 23 amino acids and a short cytoplasmic tail of 25 amino acids. A notable feature of ATR is that the extracellular domain consists of a von willebrand factor type A (VWA) domain which is known to be important in protein-protein interactions. This VWA domain is located at amino acids 44 to 216. A soluble version of ATR comprising amino acids 41-227 was shown to bind the anthrax toxin. Accordingly, the VWA domain of ATR binds directly to PA.

Described herein is an anthrax antitoxin comprising the extracellular domain of ATR fused to transferrin or modified transferrin molecule. Fusion proteins comprising fragments thereof of the extracellular domain of ATR that binds PA fused to transferrin or modified transferrin molecule are also described. Fusion proteins comprising small molecule mimetics of the extracellular domain of ATR that binds PA fused to transferrin or modified transferrin molecule are contemplated.

### Clostridium Botulinum Toxic

The clostridial neurotoxins are the most poisonous substance. Humans are exposed to the neurotoxin produced by *Clostridium tetani* (tetanus toxin) as a result of wounds. Although the tetanus toxin remains a serious public health problem in developing countries around the world, nearly everyone in the western world is protected from tetanus toxin as a consequence of childhood immunizations. Humans usually come into contact with the neurotoxin produced by *Clostridium botulinum* (botulinum toxin) through food poisoning. However, there are rare incidents of wound botulism and colonizing infection of neonates known as infant botulism. Since botulinum poisoning is rare, immunization of the general population is not warranted on the basis of cost and the expected rates of adverse reaction to the vaccine. Therefore, humans are not protected from botulinum toxins. Additionally, these toxins are relatively to produce. Consequently, botulinum toxins are likely biological warfare agents.

As discussed, the anaerobic, gram positive bacterium *Clostridium botulinum* produces the most poisonous biological neurotoxin known with a lethal human dose in the nanogram range. The effect of the toxin ranges from diarrheal diseases that can cause destruction of the colon, to paralytic effects that can cause death. The spores of *Clostridium botulinum* are found in soil and can grow in improperly sterilized and sealed food containers of home based canneries, which are the cause of many of the cases of botulism. The symptoms of botulism typically appear 18 to 36 hours after eating the foodstuffs infected with a *Clostridium botulinum* culture or spores. The botulinum toxin can apparently pass unattenuated through the lining of the gut and attack peripheral motor neurons. Symptoms of botulinum toxin intoxication can progress from difficulty walking, swallowing, and speaking to paralysis of the respiratory muscles and death.

Botulism disease may be grouped into four types, based on the method of introduction of toxin into the bloodstream. Food-borne botulism results from ingesting improperly preserved and inadequately heated food that contains botulinal toxin (i.e., the toxin is pre-formed prior to ingestion). Wound-induced botulism results from C. *botulinum* penetrating traumatized tissue and producing toxin that is absorbed into the bloodstream. Since 1950, thirty cases of wound botulism have been reported (Swartz, "Anaerobic Spore-Forming Bacilli: The Clostridia," pp. 633-646, in Davis et al., (eds.), Microbiology, 4th edition, J. B. Lippincott Co. (1990)). Inhalation botulism results when the toxin is inhaled. Inhalation botulism has been reported as the result of accidental exposure in the laboratory (Holzer, Med. Klin., 41:1735 [1962]) and is a potential danger if the toxin is used as an agent of biological warfare (Franz et al., in Botulinum and Tetanus Neurotoxins, DasGupta (ed.), Plenum Press, New York [1993], pp. 473-476). Infectious infant botulism results from *C. botulinum* colonization of the infant intestine with production of toxin and its absorption into the bloodstream.

Different strains of *Clostridium botulinum* each produce antigenically distinct toxin designated by the letters A-G. Serotype A toxin has been implicated in 26% of the cases of food botulism; types B, E, and F have also been implicated in a smaller percentage of the food botulism cases (Sugiyama, Microbiol. Rev., 44:419 (1980)). Wound botulism has been reportedly caused by only types A or B toxins (Sugiyama, supra). Nearly all cases of infant botulism have been caused by bacteria producing either type A or type B toxin (exceptionally, one New Mexico case was caused by *Clostridium botulinuna* producing type F toxin and another by *Clostridium botulinum* producing a type B-type F hybrid) (Arnon, Epidemiol. Rev., 3:45 (1981)). Type C toxin affects waterfowl, cattle, horses and mink. Type D toxin affects cattle, and type E toxin affects both humans and birds.

*Clostridium botulinum* neurotoxin acts on nerve endings to block acetylcholine release. Binding of the neurotoxin to a membrane receptor through its heavy chain is the first essential step in its mode of toxin action. Li et al. (J Nat Toxins 1998, 7(3):215-26) purified Type E botulinum neurotoxin (BoNT/E) or type A botulinum neurotoxin (BoNT/A) from rat brain synaptosomes employing a neurotoxin affinity column chromatography. The protein fraction eluted from the affinity column with 0.5 M NaCl contained a 57 kDa protein as a major eluant. Immunoblotting the eluant with anti-synaptotagmin antibodies revealed that the 57 kDa protein was synaptotagmin I. Rat synaptotagmin I has been suggested as the receptor for BoNT/B (Nishiki et al., J. Biol. Chem. 269, 10498-10503, 1994) in rat brain. Li *et al.* investigated the binding of BoNT/A and BoNT/E to synaptotagmin I by a microtiter plate-based method. Binding of synaptotagmin I to BoNT/A coated on the plate was competitively reduced upon preincubation of the proteins with BoNT/E, suggesting a competitive binding of BoNT/A and BoNT/E to the receptor. Taken together, these results suggest that the same receptor protein binds to all three BoNT serotypes tested.

Synaptotagmin I is a broad acting receptor of *Clostridium botulinum* neurotoxin serotypes A, B, and E and possibly C, D, F, and G. It is located on the motor neuronal cell. The N-terminal fragment of synaptotagmin I, amino acids 1-53 (SEQ ID NO: 4), is responsible for binding to the various neurotoxin serotypes. The binding mediates translocation of the neurotoxin into the cell and blocks neurotransmitter release which results in paralysis and in extreme cases fatality. The N-terminal fragment may be produced by recombinant means and used to bind neurotoxins in circulation. The binding of the fragment to the neurotoxin prevents the neurotoxin from binding its target receptor which results in neutralization of the toxin.

An anti-toxin of *Clostridium botulinum* with significantly increased half-life as compared to the recombinantly produced 53 amino acid fragment, so that the anti-toxin has sufficient time to find and bind the neurotoxins as they enter the circulation is described herein. Also described is a fusion protein comprising the N-terminal 53 amino acid fragment of synaptotagmin I fused to transferrin or modified transferrin, thereby increasing the half-life of the fragment without altering the binding properties of the fragment. Alternatively, the fragment could be chemically pegylated to prolong circulating life. The longer half-life of the anti-toxin will make a given dose more effective. Unlike antibodies, the present anti-toxin fusion protein is broad acting and bind to several neurotoxin serotypes, specifically neurotoxin A, B, and E.

The fusion protein may be produced in a highly efficient microbial production system which can provide large quantities of the anti-toxin at a reasonable cost to treat the population following mass exposure in acts of bioterrorism. This fusion protein can also be used in a prophylactic mode prior to exposure

Anti-toxins comprising peptide fragments of amino acids 1-53 of synaptotagmin I or small molecule mimetics of amino acids 1-53 of snynaptotagmin I fused to transferrin or modified transferrin molecule are also contemplated.

The fusion protein can also be used to block botulism spread through food or air contamination among the civilian population.

The anti-toxin fusion protein may be used to treat wound botulism resulting from drug use and accidental overdose of *botulinum* neurotoxin following the treatment of various diseases such as migraine dystonia, and hyperhidrosis.

The anti-toxin fusion protein may be used to treat botulism from food poisoning.

### Diptheria Toxin Receptor

Diphtheria is caused by a bacterium, *Corynebacterium diphtheria,* which typically infects mucous membranes: the nose and throat are favorite places for the infection to take hold, but mucous membranes of the eyes or genitalia can be infected also. The bacteria produce a toxin which causes damage to tissue both at the site of the original infection and in other parts of the body once the toxin is spread via the bloodstream. The most serious effects of diphtheria toxin are on the heart (muscle damage leading to loss of pumping ability), kidneys, and the nervous system.

Diphtheria can be treated by giving penicillin or other antibiotics to kill the bacteria, and antitoxin to clear free toxin in the body. However the antitoxin will not clear toxin that has already bound to cells and started to damage them. The better approach is to give toxoid to stimulate immunity to the toxin, thus enabling the body to clear toxin as soon as it appears. Immunity to a bacterial toxin such as diphtheria toxin (DT) may be acquired naturally during the course of an infection, or artificially by injection of a detoxified form of the toxin (*i.e.,* a toxoid) (Germanier, ed., Bacterial Vaccines, Academic Press, Orlando, Fla., 1984). Toxoids have traditionally been prepared by chemical modification of native toxins (*e.g.,* with formalin or formaldehyde (Lingood et al., Brit. J. Exp. Path. 44:177, 1963)), rendering them nontoxic while retaining an antigenicity that protects the vaccinated animal against subsequent challenges by the natural toxin: an example of a chemically-inactivated DT is that described by Michel and Dirkx (Biochem. Biophys. Acta 491:286-295, 1977), in which Trp-153 of Fragment A is the modified residue. The toxoid is given initially at ages 2, 4, and 6 months, again at ages 18 months and 5 years, and regularly every 10 years after that.

Several years it appeared that diptheria was no longer a major public health threat. However, recently, there has been a resurgence of diphtheria in the New Independent States of the former Soviet Union, Ecuador, Thailand, Algeria and other countries. Although diphtheria patients have been treated with equine antitoxin, which neutralizes unbound toxin, surviving patients have often developed serum sickness, an immune complex-type disease. Thus, there is a need to develop a better treatment for diphtheria patients.

The DT molecule is produced as a single polypeptide of 535 amino acids that is readily spliced to form two subunits linked by a disulfide bond, Fragment A (N-terminal of about 21Kda) and Fragment B (C-terminal of about 37Kda), as a result of cleavage at residue 190, 192, or 193 (Moskaug, et al., Biol Chem 264:15709-15713, 1989; Collier et al., Biol Chem, 246:1496-1503, 1971). Fragment A is the catalytically active portion of DT. It is an NAD-dependent ADP-ribosyltransferase which specifically targets a protein synthesis factor termed elongation factor 2 (EF-2), thereby inactivating EF2 and shutting down protein synthesis in the cell. Fragment A consists of the diphtheria toxin C domain. Fragment A is linked to the diphtheria toxin Fragment B by a polypeptide loop. Fragment B of DT possesses a receptor-binding domain (the R domain) which recognizes and binds the toxin molecule to a particular receptor structure found on the surfaces of many types of mammalian cells. Once DT is bound to the cell via this receptor structure, the receptor/DT complex is taken up by the cell via receptor-mediated endocytosis. A second functional region on Fragment B (the T domain) acts to translocate DT across the membrane of the endocytic vesicle, releasing catalytically active Fragment A into the cytosol of the cell. A single molecule of Fragment A is sufficient to inactivate the protein synthesis machinery in a given cell.

Naglich et al. (Cell, 1992, 69: 1051-1061) describe expression cloning of diphtheria toxin receptor from highly toxin-sensitive monkey Vero cells. The amino acid sequence of the receptor was found to be identical to that of the cell surface-expressed heparin-binding epidermal growth factor-like growth factor (HB-EGF) precursor (proHB-EGF). Although proHB-EGF is cleaved and released as soluble mature HB-EGF (Goishi et al., Mol. Biol. Cell, 1995, 6:967-980), a significant amount of proHB-EGF remains on the cell surface and functions as a juxtacrine growth factor (Hagashiyama et al., Science, 251: 929-938) and as a DT receptor (Iwamoto et al., EMBO J., 1994, 13:2322-2330; Naglich et al., Cell, 1992, 69: 1051-1061).

Hooper et al. (Biochem. Biophys. Res. Commun., 1995, 206: 710-717) show that recombinant mature human HB-EGF consisting of residues 63-148 (the extracellular domain or the mature growth factor) strongly inhibits the binding of radiolabeled DT to toxin receptor-bearing cells. This result suggests that it would be possible to treat diphtheria patients with mature HB-EGF, a natural growth factor which will not cause serum sickness. However, mature HB-EGF might produce side effects due to its growth factor activity.

Cha et al. (Infection and Immunity, 2002, 70(5): 2344-2350) developed a treatment based on human DT receptor/HB-EGF precursor. They teach a recombinant truncated HB-EGF, consisting of residues 106-149 and lacking most of the heparin binding domain, capable of inhibiting binding of radioiodinated DT to cells. Moreover, they showed that it was a more effective inhibitor of DT binding than the recombinant mature HB-EGF. Further the investigators mutated some residues in the EGF like domain of the recombinant truncated HB-EGF to destroy some of its mitogenic effect. It was demonstrated that the receptor analog (1117A/L148A) displayed a low mitogenic effect. The truncated (I117A/L148A) HB-EGF protein retained high DT binding affinity. The work of Cha *et al.* suggest that truncated (I117A/L148A) HB-EGF protein could be a safe anti-toxin for EGF receptor.

Described herein are anti-toxin fusion protein comprising truncated (I117A/L148A) HB-EGF protein fused to transferrin or modified transferrin. Also described are transferrin/anti-toxin fusion proteins comprising fragments thereof of truncated (I117A/L148A) HB-EGF protein that bind DT and has minimal mitogenic activity. Additionally, transferrin/anti-toxin fusion proteins comprising analogs of truncated HB-EGF protein that bind DT and has minimal mitogenic activity are described.

### Other Toxin Receptors

Bacterium *Bacillus thuringiensis* (BT) produces bacteriocidal proteins that are toxic to a limited range of insects, mostly in the orders Lepidoptera, Coleoptera and Diptera. Bt toxins have been used to control pests, by applying *Bacillus thuringiensis* to plants or transforming plants themselves so that they generate the toxins by virtue of their transgenic character. The toxins themselves are glycoprotein products of the cry gene as described by Hofte, H. et al. Microbiol Rev (1989) 53:242. US Patent 5,693,491 discloses the cDNA encoding a glycoprotein receptor from the tobacco hornworm that binds a *Bacillus thuringiensis* toxin. The availability of this cDNA permits the retrieval of DNAs encoding homologous receptors in other insects and organisms as well as the design of assays for the cytotoxicity and binding affinity of potential pesticides and the development of methods to manipulate natural and/or introduced homologous receptors and, thus, to destroy target cells, tissues and/or organisms.

Most Vibrio cholerae vaccine candidates constructed by deleting the ctxA gene encoding cholera toxin (CT) are able to elicit high antibody responses, but more than one-half of the vaccines still develop mild diarrhea (Levine et al., Infect. Immun., 56(1):161-167 (1988)). Given the magnitude of the diarrhea induced in the absence of CT, it was hypothesized that V. cholerae produce other enterotoxigenic factors, which are still present in strains deleted of the ctxA sequence (Levine *et al.,* supra). As a result, a second toxin, zonula occludens toxin (hereinafter "ZOT") elaborated by V. cholerae, and which contribute to the residual diarrhea, was discovered (Fasano et al., Proc. Nat. Acad. Sci., USA, 8:5242-5246 (1991)). The zot gene is located immediately adjacent to the ctx genes. The high percent concurrence of the zot gene with the ctx genes among V. cholerae strains (Johnson et al., J. Clin. Microb., 31/3:732-733 (1993); and Karasawa et al, FEBS Microbiology Letters, 106:143-146 (1993)) suggests a possible synergistic role of ZOT in the causation of acute dehydrating diarrhea typical of cholera. The zot gene has also been identified in other enteric pathogens (Tschape, 2nd Asian-Pacific Symposium on Typhoid fever and other Salomellosis, 47(Abstr.) (1994)). U.S. Patent 5,864,014 discloses the purified receptor for zonula occludens toxin.

Diarrhea can be caused by small, heat stable peptide toxins (ST) produced by various pathogenic bacteria (Thompson, M. R., 1987, Pathol. Immunopathol. Res. 6, 103-116). In developing countries, such toxins may be responsible for 50% to 80% of the reported cases of diarrhea (Giannella, R. A., 1981, Ann. Rev. Med. 32, 341-357). ST are also a major cause of diarrhea in laboratory and domestic animals (Burgess et al., 1978, Infect. Immun. 21, 526-531). It has been shown that heat stable enterotoxins bind to a cell surface receptor in the intestine which subsequently leads to an activation of guanylyl cyclase (Field et al., 1978, Proc. Natl. Acad. Sci. USA 75, 2800-2804; Guerrant et al.,1980, J. Infectios Diseases 142, 220-228). The rise in cyclic GMP then stimulates fluid secretion thereby causing diarrhea. It has been reported that the ST receptor is a distinctly different protein than quanylyl cyclase based on partial chromatographic separation of a detergent-solubilized ST-binding protein from guanylyl cyclase activity (Kuno et al., 1986, J. Biol. Chem. 261, 1470-1476; Waldman, et al., 1986, Infect. Immun. 51, 320-326). U.S. 5,237,051 discloses cloning of the nucleic acid encoding the intestinal receptor which recognizes heat stable enterotoxins and has guanylyl cylase activity. Data shows that the receptor binds enterotoxin and signals normally through the cyclic GMP second messenger system.

Sepsis is most commonly caused by infection or trauma induced by a toxin. The initial symptoms of sepsis typically include chills, profuse sweat, irregularly remittent fever, prostration and the like, followed by persistent fever, hypotension leading to shock, neutropenia, leukopenia, disseminated intravascular coagulation, adult respiratory distress syndrome and multiple organ failure. Sepsis-inducing toxins have been found associated with pathogenic bacteria, viruses, plants and venoms. Among the well described bacterial toxins are the endotoxins or lipopolysaccharides(LPS) of the gram-negative bacteria. These molecules are glycolipids that are ubiquitous in the outer membrane of all gram-negative bacteria. It has been reported that report that membrane-fixed CD14 acts as a receptor for the protein-bound endotoxin (LPS) complex and mediates the cellular effects of endotoxin (Wright et al., 1990, Science, 249: 1431). Soluble CD14 truncated at amino acid 71(N71) contains the lipolysaccharide binding sequence. N71 has been shown to neutralize circulating LPS, *i.e.,* acting as an endotoxin antagonist (Higuchi et al., Pathobiology, 2002, 70: 103).

### Methods of Delivering Antitoxin Fusion Protein

The anti-toxin fusion proteins may be packaged in a single piston syringe with two contiguous chambers. The first chamber will contain diluent and the second will contain the lyophilized anti-toxin fusion protein. As the plunger is pushed down the diluent will be driven into the next chamber to dissolve the anti-toxin fusion protein which will be expelled through a needle for direct intramuscular delivery. The diluent can contain an anti-freeze such glycerol to act in freezing conditions. The lyophilized product will remain stable in tropical conditions.

Delivering the antitoxin fusion proteins described herein in this manner to soldiers entering into a combat situation where the risk of exposure to toxins is high is contemplated. The anti-toxin fusion protein can be used for immediate treatment on the battlefied and as a prophylactic before going on the battlefield.

### Nucleic Acids

The present invention also provides nucleic acid molecules encoding transferrin fusion proteins comprising a modified transferrin protein joined to a GLP-1 protein. The fusion protein may further comprise a linker region, for instance a linker less than about 50, 40, 30, 20, or 10 amino acid residues. The linker can be covalently linked to and between the transferrin protein or portion thereof and the therapeutic protein, preferably the therapeutic protein. Nucleic acid molecules of the invention may be purified or not.

Host cells and vectors for replicating the nucleic acid molecules and for expressing the encoded fusion proteins are also provided. Any vectors or host cells may be used, whether prokaryotic or eukaryotic, but eukaryotic expression systems, in particular yeast expression systems, may be preferred. Many vectors and host cells are known in the art for such purposes. It is well within the skill of the art to select an appropriate set for the desired application.

DNA sequences encoding transferrin, portions of transferrin and GLP-1 proteins of interest may be cloned from a variety of genomic or cDNA libraries known in the art. The techniques for isolating such DNA sequences using probe-based methods are conventional techniques and are well known to those skilled in the art. Probes for isolating such DNA sequences may be based on published DNA or protein sequences (see, for example, Baldwin, G.S. (1993) Comparison of Transferrin Sequences from Different Species. Comp. Biochem. Physiol. 106B/1:203-218 and all references cited therein.
Alternatively, the polymerase chain reaction (PCR) method disclosed by Mullis et al. (U.S. Pat. No. 4,683,195) and Mullis (U.S. Pat. No. 4,683,202) may be used. The choice of library and selection of probes for the isolation of such DNA sequences is within the level of ordinary skill in the art.

As known in the art "similarity" between two polynucleotides or polypeptides is determined by comparing the nucleotide or amino acid sequence and its conserved nucleotide or amino acid substitutes of one polynucleotide or polypeptide to the sequence of a second polynucleotide or polypeptide. Also known in the art is "identity" which means the degree of sequence relatedness between two polypeptide or two polynucleotide sequences as determined by the identity of the match between two strings of such sequences. Both identity and similarity can be readily calculated (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991).

While there exist a number of methods to measure identity and similarity between two polynucleotide or polypeptide sequences, the terms "identity" and "similarity" are well known to skilled artisans (Sequence Analysis in Molecular Biology, von Heinje, G., Academic Press, 1987; Sequence Analysis Primer, Gribskov, M. and Devereux, J., eds., M Stockton Press, New York, 1991; and Carillo, H., and Lipman, D., SIAM J. Applied Math., 48: 1073 (1988). Methods commonly employed to determine identity or similarity between two sequences include, but are not limited to those disclosed in Guide to Huge Computers, Martin J. Bishop, ed., Academic Press, San Diego, 1994, and Carillo, H., and Lipman, D., SIAM J. Applied Math. 48:1073 (1988).

Preferred methods to determine identity are designed to give the largest match between the two sequences tested. Methods to determine identity and similarity are codified in computer programs. Preferred computer program methods to determine identity and similarity between two sequences include, but are not limited to, GCG program package (Devereux, et al., Nucl. Acid Res. 12(1):387 (1984)), BLASTP, BLASTN, FASTA (Atschul, et al., J. Mol. Biol. 215:403 (1990)). The degree of similarity or identity referred to above is determined as the degree of identity between the two sequences, often indicating a derivation of the first sequence from the second. The degree of identity between two nucleic acid sequences may be determined by means of computer programs known in the art such as GAP provided in the GCG program package (Needleman and Wunsch J. Mol. Biol. 48:443-453 (1970)). For purposes of determining the degree of identity between two nucleic acid sequences for the present invention, GAP is used with the following settings: GAP creation penalty of 5.0 and GAP extension penalty of 0.3.

### Codon Optimization

The degeneracy of the genetic code permits variations of the nucleotide sequence of a transferrin protein and/or therapeutic protein of interest, while still producing a polypeptide having the identical amino acid sequence as the polypeptide encoded by the native DNA sequence. The procedure, known as "codon optimization" (described in U.S. Patent 5,547,871) provides one with a means of designing such an altered DNA sequence. The design of codon optimized genes should take into account a variety of factors, including the frequency of codon usage in an organism, nearest neighbor frequencies, RNA stability, the potential for secondary structure formation, the route of synthesis and the intended future DNA manipulations of that gene. In particular, available methods may be used to alter the codons encoding a given fusion protein with those most readily recognized by yeast when yeast expression systems are used.

The degeneracy of the genetic code permits the same amino acid sequence to be encoded and translated in many different ways. For example, leucine, serine and arginine are each encoded by six different codons, while valine, proline, threonine, alanine and glycine are each encoded by four different codons. However, the frequency of use of such synonymous codons varies from genome to genome among eukaryotes and prokaryotes. For example, synonymous codon-choice patterns among mammals are very similar, while evolutionarily distant organisms such as yeast (such as *S. cerevisiae*), bacteria (such as *E. coli*) and insects (such as *D. melanogaster*) reveal a clearly different pattern of genomic codon use frequencies (Grantham, R., et al., Nucl. Acid Res., 8,49-62 (1980); Grantham, R., et al., Nucl. Acid Res., 9, 43-74 (1981); Maroyama, T., et al., Nucl. Acid Res., 14, 151-197 (1986); Aota, S., et al., Nucl. Acid Res., 16,315-402 (1988); Wada, K., et al., Nucl. Acid Res., 19 Supp., 1981-1985 (1991); Kurland, C. G., FEBS Lett., 285, 165-169 (1991)). These differences in codon-choice patterns appear to contribute to the overall expression levels of individual genes by modulating peptide elongation rates. (Kurland, C. G., FEBS Lett., 285, 165-169 (1991); Pedersen, S., EMBO J., 3, 2895-2898 (1984); Sorensen, M. A., J. Mol. Biol., 207, 365-377 (1989); Randall, L. L., et al., Eur. J. Biochem., 107, 375-379 (1980); Curran, J. F., and Yarus, M., J. Mol. Biol., 209, 65-77 (1989); Varenne, S., et al., J. Mol. Biol., 180, 549-576 (1984), Varenne, S., et al., J. Mol, Biol., 180, 549-576 (1984); Garel, J.-P., J. Theor. Biol., 43, 211-225 (1974); Ikemura, T., J. Mol. Biol., 146, 1-21 (1981); Ikemura, T., J. Mol. Biol., 151, 389-409 (1981)).

The preferred codon usage frequencies for a synthetic gene should reflect the codon usages of nuclear genes derived from the exact (or as closely related as possible) genome of the cell/organism that is intended to be used for recombinant protein expression, particularly that of yeast species. As discussed above, in one preferred embodiment the human Tf sequence is codon optimized, before or after modification as herein described for yeast expression as may be the therapeutic protein nucleotide sequence(s).

### Vectors

Expression units for use in the present invention will generally comprise the following elements, operably linked in a 5' to 3' orientation: a transcriptional promoter, a secretory signal sequence, a DNA sequence encoding a modified Tf fusion protein comprising transferrin protein or a portion of a transferrin protein joined to a DNA sequence encoding a GLP-1 protein or peptide of interest and a transcriptional terminator. As discussed above, any arrangement of the therapeutic protein or peptide fused to or within the Tf portion may be used in the vectors of the invention. The selection of suitable promoters, signal sequences and terminators will be determined by the selected host cell and will be evident to one skilled in the art and are discussed more specifically below.

Suitable yeast vectors for use in the present invention are described in U.S. Patent 6,291,212 and include YRp7 (Struhl et al., Proc. Natl. Acad. Sci. USA 76: 1035-1039, 1978), YEp13 (Broach et al., Gene 8: 121-133, 1979), pJDB249 and pJDB219 (Beggs, Nature 275:104-108, 1978), pPPC0005, pSeCHSA, pScNHSA, pC4 and derivatives thereof. Useful yeast plasmid vectors also include pRS403-406, pRS413-416 and the *Pichia* vectors available from Stratagene Cloning Systems, La Jolla, CA 92037, USA. Plasmids pRS403, pRS404, pRS405 and pRS406 are Yeast Integrating plasmids (YIps) and incorporate the yeast selectable markers *HIS3, TRP1, LEU2* and *URA3.* PlasmidspRS413~41.6 are Yeast Centromere plasmids (YCps).

Such vectors will generally include a selectable marker, which may be one of any number of genes that exhibit a dominant phenotype for which a phenotypic assay exists to enable transformants to be selected. Preferred selectable markers are those that complement host cell auxotrophy, provide antibiotic resistance or enable a cell to utilize specific carbon sources, and include *LEU2* (Broach *et al.* ibid.), URA3 (Botstein et al., Gene 8: 17, 1979), *HIS3* (Struhl *et al.,* ibid.) or POT1 (Kawasaki and Bell, EP 171,142). Other suitable selectable markers include the CAT gene, which confers chloramphenicol resistance on yeast cells. Preferred promoters for use in yeast include promoters from yeast glycolytic genes (Hitzeman et al., J Biol. Chem. 225: 12073-12080, 1980; Alber and Kawasaki, J. Mol. Appl. Genet. 1: 419-434, 1982; Kawasaki, U.S. Pat. No. 4,599,311) or alcohol dehydrogenase genes (Young et al., in Genetic Engineering of Microorganisms for Chemicals, Hollaender et al., (eds.), p. 355, Plenum, N.Y., 1982; Ammerer, Meth. Enzymol. 101: 192-201, 1983). In this regard, particularly preferred promoters are the TPI1 promoter (Kawasaki, U.S. Pat. No. 4,599,311) and the ADH2-4^{C} (see U.S. Patent 6,291,212 promoter (Russell et al., Nature 304: 652-654, 1983). The expression units may also include a transcriptional terminator. A preferred transcriptional terminator is the *TPI1* terminator (Alber and Kawasaki, ibid.). Other preferred vectors and preferred components such as promoters and terminators of a yeast expression system are disclosed in European Patents EP 0258067, EP 0286424, EP0317254, EP 0387319, EP 0386222, EP 0424117, EP 0431880, and EP 1002095; European Patent Publications EP 0828759, EP 0764209, EP 0749478, and EP 0889949; PCT Publication WO 00/44772 and WO 94/04687; and U.S. Patents 5,739,007; 5,637,504; 5,302,697; 5,260,202; 5,667,986; 5,728,553; 5,783,423; 5,965,386; 6150,133; 6,379,924; and 5,714,377,

In addition to yeast, modified fusion proteins of the present invention can be expressed in filamentous fungi, for example, strains of the fungi *Aspergillus.* Examples of useful promoters include those derived from *Aspergillus nidulans* glycolytic genes, such as the *adh3* promoter (McKnight et al., EMBO J. 4: 2093-2099, 1985) and the tpiA promoter. An example of a suitable terminator is the *adh3* terminator (McKnight *et al.,* ibid.). The expression units utilizing such components may be cloned into vectors that are capable of insertion into the chromosomal DNA of *Aspergillus,* for example.

Mammalian expression vectors for use in carrying out the present invention will include a promoter capable of directing the transcription of the modified Tf fusion protein. Preferred promoters include viral promoters and cellular promoters. Preferred viral promoters include the major late promoter from adenovirus 2 (Kaufman and Sharp, Mol. Cell. Biol. 2: 1304-13199, 1982) and the SV40 promoter (Subramani et al., Mol. Cell. Biol. 1: 854-864, 1981). Preferred cellular promoters include the mouse metallothionein 1 promoter (Palmiter et al., Science 222: 809-814, 1983) and a mouse Vκ (see U.S. Patent 6,291,212) promoter (Grant et al., Nuc. Acids Res. 15: 5496, 1987). A particularly preferred promoter is a mouse V_{H} (see U.S. Patent 6,291,212) promoter (Loh *et al.,* ibid.). Such expression vectors may also contain a set of RNA splice sites located downstream from the promoter and upstream from the DNA sequence encoding the transferrin fusion protein. Preferred RNA splice sites may be obtained from adenovirus and/or immunoglobulin genes.

Also contained in the expression vectors is a polyadenylation signal located downstream of the coding sequence of interest. Polyadenylation signals include the early or late polyadenylation signals from SV40 (Kaufman and Sharp, ibid.), the polyadenylation signal from the adenovirus 5 E1B region and the human growth hormone gene terminator (DeNoto et al., Nucl. Acid Res. 9: 3719-3730,1981). A particularly preferred polyadenylation signal is the V_{H} (see U.S. Patent 6,291,212) gene terminator (Loh *et al.,* ibid.). The expression vectors may include a noncoding viral leader sequence, such as the adenovirus 2 tripartite leader, located between the promoter and the RNA splice sites. Preferred vectors may also include enhancer sequences, such as the SV40 enhancer and the mouse µ (see U.S. Patent 6,291,212) enhancer (Gillies, Cell 33: 717-728, 1983). Expression vectors may also include sequences encoding the adenovirus VA RNAs.

### Transformation

Techniques for transforming fungi are well known in the literature, and have been described, for, instance, by Beggs (ibid.), Hinnen et al. (Proc. Natl. Acad. Sci. USA 75: 1929-1933, 1978), Yelton et al., (Proc. Natl. Acad. Sci. USA 81: 1740-1747, 1984), and Russell (Nature 301: 167-169, 1983). Other techniques for introducing cloned DNA sequences into fungal cells, such as electroporation (Becker and Guarente, Methods in Enzymol. 194: 182-187, 1991) may be used. The genotype of the host cell will generally contain a genetic defect that is complemented by the selectable marker present on the expression vector. Choice of a particular host and selectable marker is well within the level of ordinary skill in the art.

Cloned DNA sequences comprising modified Tf fusion proteins of the invention may be introduced into cultured mammalian cells by, for example, calcium phosphate-mediated transfection (Wigler et al., Cell 14: 725, 1978; Corsaro and Pearson, Somatic Cell Genetics 7: 603, 1981; Graham and Van der Eb, Virology 52: 456, 1973.) Other techniques for introducing cloned DNA sequences into mammalian cells, such as electroporation (Neumann et al., EMBO J. 1: 841-845, 1982), or lipofection may also be used. In order to identify cells that have integrated the cloned DNA, a selectable marker is generally introduced into the cells along with the gene or cDNA of interest. Preferred selectable markers for use in cultured mammalian cells include genes that confer resistance to drugs, such as neomycin, hygromycin, and methotrexate. The selectable marker may be an amplifiable selectable marker. A preferred amplifiable selectable marker is the DHFR gene. A particularly preferred amplifiable marker is the DHFR^{r} (see U.S. Patent 6,291,212) cDNA (Simonsen and Levinson, Proc. Natl. Acad. Sci. USA 80: 2495-2499, 1983). Selectable markers are reviewed by Thilly (Mammalian Cell Technology, Butterworth Publishers, Stoneham, Mass.) and the choice of selectable markers is well within the level of ordinary skill in the art.

### Host Cells

The present invention also includes a cell, preferably a yeast cell transformed to express a modified transferrin fusion protein of the invention. In addition to the transformed host cells themselves, the present invention also includes a culture of those cells, preferably a monoclonal (clonally homogeneous) culture, or a culture derived from a monoclonal culture, in a nutrient medium. If the polypeptide is secreted, the medium will contain the polypeptide, with the cells, or without the cells if they have been filtered or centrifuged away.

Host cells for use in practicing the present invention include eukaryotic cells, and in some cases prokaryotic cells, capable of being transformed or transfected with exogenous DNA and grown in culture, such as cultured mammalian, insect, fungal, plant and bacterial cells.

Fungal cells, including species of yeast (*e.g., Saccharomyces* spp., *Schizosaccharomyces* spp., *Pichia* spp.) may be used as host cells within the present invention. Examples of fungi including yeasts contemplated to be useful in the practice, of the present invention as hosts for expressing the, transferrin fusion protein of the inventions are *Pichia* (some species of which were formerly classified as *Hansenula*), *Saccharomyces, Kluyveromyces*, *Aspergillus, Candida, Torulopsis, Torulaspora, Schizosaccharomyces, Citeromyces*, *Pachysolen, Zygosaccharomyces, Debaromyces, Trichoderma, Cephalosporium, Humicola, Mucor*, *Neurospora, Yarrowia, Metschunikowia, Rhodosporidium, Leucosporidium, Botryoascus, Sporidiobolus, Endomycopsis,* and the like. Examples of *Saccharomyces* spp. are *S. cerevisiae, S. italicus and S. rouxii*. Examples of *Kluyveromyces* spp. are *K. fragilis, K. lactis and K. marxianus.* A suitable *Torulaspora* species is *T. delbrueckii.* Examples of *Pichia* spp. are *P. angusta* (formerly *H. polymorpha), P. anomala* (formerly *H. anomala*) and *P*. *pastoris.*

Particularly useful host cells to produce the Tf fusion proteins of the invention are the methylotrophic *Pichia pastoris* (Steinlein et al. (1995) Protein Express. Purif. 6:619-624). *Pichia pastoris* has been developed to be an outstanding host for the production of foreign proteins since its alcohol oxidase promoter was isolated and cloned; its transformation was first reported in 1985. *P. pastoris* can utilize methanol as a carbon source in the absence of glucose. The *P. pastoris* expression system can use the methanol-induced alcohol oxidase (AOX1) promoter, which controls the gene that codes for the expression of alcohol oxidase, the enzyme which catalyzes the first step in the metabolism of methanol. This promoter has been characterized and incorporated into a series of *P. pastoris* expression vectors. Since the proteins produced in *P. pastoris* are typically folded correctly and secreted into the medium, the fermentation of genetically engineered *P. pastoris* provides an excellent alternative to E. coli expression systems. A number of proteins have been produced using this system, including tetanus toxin fragment, Bordatella pertussis pertactin, human serum albumin and lysozyme.

Strains of the yeast *Saccharomyces cerevisiae* are another preferred host. In a preferred embodiment, a yeast cell, or more specifically, a *Saccharomyces cerevisiae* host cell that contains a genetic deficiency in a gene required for asparagine-linked glycosylation of glycoproteins is used. *S. cerevisiae* host cells having such defects may be prepared using standard techniques of mutation and selection, although many available yeast strains have been modified to prevent or reduce glycosylation or hypermannosylation. Ballou et al. (J. Biol. Chem. 255: 5986-5991, 1980) have described the isolation of mannoprotein biosynthesis mutants that are defective in genes which affect asparagine-linked glycosylation. Gentzsch and Tanner (Glycobiology 7:481-486, 1997) have described a family of at least six genes (PMT1-6) encoding enzymes responsible for the first step in O-glycosylation of proteins in yeast. Mutants defective in one or more of these genes show reduced O-linked glycosylation and/or altered specificity of O-glycosylation.

To optimize production of the heterologous proteins, it is also preferred that the host strain carries a mutation, such as the *S*. *cerevisiae* pep4 mutation (Jones, Genetics 85: 23-33, 977), which results in reduced proteolytic activity. Host strains containing mutations in other protease encoding regions are particularly useful to produce large quantities of the Tf fusion proteins of the invention.

Host cells containing DNA constructs of the present invention are grown in an appropriate growth medium. As used herein, the term "appropriate growth medium" means a medium containing nutrients required for the growth of cells. Nutrients required for cell growth may include a carbon source, a nitrogen source, essential amino acids, vitamins, minerals and growth factors. The growth medium will generally select for cells containing the DNA construct by, for example, drug selection or deficiency in an essential nutrient which is complemented by the selectable marker on the DNA construct or co-transfected with the DNA construct. Yeast cells, for example, are preferably grown in a chemically defined medium, comprising a carbon source, e.g. sucrose, a non-amino acid nitrogen source, inorganic salts, vitamins and essential amino acid supplements. The pH of the medium is preferably maintained at a pH greater than 2 and less than 8, preferably at pH 5.5-6.5. Methods for maintaining a stable pH include buffering and constant pH control. Preferred buffering agents include succinic acid and Bis-Tris (Sigma Chemical Co., St. Louis, Mo.). Yeast cells having a defect in a gene required for asparagine-linked glycosylation are preferably grown in a medium containing an osmotic stabilizer. A preferred osmotic stabilizer is sorbitol supplemented into the medium at a concentration between 0.1 M and 1.5 M., preferably at 0.5 M or 1.0 M.

Cultured mammalian cells are generally grown in commercially available serum-containing or serum-free media. Selection of a medium appropriate for the particular cell line used is within the level of ordinary skill in the art. Transfected mammalian cells are allowed to grow for a period of time, typically 1-2 days, to begin expressing the DNA sequence(s) of interest. Drug selection is then applied to select for growth of cells that are expressing the selectable marker in a stable fashion. For cells that have been transfected with an amplifiable selectable marker the drug concentration may be increased in a stepwise manner to select for increased copy number of the cloned sequences, thereby increasing expression levels.

Baculovirus/insect cell expression systems may also be used to produce the modified Tf fusion proteins of the invention. The BacPAK™ Baculovirus Expression System (BD Biosciences (Clontech)) expresses recombinant proteins at high levels in insect host cells. The target gene is inserted into a transfer vector, which is cotransfected into insect host cells with the linearized BacPAK6 viral DNA. The BacPAK6 DNA is missing an essential portion of the baculovirus genome. When the DNA recombines with the vector, the essential element is restored and the target gene is transferred to the baculovirus genome. Following recombination, a few viral plaques are picked and purified, and the recombinant phenotype is verified. The newly isolated recombinant virus can then be amplified and used to infect insect cell cultures to produce large amounts of the desired protein.

Tf fusion proteins of the present invention may also be produced using transgenic plants and animals. For example, sheep and goats can make the therapeutic protein in their milk. Or tobacco plants can include the protein in their leaves. Both transgenic plant and animal production of proteins comprises adding a new gene coding the fusion protein into the genome of the organism. Not only can the transgenic organism produce a new protein, but it can also pass this ability onto its offspring.

### Secretory Signal Sequences

The terms "secretory signal sequence" or "signal sequence" or "secretion leader sequence" are used interchangeably and are described, for example in U.S. Pat. 6,291,212 and U.S. Pat 5,547,871.
Secretory signal sequences or signal sequences or secretion leader sequences encode secretory peptides. A secretory peptide is an amino acid sequence that acts to direct the secretion of a mature polypeptide or protein from a cell. Secretory peptides are generally characterized by a core of hydrophobic amino acids and are typically (but not exclusively) found at the amino termini of newly synthesized proteins. Very often the secretory peptide is cleaved from the mature protein during secretion. Secretory peptides may contain processing sites that allow cleavage of the signal peptide from the mature protein as it passes through the secretory pathway. Processing sites may be encoded within the signal peptide or may be added to the signal peptide by, for example, *in vitro* mutagenesis.

Secretory peptides may be used to direct the secretion of modified Tf fusion proteins of the invention. One such secretory peptide that may be used in combination with other secretory peptides is the alpha mating factor leader sequence. Secretory signal sequences or signal sequences or secretion leader sequences are required for a complex series of post-translational processing steps which result in secretion of a protein. If an intact signal sequence is present, the protein being expressed enters the lumen of the rough endoplasmic reticulum and is then transported through the Golgi apparatus to secretory vesicles and is finally transported out of the cell. Generally, the signal sequence immediately follows the initiation codon and encodes a signal peptide at the amino-terminal end of the protein to be secreted. In most cases, the signal sequence is cleaved off by a specific protease, called a signal peptidase. Preferred signal sequences improve the processing and export efficiency of recombinant protein expression using viral, mammalian or yeast expression vectors. In some cases, the native Tf signal sequence may be used to express and secrete fusion proteins of the invention.

### Linkers

The Tf moiety and the therapeutic protein of the modified transferrin fusion proteins of the invention can be fused directly or using a linker peptide of various lengths to provide greater physical separation and allow more spatial mobility between the fused proteins and thus maximize the accessibility of the therapeutic protein, for instance, for binding to its cognate receptor. The linker peptide may consist of amino acids that are flexible or more rigid. For example, a linker such as but not limited to a poly-glycine stretch. The linker can be less than about 50, 40, 30, 20, or 10 amino acid residues. The linker can be covalently linked to and between the transferrin protein or portion thereof and the therapeutic protein.

### Detection of Tf Fusion Proteins

Assays for detection of biologically active modified transferrin-fusion protein may include Western transfer, protein blot or colony filter as well as activity based assays that detect the fusion protein comprising transferrin and therapeutic protein. A Western transfer filter may be prepared using the method described by Towbin et al. (Proc. Natl. Acad. Sci. USA 76: 4350-4354, 1979). Briefly, samples are electrophoresed in a sodium dodecylsulfate polyacrylamide gel. The proteins in the gel are electrophoretically transferred to nitrocellulose paper. Protein blot filters may be prepared by filtering supernatant samples or concentrates through nitrocellulose filters using, for example, a Minifold (Schleicher & Schuell, Keene, N.H.). Colony filters may be prepared by growing colonies on a nitrocellulose filter that has been laid across an appropriate growth medium. In this method, a solid medium is preferred. The cells are allowed to grow on the filters for at least 12 hours. The cells are removed from the filters by washing with an appropriate buffer that does not remove the proteins bound to the filters. A preferred buffer comprises 25 mM Tris-base, 19 mM glycine, pH 8.3, 20% methanol.

Transferrin fusion proteins of the present invention may be labeled with a radioisotope or other imaging agent and used for *in vivo* diagnostic purposes. Preferred radioisotope imaging agents include iodine-125 and technetium-99, with technetium-99 being particularly preferred. Methods for producing protein-isotope conjugates are well known in the art, and are described by, for example, Eckelman et al. (U.S. Pat. No. 4,652,440), Parker et al. (WO 87/05030) and Wilber et al. (EP 203,764). Alternatively, the transferrin fusion proteins may be bound to spin label enhancers and used for magnetic resonance (MR) imaging. Suitable spin label enhancers include stable, sterically hindered, free radical compounds such as nitroxides. Methods for labeling ligands for MR imaging are disclosed by, for example, Coffman et al. (U.S. Pat. No. 4,656,026).

Detection of a transferrin fusion protein of the present invention can be facilitated by coupling (*i.e.,* physically linking) the therapeutic protein to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

Where one is assaying for the ability of a transferrin fusion protein of the invention to bind or compete with an antigen for binding to an antibody, various immunoassays known in the art can be used, including but not limited to, competitive and non-competitive assay systems using techniques such as radioimmunoassays, ELISA (enzyme linked immunosorbent assay), sandwich immunoassays, immunoradiometric assays, gel diffusion precipitation reactions, immunodiffusion assays, *in situ* immunoassays (using colloidal gold, enzyme or radioisotope labels, for example), western blots, precipitation reactions, agglutination assays (*e.g.,* gel agglutination assays), complement fixation assays, immunofluorescence assays, protein A assays, and immunoelectrophoresis assays, *etc.* The binding of the transferrin fusion protein may be detected by detecting a label on the transferrin fusion protein. The transferrin fusion protein may be detected by detecting binding of a secondary antibody or reagent that interacts with the transferrin fusion protein. Alternatively, the secondary antibody or reagent is labeled. Many means are known in the art for detecting binding in an immunoassay.

Fusion proteins of the invention may also be detected by assaying for the activity of the therapeutic protein moiety. Specifically, transferrin fusion proteins of the invention may be assayed for functional activity (*e.g.,* biological activity or therapeutic activity) using assays known to one of ordinary skill in the art. Additionally, one of skill in the art may routinely assay fragments of a therapeutic protein corresponding to a therapeutic protein portion of a fusion protein of the invention, for activity using well-known assays. Further, one of skill in the art may routinely assay fragments of a modified transferrin protein for activity using assays known in the art.

For example, where one is assaying for the ability of a transferrin fusion protein of the invention to bind or compete with a therapeutic protein for binding to an anti-therapeutic polypeptide antibody and/or anti-transferrin antibody, various immunoassays known in the art can be used, including but not limited to, competitive and non-competitive assay systems using techniques such as radioimmunoassays, ELISA (enzyme linked immunosorbent assay), sandwich immunoassays, immunoradiometric assays, gel diffusion precipitation reactions, immunodiffusion assays, *in situ* immunoassays (using colloidal gold, enzyme or radioisotope labels, for example), western blots, precipitation reactions, agglutination assays (*e.g.,* gel agglutination assays), complement fixation assays, immunofluorescence assays, protein A assays, and immunoelectrophoresis assays, etc. In one example , antibody binding is detected by detecting a label on the primary antibody. In another example , the primary antibody is detected by detecting binding of a secondary antibody or reagent to the primary antibody. In a further example, the secondary antibody is labeled. Many means are known in the art for detecting binding in an immunoassay.

Where a binding partner (*e.g.,* a receptor or a ligand) of a therapeutic protein is identified, binding to that binding partner by a transferrin fusion protein containing that therapeutic protein as the therapeutic protein portion of the fusion can be assayed, *e.g.,* by means well-known in the art, such as, for example, reducing and non-reducing gel chromatography, protein affinity chromatography, and affinity blotting.

### Production of Fusion Proteins

The present invention further provides methods for producing a modified fusion protein of the invention using nucleic acid molecules herein described. In general terms, the production of a recombinant form of a protein typically involves the following steps.

A nucleic acid molecule is first obtained that encodes a transferrin fusion protein of the invention. The nucleic acid molecule is then preferably placed in operable linkage with suitable control sequences, as described above, to form an expression unit containing the protein open reading frame. The expression unit is used to transform a suitable host and the transformed host is cultured under conditions that allow the production of the recombinant protein. Optionally the recombinant protein is isolated from the medium or from the cells; recovery and purification of the protein may not be necessary in some instances where some impurities may be tolerated.

Each of the foregoing steps can be accomplished in a variety of ways. For example, the construction of expression vectors that are operable in a variety of hosts is accomplished using appropriate replicons and control sequences, as set forth above. The control sequences, expression vectors, and transformation methods are dependent on the type of host cell used to express the gene and were discussed in detail earlier and are otherwise known to persons skilled in the art. Suitable restriction sites can, if not normally available, be added to the ends of the coding sequence so as to provide an excisable gene to insert into these vectors. A skilled artisan can readily adapt any host/expression system known in the art for use with the nucleic acid molecules of the invention to produce a desired recombinant protein.

As discussed above, any expression system may be used, including yeast, bacterial, animal, plant, eukaryotic and prokaryotic systems. In some embodiments, yeast, mammalian cell culture and transgenic animal or plant production systems are preferred. In other embodiments, yeast systems that have been modified to reduce native yeast glycosylation, hyper-glycosylation or proteolytic activity may be used.

### Isolation/Purification of Modified Transferrin Fusion Proteins

Secreted, biologically active, modified transferrin fusion proteins may be isolated from the medium of host cells grown under conditions that allow the secretion of the biologically active fusion proteins. The cell material is removed from the culture medium, and the biologically active fusion proteins are isolated using isolation techniques known in the art. Suitable isolation techniques include precipitation and fractionation by a variety of chromatographic methods, including gel filtration, ion exchange chromatography and affinity chromatography.

A particularly preferred purification method is affinity chromatography on an iron binding or metal chelating column or an immunoaffinity chromatography using an antigen directed against the transferrin or therapeutic protein of the polypeptide fusion. The antigen is preferably immobilized or attached to a solid support or substrate. A particularly preferred substrate is CNBr-activated Sepharose (Pharmacia LKB Technologies, Inc., Piscataway, N.J.). By this method, the medium is combined with the antigen/substrate under conditions that will allow binding to occur. The complex may be washed to remove unbound material, and the transferrin fusion protein is released or eluted through the use of conditions unfavorable to complex formation. Particularly useful methods of elution include changes in pH, wherein the immobilized antigen has a high affinity for the transferrin fusion protein at a first pH and a reduced affinity at a second (higher or lower) pH; changes in concentration of certain chaotropic agents; or through the use of detergents.

### Delivery of a Drug or Therapeutic Protein to the inside of a Cell and/or across the Blood Brain Barrier (BBB)

The modified transferrin fusion proteins may be used as a carrier to deliver a molecule or small molecule therapeutic complexed to the ferric ion of transferrin to the inside of a cell or across the blood brain barrier or other barriers including across the cell membrane of any cell type that naturally or engineered to express a Tf receptor. In these examples the Tf fusion protein will typically be engineered or modified to inhibit, prevent or remove glycosylation to extend the serum half-life of the fusion protein and/or therapeutic protein portion. The addition of a targeting peptide is specifically contemplated to further target the Tf fusion protein to a particular cell type, *e.g.,* a cancer cell.

The iron-containing, anti-anemic drug, ferric-sorbitolcitrate complex may be loaded onto a modified Tf fusion protein of the invention. Ferric-sorbitol-citrate (FSC) has been shown to inhibit proliferation of various murine cancer cells *in vitro* and cause tumor regression *in vivo,* while not having any effect on proliferation of non-malignant cells (Poljak-Blazi et al. (June 2000) Cancer Biotherapy and Radiopharmaceuticals (United States), 15/3:285-293).

The antineoplastic drug Adriamycin® (doxorubicin) and/or the chemotherapeutic drug bleomycin, both of which are known to form complexes with ferric ion, may be loaded onto a Tf fusion protein of the invention.

A salt of a drug, for instance, a citrate or carbonate salt, may be prepared and complexed with the ferric iron that is then bound to Tf. As tumor cells often display a higher turnover rate for iron; transferrin modified to carry at least one antitumor agent, may provide a means of increasing agent exposure or load to the tumor cells. (Demant, E.J., (1983) Eur. J. Biochem. 137/(1-2):113-118*;* Padbury et al. (1985) J. Biol. Chem. 260/13:7820-7823).

### Pharmaceutical Formulations and Treatment Methods

The modified fusion proteins comprising a modified transferrin of the invention may be administered to a patient in need thereof using standard administration protocols. For instance, the modified Tf fusion proteins of the present invention can be provided alone, or in combination, or in sequential combination with other agents that modulate a particular pathological process. As used herein, two agents are said to be administered in combination when the two agents are administered simultaneously or are administered independently in a fashion such that the agents will act at the same or near the same time.

The fusion proteins of the present invention can be administered via parenteral, subcutaneous, intravenous, intramuscular, intraperitoneal, transdermal and buccal routes. For example, an agent may be administered locally to a site of injury via microinfusion. Alternatively, or concurrently, administration may be noninvasive by either the oral, inhalation, nasal, or pulmonary route. The dosage administered will be dependent upon the age, health, and weight of the recipient, kind of concurrent treatment, if any, frequency of treatment, and the nature of the effect desired.

While any method of administration may be used to deliver the mTF fusion proteins of the invention, administration or delivery orally may be a preferred embodiment for certain classes of fusion proteins or to treat certain conditions.

The present invention further provides compositions containing one or more fusion proteins of the invention. While individual needs vary, determination of optimal ranges of effective amounts of each component is within the skill of the art. Typical dosages comprise about 1 pg/kg to about 100 mg/kg body weight. The preferred dosages for systemic administration comprise about 100 ng/kg to about 100 mg/kg body weight. The preferred dosages for direct administration to a site via microinfusion comprise about 1 ng/kg to about 1 mg/kg body weight. When administered via direct injection or microinfusion, modified fusion proteins of the invention may be engineered to exhibit reduced or no binding of iron to prevent, in part, localized iron toxicity.

In addition to the pharmacologically active fusion protein, the compositions of the present invention may contain suitable pharmaceutically acceptable carriers comprising excipients and auxiliaries that facilitate processing of the active compounds into preparations which can be used pharmaceutically for delivery to the site of action. Suitable formulations for parenteral administration include aqueous solutions of the active compounds in water-soluble form, for example, water-soluble salts. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides. Aqueous injection suspensions may contain substances which increase the viscosity of the suspension and include, for example, sodium carboxymethyl cellulose, sorbitol and dextran. Optionally, the suspension may also contain stabilizers. Liposomes can also be used to encapsulate the agent for delivery into the cell.

The pharmaceutical formulation for systemic administration according to the invention may be formulated for enteral, parenteral or topical administration. Indeed, all three types of formulations may be used simultaneously to achieve systemic administration of the active ingredient. Suitable formulations for oral administration include hard or soft gelatin capsules, pills, tablets, including coated tablets, elixirs, suspensions, syrups or inhalations and controlled release forms thereof.

The pharmaceutical composition of the present invention can be in unit dosage form, *e.g.* as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form. The dosage to be used in the treatment must be subjectively determined by the physician.

In practicing the methods of this invention, the fusion proteins of this invention may be used alone or in combination, or in combination with other therapeutic or diagnostic agents. In certain preferred embodiments, the compounds of this invention may be co-administered along with other compounds typically prescribed for these conditions according to generally accepted medical practice. The compounds of this invention can be utilized *in vivo,* ordinarily in mammals, such as humans, sheep, horses, cattle, pigs, dogs, cats, rats and mice, or *in vitro.*

### Oral Pharmaceutical Compositions and Delivery Methods

In the present invention, modified Tf fusion proteins, may be formulated for oral delivery. In particular, certain fusion proteins of the invention that are used to treat certain classes of diseases or medical conditions may be particularly amenable for oral formulation and delivery. Such classes of diseases or conditions include, but are not limited to, acute, chronic and recurrent diseases. Chronic or recurrent diseases include, but are not limited to, viral disease or infections, cancer, a metabolic diseases, obesity, autoimmune diseases, inflammatory diseases, allergy, graft-vs.-host disease, systemic microbial infection, anemia, cardiovascular disease, psychosis, genetic diseases, neurodegenerative diseases, disorders of hematopoietic cells, diseases of the endocrine system or reproductive systems, gastrointestinal diseases. Examples of these classes of disease include diabetes, multiple sclerosis, asthma, HCV or HIV infections, hypertension, hypercholesterolemia, arterial scherosis, arthritis, and Alzheimer's disease. In many chronic diseases, oral formulations of Tf fusion proteins of the invention and methods of administration are particularly useful because they allow long-term patient care and therapy via home oral administration without reliance on injectable treatment or drug protocols.

Oral formulations and delivery methods comprising Tf fusion proteins of the invention take advantage of, in part, transferrin receptor mediated transcytosis across the gastrointestinal (GI) epithelium. The Tf receptor is found at a very high density in the human GI epithelium, transferrin is highly resistant to tryptic and chymotryptic digestion and Tf chemical conjugates have been used to successfully deliver proteins and peptides across the GI epithelium (Xia et al., (2000) J. Pharmacol. Experiment. Therap., 295:594-600; Xia et al. (2001) Pharmaceutical Res., 18(2):191-195; and Shah et al. (1996) J. Pharmaceutical Sci., 85(12):1306-1311).

Once transported across the GI epithelium, Tf fusion proteins of the invention exhibit extended half-life in serum, that is, the therapeutic protein or peptide(s) attached or inserted into Tf exhibit an extended serum half-life compared to the protein or peptide in its non-fused state.

Oral formulations of Tf fusion proteins of the invention may be prepared so that they are suitable for transport to the GI epithelium and protection of the Tf fusion protein component and other active components in the stomach. Such formulations may include carrier and dispersant components and may be in any suitable form, including aerosols (for oral or pulmonary delivery), syrups, elixirs, tablets, including chewable tablets, hard or soft capsules, troches, lozenges, aqueous or oily suspensions, emulsions, cachets or pellets granulates, and dispersible powders. Preferably, Tf fusion protein formulations are employed in solid dosage forms suitable for simple, and preferably oral, administration of precise dosages. Solid dosage forms for oral administration are preferably tablets, capsules, or the like.

For oral administration in the form of a tablet or capsule, care should be taken to ensure that the composition enables sufficient active ingredient to be absorbed by the host to produce an effective response. Thus, for example, the amount of Tf fusion protein may be increased over that theoretically required or other known measures such as coating or encapsulation may be taken to protect the polypeptides from enzymatic action in the stomach.

Traditionally, peptide and protein drugs have been administered by injection because of the poor bioavailability when administered non-parenterally, and in particular orally. These drugs are prone to chemical and conformational instability and are often degraded by the acidic conditions in the stomach, as well as by enzymes in the stomach and gastrointestinal tract. In response to these delivery problems, certain technologies for oral delivery have been developed, such as encapsulation in nanoparticles composed of polymers with a hydrophobic backbone and hydrophilic branches as drug carriers, encapsulation in microparticles, insertion into liposomes in emulsions, and conjugation to other molecules. All of which may be used with the Tf fusion molecules of the present invention.

Examples of nanoparticles include mucoadhesive nanoparticles coated with chitosan and Carbopol (Takeuchi et al., Adv. Drug Deliv. Rev. 47(1):39-54, 2001) and nanoparticles containing charged combination polyesters, poly(2-sulfobutyl-vinyl alcohol) and poly(D,L-lactic-co-glycolic acid) (Jung et al., Eur. J. Pharm. Biopharm. 50(1):147-160,2000). Nanoparticles containing surface polymers with poly-N-isopropylacrylamide regions and cationic poly-vinylamine groups showed improved absorption of salmon calcitonin when administered orally to rats.

Drug delivery particles composed of alginate and pectin, strengthened with polylysine, are relatively acid and base resistant and can be used as a carrier for drugs. These particles combine the advantages of bioadhesion, enhanced absorption and sustained release (Liu et al., J. Pharm. Pharmacol. 51(2):141-149, 1999).

Additionally, lipoamino acid groups and liposaccharide groups conjugated to the N- and C-termini of peptides such as synthetic somatostatin, creating an amphipathic surfactant, were shown to produce a composition that retained biological activity (Toth et al., J. Med. Chem. 42(19):4010-4013, 1999).

Examples of other peptide delivery technologies include carbopol-coated mucoadhesive emulsions containing the peptide of interest and either nitroso-N-acetyl-D,L-penicillamine and carbolpol or taurocholate and carbopol. These were shown to be effective when orally administered to rats to reduce serum calcium concentrations (Ogiso et al., Biol. Pharm. Bull. 24(6):656-661,2001). Phosphatidylethanol, derived from phosphatidylcholine, was used to prepare liposomes containing phosphatidylethanol as a carrier of insulin. These liposomes, when administered orally to rats, were shown to be active (Kisel et al., Int. J. Pharm. 216(1-2):105-114, 2001).

Insulin has also been formulated in poly(vinyl alcohol)-gel spheres containing insulin and a protease inhibitor, such as aprotinin or bacitracin. The glucose-lowering properties of these gel spheres have been demonstrated in rats, where insulin is released largely in the lower intestine (Kimura et al., Biol. Pharm. Bull. 19(6):897-900, 1996.

Oral delivery of insulin has also been studied using nanoparticles made of poly(alkyl cyanoacrylate) that were dispersed with a surfactant in an oily phase (Damge et al., J. Pharm. Sci. 86(12):1403-1409, 1997) and using calcium alginate beads coated with chitosan (Onal et al., Artif. Cells Blood Substit. Immobil. Biotechnol. 30(3):229-237, 2002).

In other methods, the N- and C-termini of a peptide are linked to polyethylene glycol and then to allyl chains to form conjugates with improved resistance to enzymatic degradation and improved diffusion through the GI wall (www.nobexcorp.com).

BioPORTER® is a cationic lipid mixture, which interacts non-covalently with peptides to create a protective coating or layer. The peptide-lipid complex can fuse to the plasma membrane of cells, and the peptides are internalized into the cells (www.genetherapysystems.com).

In a process using liposomes as a starting material, cochleate-shaped particles have been developed as a pharmaceutical vehicle. A peptide is added to a suspension of liposomes containing mainly negatively charged lipids. The addition of calcium causes the collapse and fusion of the liposomes into large sheets composed of lipid bilayers, which then spontaneously roll up or stack into cochleates (U.S. Patent 5,840,707; http://www.biodeliverysciences.com).

Compositions comprising Tf fusion protein intended for oral use may be prepared according to any method known to the art for the manufacture of pharmaceutical compositions and such compositions may contain one or more agents selected from the group consisting of sweetening agents in order to provide a pharmaceutically elegant and palatable preparation. For example, to prepare orally deliverable tablets, Tf fusion protein is mixed with at least one pharmaceutical excipient, and the solid formulation is compressed to form a tablet according to known methods, for delivery to the gastrointestinal tract. The tablet composition is typically formulated with additives, e.g. a saccharide or cellulose carrier, a binder such as starch paste or methyl cellulose, a filler, a disintegrator, or other additives typically usually used in the manufacture of medical preparations. To prepare orally deliverable capsules, DHEA is mixed with at least one pharmaceutical excipient, and the solid formulation is placed in a capsular container suitable for delivery to the gastrointestinal tract. Compositions comprising Tf fusion protein may be prepared as described generally in Remington's Pharmaceutical Sciences, 18th Ed. 1990 (Mack Publishing Co. Easton Pa. 18042) at Chapter 89.

As described above, many of the oral formulations of the invention may contain inert ingredients which allow for protection against the stomach environment, and release of the biologically active material in the intestine. Such formulations, or enteric coatings, are well known in the art. For example, tablets containing Tf fusion protein in admixture with non-toxic pharmaceutically acceptable excipients which are suitable for manufacture of tablets may be used. These excipients may be inert diluents, such as calcium carbonate, sodium carbonate, lactose, calcium phosphate or sodium phosphate; granulating and disintegrating agents, for example, maize starch, gelatin or acacia, and lubricating agents, for example, magnesium stearate, stearic acid, or talc.

The tablets may be uncoated or they may be coated with known techniques to delay disintegration and absorption in the gastrointestinal track and thereby provide a sustained action over a longer period of time. For example, a time delay material such as glyceryl monostearate or glyceryl distearate alone or with a wax may be employed.

Formulations for oral use may also be presented as hard gelatin capsules wherein the active ingredient is mixed with an inert solid diluent, for example, calcium carbonate, calcium phosphate, or kaolin or as soft gelatin capsules wherein the active ingredient is mixed with an aqueous or an oil medium, for example, arachis oil, peanut oil, liquid paraffin or olive oil.

Aqueous suspensions may contain Tf fusion protein in the admixture with excipients suitable for the manufacture of aqueous suspensions. Such excipients are suspending agents, for example, sodium carboxymethylcellulose, methylcellulose, hydroxypropylmethylcellulose, sodium alginate, polyvinylpyrrolidone, gum tragacanth and gum acacia; dispersing or wetting agents may be a naturally occurring phosphatide, for example, lecithin, or condensation products of an alkylene oxide with fatty acids, for example, polyoxyethylene stearate, or condensation products of ethylene oxide with long chain aliphatic alcohols, for example, heptadecylethyloxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyoxyethylene sorbitan monooleate. The aqueous suspensions may also contain one or more preservatives for example, ethyl or n-propyl p-hydroxybenzoate, one or more coloring agents, one or more flavoring agents and one or more sweetening agents such as sucrose or saccharin.

Oily suspensions may be formulated by suspending the active ingredient in a vegetable oil, for example, arachis oil, olive oil, sesame oil or coconut oil, or in a mineral oil such as liquid paraffin. The oil suspensions may contain a thickening agent, for example, beeswax, hard paraffin or cetyl alcohol. Sweetening agents, such as those set forth above, and flavoring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient and admixture with dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The pharmaceutical compositions containing Tf fusion protein may also be in the form of oil-in-water emulsions. The oil phase may be a vegetable oil, for example, olive oil or arachis oil, or a mineral oil for example, gum acacia or gum tragacanth, naturally-occurring phosphotides, for example soybean lecithin, and esters or partial esters derived from fatty acids and hexitol anhydrides, for example, sorbitan monooleate, and condensation products of the same partial esters with ethylene oxide, for example, polyoxyethylene sorbitan monooleate. The emulsions may also contain sweetening and flavoring agents.

Syrups and elixirs containing Tf fusion protein may be formulated with sweetening agents, for example, glycerol, sorbitol or sucrose. Such formulations may also contain a demulcent, a preservative and flavoring and coloring agents. The pharmaceutical compositions may be in the form of a sterile injectable preparation, for example, as a sterile injectable aqueous or oleaginous suspension. This suspension may be formulated according to the known art using those suitable dispersing or wetting agents and suspending agents which have been mentioned above. The sterile injectable preparations may also be a sterile injectable solution or suspension in a non-toxic parenterally-acceptable diluent or solvate, for example as a solution in 1, 3-butanediol. Among the acceptable vehicles and solvents that may be employed are water, Ringer's solution and isotonic sodium chloride solution. In addition, sterile, fixed oils are conventionally employed as a solvent or suspending medium. For this period any bland fixed oil may be employed including synthetic mono- or diglycerides. In addition, fatty acids such as oleic acid find use in the preparation of injectables.

Pharmaceutical compositions may also be formulated for oral delivery using polyester microspheres, zein microspheres, proteinoid microspheres, polycyanoacrylate microspheres, and lipid-based systems (see, for example, DiBase and Morrel, Oral Delivery of Microencapsulated Proteins, in Protein Delivery: Physical Systems, Sanders and Hendren (eds.), pages 255-288 (Plenum Press 1997)).

The proportion of pharmaceutically active Tf fusion protein to carrier and/or other substances may vary from about 0.5 to about 100 wt. % (weight percent). For oral use, the pharmaceutical formulation will generally contain from about 5 to about 100% by weight of the active material. For other uses, the formulation will generally have from about 0.5 to about 50 wt. % of the active material.

Tf fusion protein formulations employed in the invention provide an effective amount of Tf fusion protein upon administration to an individual. As used in this context, an "effective amount" of Tf fusion is an amount that is effective to ameliorate a symptom of a disease.

The Tf fusion protein composition of the present invention may be, though not necessarily, administered daily, in an effective amount to ameliorate a symptom. Generally, the total daily dosage will be at least about 50 mg, preferably at least about 100 mg, and more preferably at least about 200 mg, and preferably not more than 500 mg per day, administered orally, e.g., in 4 capsules or tablets, each containing 50 mg Tf fusion protein. Capsules or tablets for oral delivery can conveniently contain up to a full daily oral dose, e.g., 200 mg or more.

Oral pharmaceutical compositions comprising Tf fusion protein may be formulated in buffered liquid form which is then encapsulated into soft or hard-coated gelatin capsules which are then coated with an appropriate enteric coating. For the oral pharmaceutical compositions of the invention, the location of release may be anywhere in the GI system, including the small intestine (the duodenum, the jejunum, or the ileum), or the large intestine.

Oral compositions of the invention may be formulated to slowly release the active ingredients, including the Tf fusion proteins of the invention, in the GI system using known delayed release formulations.

Tf fusion proteins of the invention for oral delivery are capable of binding the Tf receptor found in the GI epithelium. To facilitate this binding and receptor mediated transport, Tf fusion proteins of the invention are typically produced with iron and in some instances carbonate, bound to the Tf moiety. Processes and methods to load the Tf moiety of the fusion protein compositions of the invention with iron and carbonate are known in the art

In some pharmaceutical formulations of the invention, the Tf moiety of the Tf fusion protein may be modified to increase the affinity or avidity of the Tf moiety to iron. Such methods are known in the art. For instance, mutagenesis can be used to produce mutant transferrin moieties that bind iron more avidly than natural transferrin. In human serum transferrin, the amino acids which are ligands for metal ion chelation include, but are not limited to N lobe amino acids Asp63, Tyr 95, Tyr188, Lys206, His207 and His249; and C lobe amino acids Asp392, Tyr426, Tyr517 and His585 of SEQ ID NO: 3 (the number beside the amino acid indicates the position of the amino acid residue in the primary amino acid sequence where the valine of the mature protein is designated position 1). See U.S. Patent 5,986,067.

In one embodiment, the Lys206 and His207 residues within the N lobe are replaced with Gln and Glu, respectively.

In some pharmaceutical formulations of the invention, the Tf fusion protein is engineered to contain a cleavage site between the therapeutic protein or peptide and the Tf moiety. Such cleavable sites or linkers are known in the art.

Pharmaceutical compositions of the invention and methods of treatment may include the addition of a transcytosis enhancer to facilitate transfer of the fusion protein across the GI epithelium. Such enhancers are known in the art. See Xia et al., (2000) J. Pharmacol. Experiment. Therap., 295:594-600; and Xia et al. (2001) Pharmaceutical Res., 18(2):191-195.

In preferred embodiments of the invention, oral pharmaceutical formulations include Tf fusion proteins comprising a modified Tf moiety exhibiting reduced or no glycosylation fused at the N terminal end to an insulin or GLP-1 protein or peptide as described above. Such pharmaceutical compositions may be used to treat glucose imbalance disorders such as diabetes by oral administration of the pharmaceutical composition comprising an effective dose of fusion protein.

The effective dose of fusion protein may be measured in a numbers of ways, including dosages calculated to alleviate symptoms associated with a specific disease state in a patient, such as the symptoms of diabetes. In other formulations, dosages are calculated to comprise an effective amount of fusion protein to induce a detectable change in blood glucose levels in the patient. Such detectable changes in blood glucose may include a decrease in blood glucose levels of between about 1% and 90%, or between about 5% and about 80%. These decreases in blood glucose levels will be dependent on the disease condition being treated and pharmaceutical compositions or methods of administration may be modified to achieve the desired result for each patient. In other instances, the pharmaceutical compositions are formulated and methods of administration modified to detect an increase in the activity level of the therapeutic protein or peptide in the patient, for instance, detectable increases in the activities of insulin or GLP-1. Such formulations and methods may deliver between about 1 pg to about 100 mg /kg body weight of fusion protein, about 100 ng to about 100 µg/kg body weight of fusion protein, about 100 µg/ to about 100 mg/kg body weight of fusion protein, about 1 µg to about 1 g of fusion protein, about 10 µg to about 100 mg of fusion protein or about 10 mg to about 50 mg of fusion protein. Formulations may also be calculated using a unit measurement of therapeutic protein activity, such as about 5 to about 500 units of human insulin or about 10 to about 100 units of human insulin. The measurements by weight or activity can be calculated using known standards for each therapeutic protein or peptide fused to Tf.

Also described here in are methods of orally administering the pharmaceutical compositions of the invention. Such methods may include, but are not limited to, steps of orally administering the compositions by the patient or a caregiver. Such administration steps may include administration on intervals such as once or twice per day depending on the Tf fusion protein, disease or patient condition or individual patient. Such methods also include the administration of various dosages of the individual Tf fusion protein. For instance, the initial dosage of a pharmaceutical composition may be at a higher level to induce a desired effect, such as reduction in blood glucose levels. Subsequent dosages may then be decreased once a desired effect is achieved. These changes or modifications to administration protocols may be done by the attending physician or health care worker. In some instances, the changes in the administration protocol may be done by the individual patient, such as when a patient is monitoring blood glucose levels and administering a mTf-insulin or mTf GLP-1 oral composition of the invention.

Also described are methods of producing oral compositions or medicant compositions of the invention comprising formulating a Tf fusion protein of the invention into an orally administerable form. In other instances, there are described methods of producing compositions or medicant compositions of the invention comprising formulating a Tf fusion protein of the invention into a form suitable for oral administration.

Moreover, described herein is pulmonary delivery of the Tf fusion protein formulations. Pulmonary delivery is particularly promising for the delivery of macromolecules which are difficult to deliver by other routes of administration. Such pulmonary delivery can be effective both for systemic delivery and for localized delivery to treat diseases of the lungs, since drugs delivered to the lung are readily absorbed through the alveolar region directly into the blood circulation.

The present invention provides compositions suitable for forming a drug dispersion for oral inhalation (pulmonary delivery) to treat various conditions or diseases. The Tf fusion protein formulation could be delivered by different approaches such as liquid nebulizers, aerosol-based metered dose inhalers (MDI's), and dry powder dispersion devices. In formulating compositions for pulmonary delivery, pharmaceutically acceptable carriers including surface active agents or surfactants and bulk carriers are commonly added to provide stability, dispersibility, consistency, and/or bulking characteristics to enhance uniform pulmonary delivery of the composition to the subject.

Surface active agents or surfactants promotes absorption of polypeptide through mucosal membrane or lining. Useful surface active agents or surfactants include fatty acids and salts thereof, bile salts, phospholipid, or an alkyl saccharide. Examples of fatty acids and salts thereof include sodium, potassium and lysine salts of caprylate (C₈), caprate (C₁₀), laurate (C₁₂) and myristate (C₁₄). Examples of bile salts include cholic acid, chenodeoxycholic acid, glycocholic acid, taurocholic acid, glycochenodeoxycholic acid, taurochenodeoxycholic acid, deoxycholic acid, glycodeoxycholic acid, taurodeoxycholic acid, lithocholic acid, and ursodeoxycholic acid.
Examples of phospholipids include single-chain phospholipids, such as lysophosphatidylcholine, lysophosphatidylglycerol, lysophosphatidylethanolamine, lysophosphatidylinositol and lysophosphatidylserine; or double-chain phospholipids, such as diacylphosphatidylcholines, diacylphosphatidylglycerols, diacylphosphatidylethanolamines, diacylphosphatidylinositols and diacylphosphatidylserines. Examples of alkyl saccharides include alkyl glucosides or alkyl maltosides, such as decyl glucoside and dodecyl maltoside.

Pharmaceutical excipients that are useful as carriers include stabilizers such as human serum albumin (HSA); bulking agents such as carbohydrates, amino acids and polypeptides; pH adjusters or buffers; salts such as sodium chloride; and the like. These carriers may be in a crystalline or amorphous form or may be a mixture of the two.

Examples of carbohydrates for use as bulking agents include monosaccharides such as galactose, D-mannose, sorbose, and the like; disaccharides, such as lactose, trehalose, and the like; cyclodextrins, such as 2-hydroxypropyl-.beta.-cyclodextrin; and polysaccharides, such as raffinose, maltodextrins, dextrans, and the like; alditols, such as mannitol, xylitol, and the like. Examples of polypeptides for use as bulking agents include aspartame. Amino acids include alanine and glycine, with glycine being preferred.

Additives, which are minor components of the composition, may be included for conformational stability during spray drying and for improving dispersibility of the powder. These additives include hydrophobic amino acids such as tryptophan, tyrosine, leucine, phenylalanine, and the like.

Suitable pH adjusters or buffers include organic salts prepared from organic acids and bases, such as sodium citrate, sodium ascorbate, and the like; sodium citrate is preferred.

The Tf fusion compositions for pulmonary delivery may be packaged as unit doses where a therapeutically effective amount of the composition is present in a unit dose receptacle, such as a blister pack, gelatin capsule, or the like. The manufacture of blister packs or gelatin capsules is typically carried out by methods that are generally well known in the packaging art.

U.S. Patent 6,524,557 discloses a pharmaceutical aerosol formulation comprising (a) a HFA propellant; (b) a pharmaceutically active polypeptide dispersible in the propellant; and (c) a surfactant which is a C₈ -C₁₆ fatty acid or salt thereof, a bile salt, a phospholipid, or an alkyl saccharide, which surfactant enhances the systemic absorption of the polypeptide in the lower respiratory tract. The application also describes methods of manufacturing such formulations and the use of such formulations in treating patients.

One approach for the pulmonary delivery of dry powder drugs utilizes a handheld device with a hand pump for providing a source of pressurized gas. The pressurized gas is abruptly released through a powder dispersion device, such as a venturi nozzle, and the dispersed powder made available for patient inhalation.

Dry powder dispersion devices are described in several patents. U.S. Pat. No. 3,921,637 describes a manual pump with needles for piercing through a single capsule of powdered medicine. The use of multiple receptacle disks or strips of medication is described in European Patent Application No. EP 0 467 172; International Patent Publication Nos. WO 91/02558; and WO 93/09832; U.S. Pat. Nos. 4,627,432; 4,811,731; 5,035,237; 5,048,514; 4,446,862; 5,048,514, and 4,446,862.

The aerosolization of protein therapeutic agents is disclosed in European Patent Application No. EP 0 289 336. Therapeutic aerosol formulations are disclosed in International Patent Publication No. WO 90/09781.

The present invention provides Tf fusion protein formulated for oral inhalation. The formulation comprises Tf fusion protein and suitable pharmaceutical excipients for pulmonary delivery. Also described is administering the Tf fusion protein composition via oral inhalation to subjects in need thereof.

### Transgenic Animals

The production of transgenic non-human animals that contain a modified transferrin fusion construct with increased serum half-life increased serum stability or increased bioavailability of the instant invention is contemplated in one embodiment of the present invention. In some examples , lactoferrin may be used as the Tf portion of the fusion protein so that the fusion protein is produced and secreted in milk.

The successful production of transgenic, non-human animals has been described in a number of patents and publications, such as, for example U.S. Patent 6,291,740 (issued September 18,2001); U.S. Patent 6,281,408 (issued August 28, 2001); and U.S. Patent 6,271,436 (issued August 7,2001).

The ability to alter the genetic make-up of animals, such as domesticated mammals including cows, pigs, goats, horses, cattle, and sheep, allows a number of commercial applications. These applications include the production of animals which express large quantities of exogenous proteins in an easily harvested form (e.g., expression into the milk or blood), the production of animals with increased weight gain, feed efficiency, carcass composition, milk production or content, disease resistance and resistance to infection by specific microorganisms and the production of animals having enhanced growth rates or reproductive performance. Animals which contain exogenous DNA sequences in their genome are referred to as transgenic animals.

The most widely used method for the production of transgenic animals is the microinjection of DNA into the pronuclei of fertilized embryos (Wall et al., J. Cell. Biochem. 49:113 [1992]). Other methods for the production of transgenic animals include the infection of embryos with retroviruses or with retroviral vectors. Infection of both pre- and post-implantation mouse embryos with either wild-type or recombinant retroviruses has been reported (Janenich, Proc. Natl. Acad. Sci. USA 73:1260 [1976]; Janenich et al., Cell 24:519 [1981]; Stuhlmann et al., Proc. Natl. Acad. Sci. USA 81:7151 [1984]; Jahner et al., Proc. Natl. Acad Sci. USA 82:6927 [1985]; Van der Putten et al., Proc. Natl. Acad Sci. USA 82:6148-6152 [1985]; Stewart et al., EMBO J. 6:383-388 [1987]).

An alternative means for infecting embryos with retroviruses is the injection of virus or virus-producing cells into the blastocoele of mouse embryos (Jahner, D. et al., Nature 298:623 [1982]). The introduction of transgenes into the germline of mice has been reported using intrauterine retroviral infection of the midgestation mouse embryo (Jahner *et al.*, supra [1982]). Infection of bovine and ovine embryos with retroviruses or retroviral vectors to create transgenic animals has been reported. These protocols involve the micro-injection of retroviral particles or growth arrested (*i.e.,* mitomycin C-treated) cells which shed retroviral particles into the perivitelline space of fertilized eggs or early embryos (PCT International Application WO 90/08832 [1990]; and Haskell and Bowen, Mol. Reprod. Dev., 40:386 [1995]. PCT International Application WO 90/08832 describes the injection of wild-type feline leukemia virus B into the perivitelline space of sheep embryos at the 2 to 8 cell stage. Fetuses derived from injected embryos were shown to contain multiple sites of integration.

U.S. Patent 6,291,740 (issued September 18, 2001) describes the production of transgenic animals by the introduction of exogenous DNA into pre-maturation oocytes and mature, unfertilized oocytes (*i.e.,* pre-fertilization oocytes) using retroviral vectors which transduce dividing cells (*e.g*., vectors derived from murine leukemia virus [MLV]). This patent also describes methods and compositions for cytomegalovirus promoter-driven, as well as mouse mammary tumor LTR expression of various recombinant proteins.

U.S. Patent 6,281,408 (issued August 28, 2001) describes methods for producing transgenic animals using embryonic stem cells. Briefly, the embryonic stem cells are used in a mixed cell co-culture with a morula to generate transgenic animals. Foreign genetic material is introduced into the embryonic stem cells prior to co-culturing by, for example, electroporation, microinjection or retroviral delivery. ES cells transfected in this manner are selected for integrations of the gene via a selection marker such as neomycin.

U.S. Patent 6,271,436 (issued August 7, 2001) describes the production of transgenic animals using methods including isolation of primordial germ cells, culturing these cells to produce primordial germ cell-derived cell lines, transforming both the primordial germ cells and the cultured cell lines, and using these transformed cells and cell lines to generate transgenic animals. The efficiency at which transgenic animals are generated is greatly increased, thereby allowing the use of homologous recombination in producing transgenic non-rodent animal species.

### Gene Therapy

The use of modified transferrin fusion constructs for gene therapy wherein a modified transferrin protein or transferrin domain is joined to a therapeutic protein or peptide is contemplated. The modified transferrin fusion constructs with increased serum half-life or serum stability of the instant invention are ideally suited to gene therapy treatments.

The successful use of gene therapy to express a soluble fusion protein has been described. Briefly, gene therapy via injection of an adenovirus vector containing a gene encoding a soluble fusion protein consisting of cytotoxic lymphocyte antigen 4 (CTLA4) and the Fc portion of human immunoglubulin G1 was recently shown in Ijima et al. (June 10, 2001) Human Gene Therapy (United States) 12/9:1063-77. In this application of gene therapy, a murine model of type II collagen-induced arthritis was successfully treated via intraarticular injection of the vector.

Gene therapy is also described in a number of U.S. patents including U.S. Pat. 6,225,290 (issued May 1, 2001); U.S. Pat. 6,187,305 (issued February 13,2001); and U.S. Pat. 6,140,111 (issued October 31, 2000).

U.S. Patent 6,225,290 provides methods and constructs whereby intestinal epithelial cells of a mammalian subject are genetically altered to operatively incorporate a gene which expresses a protein which has a desired therapeutic effect. Intestinal cell transformation is accomplished by administration of a formulation composed primarily of naked DNA, and the DNA may be administered orally. Oral or other intragastrointestinal routes of administration provide a simple method of administration, while the use of naked nucleic acid avoids the complications associated with use of viral vectors to accomplish gene therapy. The expressed protein is secreted directly into the gastrointestinal tract and/or blood stream to obtain therapeutic blood levels of the protein thereby treating the patient in need of the protein. The transformed intestinal epithelial cells provide short or long term therapeutic cures for diseases associated with a deficiency in a particular protein or which are amenable to treatment by overexpression of a protein.

U.S. Pat. 6,187,305 provides methods of gene or DNA targeting in cells of vertebrate, particularly mammalian, origin. Briefly, DNA is introduced into primary or secondary cells of vertebrate origin through homologous recombination or targeting of the DNA, which is introduced into genomic DNA of the primary or secondary cells at a preselected site.

U.S. Pat. 6,140,111 (issued October 31, 2000) describes retroviral gene therapy vectors. The disclosed retroviral vectors include an insertion site for genes of interest and are capable of expressing high levels of the protein derived from the genes of interest in a wide variety of transfected cell types. Also disclosed are retroviral vectors lacking a selectable marker, thus rendering them suitable for human gene therapy in the treatment of a variety of disease states without the co-expression of a marker product, such as an antibiotic. These retroviral vectors are especially suited for use in certain packaging cell lines. The ability of retroviral vectors to insert into the genome of mammalian cells has made them particularly promising candidates for use in the genetic therapy of genetic diseases in humans and animals. Genetic therapy typically involves (1) adding new genetic material to patient cells *in vivo,* or (2) removing patient cells from the body, adding new genetic material to the cells and reintroducing them into the body, *i.e., in vitro* gene therapy. Discussions of how to perform gene therapy in a variety of cells using retroviral vectors can be found, for example, in U.S. Pat. Nos. 4,868,116, issued Sep. 19, 1989, and 4,980,286, issued Dec. 25, 1990 (epithelial cells), WO 89/07136 published Aug. 10, 1989 (hepatocyte cells), EP 378,576 published Jul. 25, 1990 (fibroblast cells), and WO 89/05345 published Jun. 15, 1989 and WO/90/06997, published Jun. 28, 1990 (endothelial cells)

### Kits Containing Transferrin Fusion Proteins

Also described herein are kits containing transferrin fusion proteins, which can be used, for instance, for the therapeutic or non-therapeutic applications. The kit comprises a container with a label. Suitable containers include, for example, bottles, vials, and test tubes. The containers may be formed from a variety of materials such as glass or plastic. The container holds a composition which includes a transferrin fusion protein that is effective for therapeutic or non-therapeutic applications, such as described above. The active agent in the composition is the therapeutic protein. The label on the container indicates that the composition is used for a specific therapy or non-therapeutic application, and may also indicate directions for either *in vivo* or *in vitro* use, such as those described above.

The kit will typically comprise the container described above and one or more other containers comprising materials desirable from a commercial and user standpoint, including buffers, diluents, filters, needles, syringes, and package inserts with instructions for use.

Without further description, it is believed that a person of ordinary skill in the art can, using the preceding description and the following illustrative examples, make and utilize the present invention and practice the claimed methods. For example, a skilled artisan would readily be able to determine the biological activity, both *in vitro* and *in vivo*, for the fusion protein constructs of the present invention as compared with the comparable activity of the therapeutic moiety in its unfused state. Similarly, a person skilled in the art could readily determine the serum half life and serum stability of constructs according to the present invention. The following working examples therefore, specifically point out the preferred embodiments of the present invention, and are not to be construed as limiting in any way the remainder of the disclosure.

### EXAMPLES

### Example 1: T-20/Transferrin Fusion Protein

T-20 is a HIV fusogenic inhibitor peptide with the following amino acid sequence: YTSLIHSLIEESQNQQEKNEQELLELDKWASLWNWF (SEQ ID NO: 17). The present disclosure provides fusion proteins comprising T-20 peptide and modified transferrin (mTf) with increased half-life and pharmaceutical compositions of such fusion proteins for the treatment of diseases associated with the transmission of a virus. The example described below may also be used to generate transferrin fusion proteins with analogs of the T-20 peptides.

The transferrin fusion protein with anti-HIV activity was produced by fusing T-20 to modified transferrin (mTf) using *Saccharomyces cerevisiae.* Accordingly, in the first step, SEQ ID NO: 17 was back translated into DNA codon optimized for *Saccharomyces cerevisiae* and used to produce fusion constructs of T-20 at the N- or C-terminus of mTf.

### 5' fusion

As an example, T-20 is fused to the '5' end of mTf using overlapping primers with restriction site overhangs at each end, such as *Xba*I and *Kpn*I sites for ligation into an appropriate vector. Alternatively, overlapping primers with other restriction site overhangs could be generated or the primers could be annealed to adapters with appropriate restriction site overhangs for ligation into a specifically designed vector.

A vector is specifically designed with restriction sites such as *Xba*I and *Kpn*I sites to allow fusion of therapeutic molecules into the N-terminus of mTf. The primers are annealed and cloned into this vector using the *Xba*I and *Kpn*I sites or other appropriate restriction sites at the 5' end of the modified transferrin (mTf) vector. The cassette encoding the T-20/mTf fusion protein is then removed from the vector and cloned into a yeast vector for protein expression

Specifically, the following overlapping primers with restriction overhangs are designed for producing the 5' T-20 fusion: The overlapping primers have the following sequences:
P0038:
   CTAGAGAAAAGGTACACTAGCTTAATACAC (SEQ ID NO: 18)
P0039:
   TGCGATTCTTCAATTAAGGAGTGTATTAAGCTAGTGTACCTTTTCT (SEQ ID NO: 19)
P0040:
   TCCTTAATTGAAGAATCGCAAAACCAGCAAGAAAAGAATG (SEQ ID NO: 20)
P0041:
   TAATTCCAATAATTCTTGTTCATTCTTTTCTTGCTGGTTT (SEQ ID NO: 21)
P0042:
P0043:
   AAACCAATTCCACAAACTTGCCCATTTATC (SEQ ID NO: 23)

The primers are annealed at 65°C and ligated into the specifically designed vector pREX0004, cut with *Xba*I and *Kpn*I to create pREX0016. Following identification of a correct clone by sequencing, the correct clone is bulked up and digested with another pair of appropriate restriction enzymes such as *Psi*I and *Age*I to remove the cassette encoding the T-20/mTf fusion protein out of pREX0016. The cassette is ligated into a yeast vector, such as pSAC3, cut with *Psi*I and *Age*I. pSAC3 containing the T-20/mTf N-terminus fusion protein is then electroporated into yeast for protein production.

### 3' Fusion

In the same manner, T-20 fusion is fused to the 3' end of T-20 using overlapping primers with restriction site overhangs, such as *Sal*I and *Hind*III or other restriction enzymes, at each end. Once annealed, this fragment is cloned into a specifically designed vector, pREX0001, using restriction sites such as *Sal*I and *Hin*dIII sites. Following identification of the correct clone through sequencing, the plasmid is cut with *Sal*I and *Hin*dIII and the fragment is sub-cloned into pREX0004 which is specifically designed to with unique *Sal*I and *Hin*dIII sites to allow fusion of therapeutic protein to the C-terminus of mTf. The resulting plasmid is pREX0017. pREX0017 is then cut with appropriate enzymes such as *Psi*I and *Age*I to remove the T-20/mTf cassette out of the vector. The T-20/m-Tf cassette is sub-cloned into a yeast vector, such as pSAC3, for expression in yeast.

Specifically, the following overlapping primers with restriction overhangs are designed for producing the T-20 fusion to the C-terminus of mTf:

The overlapping primers have the following sequences:
P0044:
   TCGACCTTACACTAGCTTAATACAC (SEQ ID NO: 24)
P0045:
   TGCGATTCTTCAATTAAGGAGTGTATTAAGCTAGTGTAAGG (SEQ ID NO: 25)
P0040:
   TCCTTAATTGAAGAATCGCAAAACCAGCAAGAAAAGAATG (SEQ ID NO: 26)
P0041:
   TAATTCCAATAATTCTTGTTCATTCTTTTCTTGCTGGTTT (SEQ ID NO: 27)
P0046:
P0047:
   AGCTTATTAAAACCAATTCCACAAACTTGCCCATTTATC (SEQ ID NO: 29)

The primers are annealed at 65 °C and ligated into the specifically designed vector cut with *Sal*I and *Hin*dIII. Following identification of a correct clone by sequencing, the correct clone is bulked up and digested with another pair of appropriate restriction enzymes such as *Psi*I and *Age*I to remove the cassette encoding the T-20/mTf C-terminus fusion protein out of the vector. This cassette is ligated into a yeast vector, such as pSAC3, cut with *Psi*I *a*nd *Age*I. pSAC3 containing the T-20/mTf fusion protein is then electroporated into yeast for protein production.

### C-terminus modifications

Modifications are made at the 3' end of mTf to determine if they would effect an increase in fusion expression levels, T-20 fusion activity, and/or improve mobility of the peptide at the C terminus of mTf.

### RRP deletion

In the first step, proline at the 3' end of mTf is removed. In addition to the proline, the adjoining two arginine residues are removed since they may present a potential Kex2p protease cleavage site. The deleted sequence is shown in the alignment below of unmodified 3' T-20 mTf fusion insert (pREX0017, SEQ ID NO: 31) and 3' T-20 mTf fusion insert with RRP deletion (pREX0032, SEQ ID NO: 30):

Specifically, the RRP sequence is deleted using primers homologous to adjoining regions of the sequence. The primers used for generating the deletion are:

P0060:
   TCATCACTCCTGGAAGCCTGCACTTTCTACACTAGCTTAATACACTCCTT (SEQ ID NO: 32)
P0061:
   AAGGAGTGTATTAAGCTAGTGTAGAAAGTGCAGGCTTCCAGGAGTGATGA (SEQ ID NO: 33)

A mutagenic PCR reaction is performed using the external primers and pREX0017 as the template to generate a product missing the 9 bases coding for RRP. This product is then cut with *Sph*I/*Hin*dIII and cloned into *Sph*I/*Hin*dIII cut pREX0017 to make pREX0032. pREX0032 was then cut with *Age*I/*Psi*I, sub-cloned into a yeast vector such as pSAC3 and transformed into yeast for protein expression.

### C402-C674 disulfide removal

In the next step, the disulfide bond that stretches between Cys402 and Cys674 in the mTF molecule, is removed. This is accomplished by mutating both cysteine residues to glycine residues. First, using mutagenic primers Cys402 is mutated to Gly. The mutagenic primers are:
P0064:
   TTGTCTACATAGCGGGCAAGGGTGGTCTGGTGCCTGTCTTG (SEQ ID NO: 34)
P0065:
   CAAGACAGGCACCAGACCACCCTTGCCCGCTATGTAGACAA (SEQ ID NO: 35)

A PCR reaction is performed using the external primers and pREX0017 as the template to generate the product with the desired mutation. This product is then cut with *Hpa*I/*Pst*I and cloned back into pREX0017 making a Gly402 intermediate, pREX0039. Next, Cys674 is mutated into Gly674 following the same procedure and using pREX0004 as a template and the following primers:
P0068:
   TCCACCTCATCACTCCTGGAAGCCGGTACTTTCCGTCGACCTTAA (SEQ ID NO: 36)
P0069:
   CTTATTAAGGTCGACGGAAAGTACCGGCTTCCAGGAGTGATGAGGTGG (SEQ ID NO: 37)

The resulting product is cut with *Sph*I/*Hind*III and sub-cloned into the new Gly402 intermediate, pREX0039, making pREX0038. This plasmid does not contain the T-20 peptide making it useful in the future for other mTf fusions. To insert the T-20 peptide into pREX0038, the *Sal*I/*Age*I fragment of pREX0017 is cloned to form pREX0034. pREX0034 was then cut with *Age*I/*Psi* I. The cassette is sub-cloned into a yeast vector, such as pSAC3, and transformed into yeast for protein expression. The mutations are shown in the alignment below of unmodified 3' T-20 mTf fusion plasmid insert (pREX0017) and 3' T-20 mTf fusion insert with C402-C674 disulfide deletion:

### Disulfide and RRP removal combination

In the third step, RRP deletion and the C402-C674 disulfide deletion are combined. To begin, an intermediate plasmid, pREX0041, is made in the manner as pREX0032 with the exception that the Cys674 mutation was present in the primers. The following primers are used:
P0066:
   TCCACCTCATCACTCCTGGAAGCCGGCACTTTCTACACTAGCTTAATA (SEQ ID NO: 42)
P0067:
   GTGTATTAAGCTAGTGTAGAAAGTACCGGCTTCCAGGAGTGATGAGGT (SEQ ID NO: 43)

pREX0041 is then cut with *Sph*I/*Hin*dIII and sub-cloned into pREX0039 to create pREX0033. pREX0033 is then cut with *Age*I/*Psi*I. The cassette is sub-cloned into a yeast vector, such as pSAC3, and then transformed into yeast for protein expression. The following alignment of the mTf 3' T-20 fusion of pREX0033 with pREX0017 shows the mutations of the resulting product.

### Pharmacokinetics of mTf-T20 in rabbits-

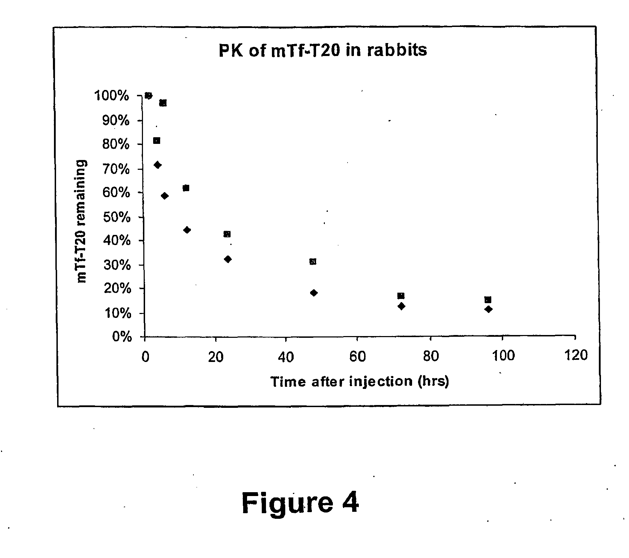

T20 was fused to the C-terminus of mTf by the method described above to generate the mTf-T-20 fusion protein. 50 ug of the mTf-T20 fusion protein was injected intravenously in New Zealand white rabbits and at indicated times (see Fig. 4) 3 ml of blood was removed and plasma was separated and frozen. Plasma samples were later analyzed in an ELISA assay which is specific for human transferrin and does not cross react with endogenous rabbit transferrin. The concentration of mTf-T20 measured at 2 hours after injection is normally the highest and was considered as 100%. All other concentrations where compared to that of the two-hour sample. The elimination half-life was calculated to be 44 ± 2 hrs. Figure 4 shows the pharmacokinetics of mTf-T20 in two rabbits.

mTf has half-life of about 14 days in man and 60 hrs in rabbits. Therefore, a half-life of over 7 days for mTf-T20 in man is anticipated which is significantly longer than the reported 2 hrs for T20.

### Example 2: EMP1/Transferrin Fusion Protein

EMP1 (SEQ ID NO: 11) has been shown to mimic EPO activity by causing dimerization of the EPO receptor. The peptide, which is cyclic, has no homology to EPO. To become active, the peptide has to act in concert with another peptide, *i.e.* as a dimer, such that two copies of the receptor are brought in close enough proximity to form an active complex. As with many peptides, the peptide dimer suffers from short half life and would benefit from the longevity that fusion to transferrin would give. The present disclosure provides fusion protein with EPO mimetic activity. As an example, the fusion protein comprises EMP1 peptide (SEQ ID NO: 11) and modified transferrin (mTf) with increased half-life. The present disclosure also provides pharmaceutical compositions of such fusion proteins for the treatment of diseases associated with low or defective red blood cell production.

### EMP1-mTF fusions and insertions

The initial fusions to mTf comprises fusions to the N-, C-, and N- and C-termini of mTf. The individual fusions will bind the receptor but not cause activation of the receptor. The dual fusion, one of which must be a different codon composition than the other to prevent recombination, will enable binding to the receptor and cause activation.

Examination of the N-domain of human Tf (PDB identifier 1A8E) and the full Tf model AAAaoTfwo, generated using the ExPasy Swiss Model Server with the rabbit model 1JNF as template, reveals a number of potential sites for insertion of a peptide, either directly or by replacement of a number of residues. These sites are duplicated by their equivalent sites in the C domain.

| **N₁** | **N₂** |
|---|---|
| Asp33 | Ser105 |
| Asn55 | Glu141 |
| Asn75 | Asp166 |
| Asp90 | Gln184 |
| Gly257 | Asp197 |
| Lys280 | Lys217 |
| His289 | Thr231 |
| Ser298 | Cys241 |

Two of these loops are the preferred sites into which the EMP1 peptide may be inserted: N₁ His289 (286-292) and N₂ Asp166 (162-170). These positions give the correct orientation required for binding to the two halves of the EPO receptor. As the insertion sites are on the N₁ and N₂ domains of the N domain, they have the flexibility of the hinge between these two sub domains, which allows them to work their way into the receptor.

Due to the structural similarity between the N and C domain the equivalent insertion sites on the C domain (C₁ 489-495, C₂ 623-628) may also be used to make the molecule multivalent. This is done using a variety of the potential insert sites indicated above either on just the N or C domain or by a combination of sites on both domains. N₁ = SEQ ID NO: 13
N₂ = SEQ ID NO: 14
C₁ = SEQ ID NO: 15
C₂ = SEQ ID NO: 16

### Steps for Producing the EMP1/mTf Fusion Protein

In this Example, two EMP1 peptides (SEQ ID NO: 11) are engineered into the transferrin scaffold using the encoding nucleic acids of the peptides and mTf. nucleic acid sequence: SEQ ID NO: 45
amino acid sequence: SEQ ID NO: 11

A EMP1 peptide is engineered into mTf between His289 and Gly290. The duplication inherent to the transferrin molecule, with the two domains mirroring each other, makes it possible to engineer a second EMP1 peptide into the duplicate region of the C domain, between Glu625 and Thr626. N domain: SEQ ID NO: 13
C domain: SEQ ID NO: 16

As an example, for each insertion two overlapping mutagenic primers are synthesized (see below). Using a vector containing the nucleic acid encoding mTf, such as pREX0010, as a template, reactions are performed with each mutagenic primer and an external primer from the 5' or 3' of the Tf cDNA. The products from these two reactions were then mixed and a further reaction performed with the external primers to join the two products together. The His289-Gly290 insert PCR product is digested with *Xba*I and *Hpa*I for ligation into *Xba*I/*Hpa*I digested PREX0010. The resulting vector is then digested with *Hpa*I and *Sal*I for ligation of *Hpa*I/*Sal*I digested Glu625-Thr626 insert PCR product.

The cassette containing the EMP1/mTF fusion protein is cut out of the vector with appropriate restriction enzymes and ligated into a yeast vector, such as pSAC35. pSAC35 is transformed into yeast for protein expression.

Alternative points for insertion of the EPO mimetic peptide(s), or any other peptide(s) are the two glycosylation sites on the C domain of Transferrin at N413 and N611. The advantage of these sites is that once insertion is achieved, glycosylation is prevented by through disruption of the N-X-S/T sequence.

### Example 3: GLP-1/Transferrin Fusion Protein

GLP-1 is a peptide that regulates insulin secretion. It possesses anti-diabetic activity in human subjects suffering diabetes, especially type II diabetes. Like other peptides, GLP-1 has a short plasma half-life in humans. The present invention provides fusion proteins with GLP-1 fused to mTf with increased half-life and pharmaceutical compositions of such fusion proteins for the treatment of diseases associated with abnormal glucose levels that can be administered orally.

The present invention also provides fusion proteins comprising an GLP-1 analog and mTF. In one embodiment of the invention, the GLP-1 analog comprises an additional His residue at the N-terminus. The His residue could be added to the N-terminus of GLP-1 or inserted after the His residue at the N-terminus of GLP-1. In another embodiment, the GLP-1 analog comprises an amino acid substitution at position 2. For example, the Ala in GLP-1(7-36) or GLP-1(7-37) peptide (SEQ ID NO: 6) is substituted with another amino acid.

In this example, the steps for producing a GLP-1/mTf fusion protein are described. The same steps may be used to generate transferrin fusion proteins with analogs of the GLP-1 peptides.

To produce the GLP-1/mTf fusion protein, the amino acid sequence of GLP-1(7-36) and GLP-1(7-37) may be used.
haegtftsdvssylegqaakefiawlvkgr (SEQ ID NO: 48)
haegtftsdvssylegqaakefiawlvkgrg (SEQ ID NO: 64)

For example, the peptide sequence of GLP-1(7-36) may be back translated into DNA and codon optimized for yeast:

The primers were specifically designed to form 5' *Xba*I and 3' *Kpn*I sticky ends after annealing and to enable direct ligation into *Xba*I/*Kpn*I cut pREX0052, just 5' of the end of the leader sequence and at the N-terminus of mTf. Alternatively, other sticky ends may be engineered for ligations into other vectors. Top strand: SEQ ID NO: 50
Bottom strand: SEQ ID NO: 51
Top strand primer: P0056 (nucleotides 7-112 of SEQ ID NO: 50)
Bottom strand primer: P0057 (nucleotides 9-108 of SEQ ID NO: 51)

After annealing and ligation, the clones were sequenced to confirm correct insertion. This vector was designated pREX0094. The cassette was cut out of pREX0094 with *Not*I and sub-cloned into *Not*I cut yeast vector, pSAC35, to make pREX0100.

This plasmid was then electroporated into the host *Saccharomyces* yeast strains and transformants selected for leucine prototrohy on minimal media plates. Expression was determined by growth in liquid minimal media and analysis of supernatant by SDS-PAGE, western blot, and ELISA.

### Example 4: β-IFN/Transferrin Protein

β-IFN is effective in the treatment of various diseases, such as, but not limited to, multiple sclerosis, brain tumor, skin cancer, and hepatitis B and C. Like most cytokines, β-IFN has a short circulation half-life. The present disclosure provides fusion proteins comprising β-IFN fused to mTf with increased half-life and efficacy in patients. This example describes the steps in generating a β-IFN/mTf fusion protein that may be administered orally.

In this example, IFNβ-1 is fused to modified transferrin at either the N- or C-termini. The IFNβ-1 clone was obtained from the ATCC (No. 39517). Specifically designed primers were used to confirm the DNA sequence of the IFNβ-1 clone. These primers were external to the IFNβ-1 DNA sequence and designed to read in from the vector such that the full-length sequence of the clone was obtained. The primers used were:
P0070 GCTATGACCAACAAGTGTCTC (SEQ ID NO: 53)
P0071 CGCACCTGTGGCGCCGGTGATG (SEQ ID NO: 54)

### N-terminal fusion

Once the DNA sequence was confirmed, primers were designed for fusion of IFNβ-1 to mTf. The N-terminal fusion was a two step process. A straight fusion using primers with *Xba*I and *Kpn*I sites would have destroyed the *Kpn*I site and clipped the beginning of mTf. A linker, primers P0082 (nucleotides 18-48 of SEQ ID NO: 55) and P0083 (nucleotides 17-39 SEQ ID NO: 56), was designed to create an internal *Kpn*I site at the 3' end of IFNβ-1, by a single silent mutation of bp 486 from T to G (bold), and with a 5' *Xba*I overhang and 3' GTAC which would anneal with a *Kpn*I site. The overhang destroyed the existing *Kpn*I site in pREX0052. The linkers were annealed and ligated into pREX0054 cut with *Xba*I/*Kpn*I, creating an intermediate vector with mTf untouched and a *Kpn*I site that could be used to fuse the IFNβ-1 gene at the N-terminus of mTf.

A second set of primers, P0084 (SEQ ID NO: 60) and P0085 (SEQ ID NO: 61), were designed to tailor the ends of the IFNβ-1 gene by mutagenic PCR for subsequent insertion into the intermediate vector via the *Xba*I and *Kpn*I sites. A *Xba*I/*Kpn*I digest of this tailored gene removed the last 5 amino acids of IFNβ-1; however, these were already engineered into the intermediate vector. The resulting construct, pREX0048, was created by ligating the IFNβ-1 gene cut with *Xba*I/*Kpn*I into the *Xba*I/*Kpn*I cut intermediate vector.

After the pREX0048 construct was created, it was sequenced to confirm correct insertion. The expression cassette, as a *Not*I fragment, was then sub-cloned into *Not*I cut yeast vector, pSAC35, to make the pREX0050.

### C-terminal fusion

Specifically designed primers, P0086 (SEQ ID NO: 62) and P0087 (SEQ ID NO: 63), were used to PCR amplify the original clone and, in addition, to tailor the ends of IFNβ-1 to have *Sal*I and *Hind*III sites at the 5' and 3' ends, respectively. The newly tailored product was ligated into *Sal*I/*Hin*dIII cut pREX0052 to create pREX0049.

After the Prex0049 construct was created, it was sequenced to confirm correct insertion. The expression cassette, as a *Not*Ifragment, is then sub-cloned into *Not*Icut yeast vector, such as pSAC35, to make pREX0051.

In one example, β-IFN-1 (GenBank Acc. No. NM_002176, SEQ ID NO: 65) is made more stable and soluble by mutating Cys17 (in the mature protein) to Ser. The mutation of Cys 17 to Ser can be performed by routine mutagenic reactions such as a mutagenic PCR reaction using specifically designed primers and the nucleic acid encoding β-IFN-1 as the template.

Further, the β-IFN-1 is modified to prevent glycosylation by modifying the N-linked glycosylation site, NES (residues 80 to 82 of SEQ ID NO: 65)/T. As an example, N could be mutagenized to Q and S/T could be mutagenized to Ala or other amino acid acids. Such mutagenesis could be performed with mutagenic PCR reaction using specifically designed primers and the nucleic acid encoding β-IFN-1 as the template.

### Example 5: Insulin-transferrin fusion proteins

Insulin is a peptide hormone that is secreted by the islets of Langerhans in the pancreas and that regulates the metabolism of carbohydrates and fats, in particular the conversion of glucose to glycogen. It is given to humans suffering from type I and type II diabetes, as well as to diabetic animals. Currently, insulin must be administered by subcutaneous injection and has a short plasma half-life in humans. The present disclosure provides fusion proteins of insulin fused to mTf that have increased half-life and pharmaceutical compositions of such fusion proteins for the treatment of diseases associated with abnormal glucose levels that can be administered orally.

In this example, the steps for producing an insulin/mTf fusion protein are described. Similar steps may be followed to generate transferrin fusion proteins with analogs of insulin peptides.

For expression in *Saccharomyces* constructs were initially made in the base vector pREX0052, comprising an *E. coli* cloning vector with a cassette for the expression of mTf in yeast, as either inserts between the 5' *Xba*I/*Kpn*I sites for the N-terminal fusion, or 3' *Sal*I/*Hind*III sites for the C-terminal fusion.

These constructs form an expression cassette with (5' to 3') the yeast *PRB*1 promoter, leader sequence directing secretion into the growth media, (N-terminal fusion), mTf sequence, (C-terminal fusion), stop codons and the ADH1 terminator sequence. Once constructed, the expression cassettes were recovered as *Not*I fragments and inserted into *Not*I digested pSAC35, an *E.coli*/yeast shuttle vector.

The insulin sequence used corresponds to that of Genbank Accession No. NM_000207, SEQ ID NOS: 71 (DNA) and 72 (protein), as shown below.

The cDNA for the above sequence can be generated in a number of ways, e.g., by RT-PCR, from a cDNA pool, or by overlapping synthetic oligonucleotides. To generate a clone from a cDNA pool, two primers were synthesized and used as PCR primers.

| | |
|---|---|
| 5' primer | 5'-tttgtgaaccaacacctgtgcggc-3' (SEQ ID NO: 73) |
| 3' primer | 3'-gacgagggagatggtcgacctcttgatgacgttg-5' (SEQ ID NO: 74) |

To make the N-terminal insert, a 5' mutagenic primer was used to create a second PCR product using the first PCR product as template. This primer inserted the last 5 amino acids of the leader sequence and the *Xba*I site. The *Kpn*I site could not be inserted by this method, as an amino acid change would have resulted from the creation of the *Kpn*I site. Instead, the PCR product was digested with XbaI/PvuII. A linker was then made of two overlapping oligos with a *Pvu*II 5' end and 3' overhang which would ligate to the *Kpn*I overhang on *Kpn*I digested pREX0052. By annealing and ligating this linker to the digested PCR fragment and ligating the resulting product into *Xba*I/*Kpn*I digested pREX0052 the plasmid pREX0052 N-insulin (SEQ ID NOS: 75 and 76, DNA and protein, respectively) was generated. 5' primer: 5'-gcttactctaggtctctagataagaggtttgtgaaccaacacctgtgcg-3' (SEQ ID NO: 77)
Linkers: 5'-ctggagaactactgcaacgtac-3' (SEQ ID NO: 78)
3'-gacctcttgatgacgttg-5' (SEQ ID NO: 79)

To make the C terminal insert, 5' and 3' mutagenic primers were used to create a second PCR product using the first PCR product as template. This product was then digested with *Sal*I/*Hind*III and ligated into *Sal*I/*Hind*III digested pREX0052. This resulted in the plasmid pREX0052 C-insulin (SEQ ID NOS: 80 and 81). 5' primer: 5'-tgcactttccgtcgaccttttgtgaaccaacacctgtgcg-3' (SEQ ID NO: 82)
3' primer: 3'-gacctcttgatgacgttgattattcgaattaa-5' (SEQ ID NO: 83)

Once the DNA sequence for both the N- and C- terminal inserts had been checked and confimed, the plasmids pREX0052 N-insulin and pREX0052 C-insulin were digested with *Not*I and the expression cassettes recovered. These were then ligated into *Not*I digested pSAC35 to give pSAC35 N-insulin and pSAC35 C-insulin. These plasmids were then electroporated into the host *Saccharomyces* yeast strains and transformants selected for leucine prototrohy on minimal media plates. Expression was determined by growth in liquid minimal media and analysis of superanatant by SDS-PAGE, western blot, ELISA and BIAcore.

These fusion constructs result in the production of proinsulin attached to transferrin. Proteases in yeast may convert the proinsulin to insulin as it is being made and secreted, although the final expression product may contain only proinsulin. In that case, the proinsulin can be converted to insulin post-expression using an appropriate purified protease.

### Oral administration of insulin/modified transferrin fusion protein to rats

To test the insulin activity of the insulin/mTf fusion protein, diabetic rats are first prepared. Female Sprague-Dawley rats are fasted for 24 hours and their blood glucose level determined to establish a baseline. The rats are then injected intraperitoneally with a solution of streptozotocin (STZ), 60 mg/ml, at a dosage of 60 mg/kg. I.p. injections of STZ are continued for four more days, and rats with a fasting blood glucose level above 300 mg/dl are selected as diabetic rats.

Solutions of the fusion protein and of insulin alone are prepared in PBS or sodium bicarbonate to provide dosages of 7 to 80 units of insulin/kg when administered to rats. As a control, rats are also treated with PBS alone. The solutions or PBS are administered by oral gavage to rats following a 12 hour fast, and blood samples are collected from the tail after 0,30 and 60 minutes, and then at 2-hour intervals. Blood glucose levels at 0, 0.5, 1, 3, 5, 7, 9 and 11 hours after dosing are measured with a blood glucose monitoring device designed for diabetics, and the rats are fed again at 11 hours post-dose.

The activity of the insulin is determined by measuring the decrease in blood glucose level over time, correcting the decrease by any increases or decreases in the PBS-only samples. The insulin activities of the fusion protein versus unfused insulin are compared.

To examine the uptake of the fusion protein by transferrin receptors in the intestinal mucosa, fusion protein and unfused insulin as a control are administered to diabetes induced rats as described above and transport measured using standard sandwich ELISAs and serum samples. Alternatively, ¹²⁵I-labeled fusion protein or ¹²⁵I-labeled unfused insulin is administered to diabetes-induced rats at dosage of 80 U insulin/kg by oral gavage, as described above. Blood samples are collected from the tail after 0, 30 and 60 minutes, then at 2-hour intervals, also as described above, and serum samples are analyzed by HPLC, using, for example a Sephacryl column and eluting samples with PBS. Standards containing ¹²⁵I-labeled transferrin, ¹²⁵I-labeled insulin and ¹²⁵I-labeled fusion protein are also run on the Sephacryl column to determine their peak elution times and fraction numbers. The radioactivity of each fraction is measured with a gamma counter, and the protein content of each fraction is measured by the absorbance at 280 nm. Serum samples from rats treated with the fusion protein may not show the appearance of the fusion protein immediately, as there may be a delay of a few hours.

### Example 6: Preparation of therapeutic mTf fusion proteins with increased iron affinity

Therapeutic mTf fusion protein with increased iron affinity may be prepared. As an example for preparing modified transferrin fusion proteins with increased iron binding ability, the procedure in Example 5 above may be carried out with the following modification. These fusion proteins may be used to facilitate uptake and transfer of the fusion protein across the gastrointestinal epithelium.

A cloning vector such as pREX0052, described above, which contains the mTf sequence is cut with a restriction enzyme, or a pair of restriction enzymes, to remove a portion of the mTf gene. Using techniques standard in the art, this fragment is then subjected to site-directed mutagenesis using primers that introduce a mutation at a position corresponding to nucleotide 723 of SEQ ID NO: 1, converting the codon AAG (Lys) to CAG (Gln) or GAG (Glu). Similarly, primers are used that introduce mutations at positions corresponding to nucleotides 726 and 728 of SEQ ID NO: 1, converting the codon CAC (His) to CAG (Gln) or GAG (Glu). Primers may also be used that introduce mutations at all three nucleotide positions, resulting in the substitution of two adjacent amino acids. These nucleotide positions correspond to amino acids 225 and 226 of the protein encoded with the leader sequence and to amino acids 206 and 207 of the mature protein. The mutated fragment is then amplified by RT-PCR and religated into the cloning vector. This vector containing the mutation or mutations is used in a subsequent step for introduction of a DNA molecule coding for the therapeutic protein. As described in Example 5, above, the mTf fusion protein sequence may be introduced into yeast expression vectors and transformed into Saccharomyces or other yeasts for protein production.

As discussed previously, other amino acids may also be mutated to obtain therapeutic mTf proteins with increased iron affinity.

### Example 7: Soluble Toxin Receptor/Transferrin Protein

Clostridial neurotoxins are poisonous substance. Synaptotagmin I is a broad acting receptor of *Clostridium botulinum* neurotoxin serotypes. Amino acids 1-53 (SEQ ID NO: 4) of synaptotagmin I is responsible for binding to various neurotoxin serotypes. Like other peptides, a soluble toxin receptor, such as amino acids 1-53, has a short half-life. The present disclosure provides fusion protein comprising amino acids 1-53 fused mTf with increased half-life as compared to the soluble toxin receptor having SEQ ID NO: 1-53.

The present disclosure provides fusion proteins with anti-toxin activity and increased half-life. Specifically, in this example, a fusion protein comprising modified Tf and a peptide consisting of amino acids 1-53 (SEQ ID NO: 4) of synaptotagmin I, is produced by fusing one or more copies of the nucleotide sequence encoding the peptide to the nucleotide sequence of Tf to produce a fusion protein with a peptide fused to the N- or C-terminus of Tf.

To insert the sequence encoding SEQ ID NO: 4, the vector pREX0010 with the modified transferrin cDNA, is digested with the restriction enzymes *Xba* I/*Kpn*I for insertion at the 5' end and *Sal* I/*Hind* III for insertion at the 3' end.

For the 5' insertion, two overlapping oligos that form an *Xba* I overhang at the 5' end and a *Kpn* I overhang at the 3' end of the nucleic acid encoding SEQ ID NO: 4 are synthesized. These oligos are then annealed together and ligated into the *Xba* I/ *Kpn* I digested pREX0010 vector.

Transformation, selection, and expression are then performed in yeast.

Although the present invention has been described in detail with reference to examples above, it is understood that various modifications can be made.

Accordingly, the invention is limited only by the following claims.

## Claims

1. A fusion protein comprising a modified transferrin (mTf) protein fused to a glucagon-like peptide 1 (GLP-1) molecule, wherein the mTf protein is human transferrin of SEQ ID NO.3 modified by amino acid substitution, deletion or insertion of between 1 and 30 amino acids, and wherein the mTf protein exhibits reduced glycosylation, reduced metal binding, or reduced receptor binding as compared to human transferrin of SEQ ID NO. 3.

2. A fusion protein of claim 1, wherein the GLP-1 molecule is fused to the C-Terminal end of mTf, or is fused to the N-terminal end of mTf, or is inserted into at least one loop of the mTf, or replaces at least one loop of the mTf.

3. A fusion protein of claim 1, wherein the mTf protein has reduced affinity for or does not bind a Tf receptor (TfR).

4. A fusion protein comprising a modified transferrin (mTf) protein fused to a glucagon-like peptide 1 (GLP-1) molecule, wherein the mTf protein is a Tf protein modified by amino acid substitution, deletion or insertion of between 1 and 30 amino acids, and has reduced affinity for or does not bind iron as compared to the Tf protein; and wherein the Tf protein is selected from the group consisting of human Tf, (SEQ ID NO. 3) rabbit Tf (SEQ ID NO. 84), rat Tf (SEQ ID NO. 85), mouse Tf (SEQ ID NO. 86), horse Tf (SEQ ID NO. 87), bovine Tf (SEQ ID NO. 88), pig Tf (SEQ ID NO. 89) and chicken Tf (SEQ ID NO. 90).

5. A fusion protein comprising a modified transferrin (mTf) protein fused to a glucagon-like peptide 1 (GLP-1) molecule, wherein the mTf protein is human transferrin of SEQ ID NO.3 modified by amino acid substitution, deletion or insertion of between 1 and 30 amino acids, and wherein said mTf protein exhibits reduced or no glycosylation as compared to human transferrin of SEQ ID NO. 3.

6. A fusion protein of claim 1 or claim 5, wherein said mTf protein comprises at least one mutation that prevents glycosylation.

7. A fusion protein of claim 1, which is expressed in the presence of tunicamycin.

8. A fusion protein of claim 1, further comprising a linker peptide.

9. A fusion protein of claim 8, wherein the linker peptide links the GLP-1 molecule to mTf.

10. A fusion protein of claim 1, wherein the mTf protein comprises at least one amino acid substitution, deletion or addition in the hinge region.

11. A fusion protein of claim 10, wherein said hinge region is selected from the group consisting of residue 94 to residue 96 of SEQ ID NO: 3, residue 245 to residue 247 of SEQ ID NO: 3, residue 316 to residue 318 of SEQ ID NO: 3, residue 425 to residue 427 of SEQ ID NO: 3, residue 581 to residue 582 of SEQ ID NO: 3, and residue 652 to residue 658 of SEQ ID NO: 3.

12. A fusion protein of claim 6, wherein the mutation is within the N-linked glycosylation site comprising the sequenceAsn-X-Ser/Thr wherein X can be an amino acid except proline.

13. A fusion protein of claim 12, wherein the N-linked glycosylation site is selected from the group consisting of amino acids N413 of SEQ ID NO: 3 and N611 of SEQ ID NO: 3.

14. A fusion protein of claim 1 or 3, wherein the mTf comprises at least one amino acid substitution, deletion or addition at an amino acid residue in SEQ ID NO: 3 selected from the group consisting of Asp 63, Gly 65, Tyr 95, Tyr 188, Lys 206, His 207, His 249, Asp 392, Tyr 426, Tyr 514, Tyr 517, His 585, Thr 120, Arg 124, Ala 126, Gly 127, Thr 452, Arg 456, Ala 458 and Gly 459.

15. A fusion protein of claim 2, wherein the mTf C-terminal proline residue or terminal cysteine loop is deleted.

16. A fusion protein of claim 1, wherein the mTf protein exhibits reduced glycosylation as compared to human transferrin of SEQ ID NO. 3, and wherein the serum half-life of the GLP-1 molecule is increased over the serum half-life of the GLP-1 molecule in an unfused state.

17. The fusion protein of claim 1, wherein the mTf protein has a N-terminal domain at each end of the protein.

18. The fusion protein of claim 17, wherein the GLP-1 molecule is fused to each N-terminal domain of the mTf protein.

19. A fusion protein of claim 2, wherein a GLP-1 molecule is inserted in each of the 5 transferrin loops.

20. A fusion protein of claim 1, wherein the GLP-1 molecule has been modified to prevent dipeptidyl cleavage.

21. A fusion protein of claim 1, wherein the GLP-1 molecule comprises GLP-1 with one or more amino acid additions or substitutions at the N-terminus.

22. The fusion protein of claim 1, wherein the GLP-1 molecule has a chemical modification of the N-terminal amino group.

23. The fusion protein of claim 21, wherein the GLP-1 molecule is fused to modified transferrin protein at the N-terminal end.

24. A fusion protein of claim 1, wherein the GLP-1 molecule is inserted into the N-domain or C-domain of the mTf at one or more of the sites in SEQ ID NO: 3 selected from the group consisting of Asp33, Asn55, Asn 75, Asp90, Gly257, Lys280, His289, Ser298, Ser105, Glu141, Asp166, Gln184, Asp197, Lys217, Thr231, and Cys241.

25. A fusion protein of claim 24, wherein the GLP-1 molecule is inserted into the N-domain of mTf and is further inserted into the mTf at one or more sites in SEQ ID NO: 3 selected from Asp33, Asn55, Asn 75, Asp90, Gly257, Lys280, His289, Ser298, Ser105, Glu141, Asp166, G1n184, Asp197, Lys217, Thr231, and Cys241.

26. A fusion protein of claim 1, wherein the mTf is further modified by deletion of the C-terminus Pro.

27. A fusion protein of claim 26, wherein mTf is further modified by deletion of Arg-Arg adjacent to the C-terminus Pro.

28. A fusion protein of claim 1, wherein the mTf is further modified by removing the disulfide bond between Cys402 and Cys674 of SEQ ID NO: 3.

29. A fusion protein of claim 26 or 27, wherein the mTf is further modified by mutating Cys402 and Cys674 of SEQ ID NO: 3 into Gly residues.

30. A fusion protein of claim 1, wherein the GLP-1 molecule is inserted into one or more of the loops in SEQ ID NO: 3 selected from the group consisting of N₁(286-292), N₂(162-170), C₁(489-495), and C₂(623-628).

31. A fusion protein of claim 1, wherein the mTf protein comprises a single N-terminus domain.

32. A fusion protein according to any one of the preceding claims, wherein the GLP-1 molecule is GLP-1(7-37) or GLP-1 (7-36).

33. A fusion protein according to claim 32, wherein the GLP-1 molecule is GLP-1(7-37) consisting of SEQ ID NO: 6 or GLP-1 (7-36) consisting of amino acids 1-30 of SEQ ID NO: 6.

34. A fusion protein according to claim 32, wherein the Ala at the second position in SEQ ID NO: 6 has been mutated.

35. A fusion protein according to claim 34, wherein Ala at the second position in SEQ ID NO: 6 has been mutated to Gly, Ser or Val.

36. A nucleic acid molecule encoding a fusion protein of any one of the preceding claims.

37. A vector comprising a nucleic acid molecule of claim 36.

38. A host cell comprising a vector of claim 37.

39. A host cell comprising a nucleic acid molecule of claim 36.

40. A method of expressing a mTf fusion protein comprising culturing a host cell of claim 38 or 39 under conditions which express the encoded fusion protein.

41. A host cell of claim 38 or 40, wherein the cell is prokaryotic or eukaryotic.

42. A host cell of claim 41, wherein the cell is a yeast cell.

43. A non human transgenic animal comprising a nucleic acid molecule of claim 36.

44. A pharmaceutical composition comprising the fusion protein of any one of claims 1 to 35 and a carrier.

45. A method of producing a mTf fusion protein comprising isolating a fusion protein from a transgenic animal of claim 43.

46. A fusion protein of any one of claims 1 to 35 for use in a method of medical treatment.

47. A fusion protein according to claim 46 for treating an elevated level of glucose as compared to a healthy subject.

48. A fusion protein according to claim 47, wherein the elevated level of glucose is associated with diabetes.

49. A fusion protein according to claim 48, wherein the diabetes is Type II diabetes.

50. A fusion protein according to claim 46 for treating congestive heart failure.

51. A fusion protein according to claim 46 for treating obesity.

52. A fusion protein according to claim 46 for regulating glucose level in a subject.

## Patentansprüche

1. Fusionsprotein, das ein modifiziertes Transferrin- (mTf-) Protein umfasst, das an ein Glucagon-ähnliches-Peptid-1- (GLP-1-) Molekül fusioniert ist, worin das mTf-Protein menschliches Transferrin der Seq.-ID Nr. 3 ist, das durch Aminosäuresubstitution, -deletion oder -insertion von zwischen 1 und 30 Aminosäuren modifiziert wurde, und worin das mTf-Protein verglichen mit menschlichem Transferrin der Seq.-ID Nr. 3 eine verminderte Glykosylierung, eine verminderte Metallbindung oder eine verminderte Rezeptorbindung aufweist.

2. Fusionsprotein nach Anspruch 1, worin das GLP-1-Molekül an das C-terminale Ende von mTf fusioniert ist oder an das N-terminale Ende von mTf fusioniert ist oder in zumindest eine Schleife des mTf insertiert ist oder zumindest eine Schleife des mTf ersetzt.

3. Fusionsprotein nach Anspruch 1, worin das mTf-Protein eine reduzierte Affinität für einen Tf-Rezeptor (TfR) aufweist oder diesen nicht bindet.

4. Fusionsprotein, das ein modifziertes Transferrin- (mTf-) Protein umfasst, das an ein Glucagon-ähnliches-Peptid-1- (GLP-1-) Molekül fusioniert ist, worin das mTf-Protein ein Tf-Protein ist, das durch Aminosäuresubstitution, -deletion oder -insertion von zwischen 1 und 30 Aminosäuren modifiziert wurde und im Vergleich mit dem Tf-Protein eine verminderte Affinität für Eisen aufweist oder dieses nicht bindet; und worin das Tf-Protein aus der Gruppe bestehend aus menschlichem Tf, (Seq.-ID Nr. 3), Kaninchen-Tf (Seq.-ID Nr. 84), Ratten-Tf (Seq.-ID Nr. 85), Mäuse-Tf (Seq.-ID Nr. 86), Pferde-Tf (Seq.-ID Nr. 87), Rinder-Tf (Seq.-ID Nr. 88), Schweine-Tf (Seq.-ID Nr. 89) und Hühner-Tf (Seq.-ID Nr. 90) ausgewählt ist.

5. Fusionsprotein, das ein modifiziertes Transferrin- (mTf-) Protein umfasst, das an ein Glucagon-ähnliches-Peptid-1- (GLP-1-) Molekül fusioniert ist, worin das mTf-Protein menschliches Transferrin der Seq.-ID Nr. 3 ist, das durch Aminosäuresubstitution, -deletion oder -insertion von zwischen 1 und 30 Aminosäuren modifiziert wurde, und worin das mTf-Protein im Vergleich zu menschlichem Transferrin der Seq.-ID Nr. 3 eine verringerte oder keine Glykosylierung aufweist.

6. Fusionsprotein nach Anspruch 1 oder Anspruch 5, worin das mTf-Protein zumindest eine Mutation umfasst, die die Glykosylierung verhindert.

7. Fusionsprotein nach Anspruch 1, das in der Gegenwart von Tunicamycin exprimiert wird.

8. Fusionsprotein nach Anspruch 1, das ferner ein Linker-Peptid umfasst.

9. Fusionsprotein nach Anspruch 8, worin das Linker-Peptid das GLP-1-Molekül mit mTf verbindet.

10. Fusionsprotein nach Anspruch 1, worin das mTf-Protein zumindest eine Aminosäuresubstitution, -deletion oder -addition in der Gelenksregion umfasst.

11. Fusionsprotein nach Anspruch 10, worin die Gelenksregion aus der Gruppe bestehend aus Rest 94 bis Rest 96 aus Seq.-ID Nr. 3, Rest 245 bis Rest 247 aus Seq.-ID Nr. 3, Rest 316 bis Rest 318 aus Seq.-ID Nr. 3, Rest 425 bis Rest 427 aus Seq.-ID Nr. 3, Rest 581 bis Rest 582 aus Seq.-ID Nr. 3 und Rest 652 bis Rest 658 aus Seq.-ID Nr. 3 ausgewählt ist.

12. Fusionsprotein nach Anspruch 6, worin die Mutation innerhalb der N-gebundenen Glykosylierungsstelle liegt, die die Sequenz Asn-X-Ser/Thr umfasst, worin X eine Aminosäure außer Prolin sein kann.

13. Fusionsprotein nach Anspruch 12, worin die N-gebundene Glykosylierungsstelle aus der Gruppe bestehend aus Aminosäuren N413 aus Seq.-ID Nr. 3 und N611 aus Seq.-ID Nr. 3 ausgewählt ist.

14. Fusionsprotein nach Anspruch 1 oder 3, worin das mTf zumindest eine Aminosäuresubstitution, -deletion oder -addition an einem Aminosäurerest in Seq.-ID Nr. 3 umfasst, der aus der Gruppe bestehend aus Asp 63, Gly 65, Tyr 95, Tyr 188, Lys 206, His 207, His 249, Asp 392, Tyr 426, Tyr 514, Tyr 517, His 585, Thr 120, Arg 124, Ala 126, Gly 127, Thr 452, Arg 456, Ala 458 und Gly 459 ausgewählt ist.

15. Fusionsprotein nach Anspruch 2, worin der mTf-C-terminale Prolinrest oder die endständige Cysteinschleife deletiert ist.

16. Fusionsprotein nach Anspruch 1, worin das mTf-Protein verglichen mit menschlichem Transferrin aus Seq.-ID Nr. 3 eine verminderte Glykosylierung aufweist und worin die Serumhalbwertszeit des GLP-1-Moleküls über die Serumhalbwertszeit des GLP-1-Moleküls in nichtfusioniertem Zustand verlängert ist.

17. Fusionsprotein nach Anspruch 1, worin das mTf-Protein eine N-terminale Domäne an jedem Ende des Proteins aufweist.

18. Fusionsprotein nach Anspruch 17, worin das GLP-1-Molekül an jede N-terminale Domäne des mTf-Proteins fusioniert ist.

19. Fusionsprotein nach Anspruch 2, worin ein GLP-1-Molekül in jede der 5 Transferrin-Schleifen insertiert ist.

20. Fusionsprotein nach Anspruch 1, worin das GLP-1-Molekül modifiziert wurde, um eine Dipeptidyl-Spaltung zu verhindern.

21. Fusionsprotein nach Anspruch 1, worin das GLP-1-Molekül GLP-1 mit einer oder mehreren Aminosäureadditionen oder -substitutionen am N-Terminus umfasst.

22. Fusionsprotein nach Anspruch 1, worin das GLP-1-Molekül eine chemische Modifikation der N-terminalen Aminogruppe aufweist.

23. Fusionsprotein nach Anspruch 21, worin das GLP-1-Molekül an dem N-terminalen Ende an das modifizierte Transferrin-Protein fusioniert ist.

24. Fusionsprotein nach Anspruch 1, worin das GLP-1-Molekül in die N-Domäne oder C-Domäne des mTf an einer oder mehreren Stellen in Seq.-ID Nr. 3, ausgewählt aus der Gruppe bestehend aus Asp33, Asn55, Asn75, Asp90, Gly257, Lys280, His289, Ser298, Ser105, Glu141, Asp166, Gln184, Asp197, Lys217, Thr231 und Cys241, insertiert ist.

25. Fusionsprotein nach Anspruch 24, worin das GLP-1-Molekül in die N-Domäne von mTf insertiert ist und ferner in das mTf an einer oder mehreren Stellen in Seq.-ID Nr. 3, ausgewählt aus Asp33, Asn55, Asn75, Asp90, Gly257, Lys280, His289, Ser298, Ser105, Glu141, Asp166, Gln184, Asp197, Lys217, Thr231 und Cys241, insertiert ist.

26. Fusionsprotein nach Anspruch 1, worin das mTf ferner durch Deletion des C-Terminus Pro modifiziert ist.

27. Fusionsprotein nach Anspruch 26, worin mTf ferner durch Deletion von Arg-Arg benachbart zu dem C-Terminus Pro modifiziert ist.

28. Fusionsprotein nach Anspruch 1, worin das mTf ferner durch Entfernen der Disulfidbindung zwischen Cys402 und Cys674 von Seq.-ID Nr. 3 modifiziert ist.

29. Fusionsprotein nach Anspruch 26 oder 27, worin das mTf ferner durch Mutieren von Cys402 und Cys674 von Seq.-ID Nr. 3 zu Gly-Resten modifiziert ist.

30. Fusionsprotein nach Anspruch 1, worin das GLP-1-Molekül in eine oder mehrere der Schleifen in Seq.-ID Nr. 3, ausgewählt aus der Gruppe bestehend aus N₁ (286-292), N₂ (162-170), C₁ (489-495) und C₂ (623-628), insertiert ist.

31. Fusionsprotein nach Anspruch 1, worin das mTf-Protein eine einzige N-terminale Domäne umfasst.

32. Fusionsprotein nach einem der vorangegangenen Ansprüche, worin das GLP-1-Molekül GLP-1 (7-37) oder GLP-1 (7-36) ist.

33. Fusionsprotein nach Anspruch 32, worin das GLP-1-Molekül GLP-1 (7-37) ist, das aus Seq.-ID Nr. 6 besteht, oder GLP-1 (7-36) ist, das aus Aminosäuren 1-30 von Seq.-ID Nr. 6 besteht.

34. Fusionsprotein nach Anspruch 32, worin das Ala an der zweiten Position in Seq.-ID Nr. 6 mutiert wurde.

35. Fusionsprotein nach Anspruch 34, worin Ala an der zweiten Position in Seq.-ID Nr. 6 zu Gly, Ser oder Val mutiert wurde.

36. Nucleinsäuremolekül, das für ein Fusionsprotein nach einem der vorangegangenen Ansprüche kodiert.

37. Vektor, der ein Nucleinsäuremolekül nach Anspruch 36 umfasst.

38. Wirtszelle, die einen Vektor nach Anspruch 37 umfasst.

39. Wirtszelle, die ein Nucleinsäuremolekül nach Anspruch 36 umfasst.

40. Verfahren zum Exprimieren eines mTf-Fusionsproteins, das das Kultivieren einer Wirtszelle nach Anspruch 38 oder 39 unter Bedingungen umfasst, unter denen das kodierte Fusionsprotein exprimiert wird.

41. Wirtszelle nach Anspruch 38 oder 40, worin die Zelle prokaryotisch oder eukaryotisch ist.

42. Wirtszelle nach Anspruch 41, worin die Zelle eine Hefezelle ist.

43. Nichtmenschliches transgenes Tier, das ein Nucleinsäuremolekül nach Anspruch 36 umfasst.

44. Pharmazeutische Zusammensetzung, die das Fusionsprotein nach einem der Ansprüche 1 bis 35 und einen Träger umfasst.

45. Verfahren zur Herstellung eines mTf-Fusionsproteins, das das Isolieren eines Fusionsproteins aus einem transgenen Tier nach Anspruch 43 umfasst.

46. Fusionsprotein nach einem der Ansprüche 1 bis 35 zur Verwendung in einem Verfahren einer medizinischen Behandlung.

47. Fusionsprotein nach Anspruch 46 zur Behandlung eines im Vergleich zu einem gesunden Individuum erhöhten Glucosewerts.

48. Fusionsprotein nach Anspruch 47, worin der erhöhte Glucosewert im Zusammenhang mit Diabetes steht.

49. Fusionsprotein nach Anspruch 48, worin der Diabetes Typ-II-Diabetes ist.

50. Fusionsprotein nach Anspruch 46 zur Behandlung von kongestivem Herzversagen.

51. Fusionsprotein nach Anspruch 46 zur Behandlung von Adipositas.

52. Fusionsprotein nach Anspruch 46 zur Regulation des Glucosewerts in einem Individuum.

## Revendications

1. Protéine de fusion comprenant une protéine transferrine modifiée (mTf) fusionnée à une molécule de peptide analogue au glucagon-1 (GLP-1), dans laquelle la protéine mTf est la transferrine humaine de SEQ ID NO: 3 modifiée par une substitution, une délétion ou une insertion d'acides aminés de 1 à 30 acides aminés, et dans laquelle la protéine mTf exhibe une glycosylation réduite, une liaison aux métaux réduite, ou une liaison à un récepteur réduite par comparaison à la transferrine humaine de SEQ ID NO: 3.

2. Protéine de fusion selon la revendication 1, dans laquelle la molécule GLP-1 est fusionnée à l'extrémité C-terminale de la mTf, ou est fusionnée à l'extrémité N-terminale de la mTf, ou est insérée dans au moins une boucle de la mTf, ou remplace au moins une boucle de la mTf.

3. Protéine de fusion selon la revendication 1, dans laquelle la protéine mTf possède une affinité réduite pour ou ne se lie pas à un récepteur de la Tf (TfR).

4. Protéine de fusion comprenant une protéine transferrine modifiée (mTf) fusionnée à une molécule de peptide analogue au glucagon-1 (GLP-1), dans laquelle la protéine mTf est une protéine Tf modifiée par une substitution, une délétion ou une insertion d'acides aminés de 1 à 30 acides aminés, et possède une affinité réduite pour ou ne se lie pas au fer par comparaison à la protéine Tf ; et dans laquelle la protéine Tf est sélectionnée dans le groupe consistant en la Tf humaine (SEQ ID NO: 3), la Tf du lapin (SEQ ID NO: 84), la Tf du rat (SEQ ID NO: 85), la Tf de la souris (SEQ ID NO: 86), la Tf du cheval (SEQ ID NO: 87), la Tf bovine (SEQ ID NO: 88), la Tf du porc (SEQ ID NO: 89) et la Tf du poulet (SEQ ID NO: 90).

5. Protéine de fusion comprenant une protéine transferrine modifiée (mTf) fusionnée à une molécule de peptide analogue au glucagon-1 (GLP-1), dans laquelle la protéine mTf est la transferrine humaine de SEQ ID NO: 3 modifiée par une substitution, une délétion ou une insertion d'acides aminés de 1 à 30 acides aminés, et dans laquelle ladite protéine mTf exhibe une glycosylation réduite ou aucune glycosylation par comparaison à la transferrine humaine de SEQ ID NO: 3.

6. Protéine de fusion selon la revendication 1 ou la revendication 5, dans laquelle ladite protéine mTf comprend au moins une mutation qui empêche la glycosylation.

7. Protéine de fusion selon la revendication 1, qui est exprimée en présence de tunicamycine.

8. Protéine de fusion selon la revendication 1, comprenant en outre un peptide de liaison.

9. Protéine de fusion selon la revendication 8, dans laquelle le peptide de liaison relie la molécule GLP-1 à la mTf.

10. Protéine de fusion selon la revendication 1, dans laquelle la protéine mTf comprend au moins une substitution, une délétion ou une addition d'acides aminés dans la région charnière.

11. Protéine de fusion selon la revendication 10, dans laquelle ladite région charnière est sélectionnée dans le groupe consistant en le résidu 94 au résidu 96 de SEQ ID NO: 3, le résidu 245 au résidu 247 de SEQ ID NO: 3, le résidu 316 au résidu 318 de SEQ ID NO: 3, le résidu 425 au résidu 427 de SEQ ID NO: 3, le résidu 581 au résidu 582 de SEQ ID NO: 3, et le résidu 652 au résidu 658 de SEQ ID NO: 3.

12. Protéine de fusion selon la revendication 6, dans laquelle la mutation est au sein du site de glycosylation N-lié comprenant la séquence Asn-X-Ser/Thr, dans laquelle X peut être un acide aminé à l'exception de la proline.

13. Protéine de fusion selon la revendication 12, dans laquelle le site de glycosylation N-lié est sélectionné dans le groupe consistant en les acides aminés N413 de SEQ ID NO: 3 et N611 de SEQ ID NO: 3.

14. Protéine de fusion selon la revendication 1 ou 3, dans laquelle la mTf comprend au moins une substitution, une délétion ou une addition d'acides aminés au niveau d'un résidu d'acide aminé dans SEQ ID NO: 3 sélectionné dans le groupe consistant en Asp 63, Gly 65, Tyr 95, Tyr 188, Lys 206, His 207, His 249, Asp 392, Tyr 426, Tyr 514, Tyr 517, His 585, Thr 120, Arg 124, Ala 126, Gly 127, Thr 452, Arg 456, Ala 458 et Gly 459.

15. Protéine de fusion selon la revendication 2, dans laquelle le résidu proline C-terminal ou la boucle cystéine terminale de la mTf est supprimé(e).

16. Protéine de fusion selon la revendication 1, dans laquelle la protéine mTf exhibe une glycosylation réduite par comparaison à la transferrine humaine de SEQ ID NO: 3, et dans laquelle la demi-vie sérique de la molécule GLP-1 est augmentée par rapport à la demi-vie sérique de la molécule GLP-1 dans un état non fusionné.

17. Protéine de fusion selon la revendication 1, dans laquelle la protéine mTf possède un domaine N-terminal à chaque extrémité de la protéine.

18. Protéine de fusion selon la revendication 17, dans laquelle la molécule GLP-1 est fusionnée à chaque domaine N-terminal de la protéine mTf.

19. Protéine de fusion selon la revendication 2, dans laquelle une molécule GLP-1 est insérée dans chacune des 5 boucles de la transferrine.

20. Protéine de fusion selon la revendication 1, dans laquelle la molécule GLP-1 a été modifiée pour empêcher un clivage dipeptidyle.

21. Protéine de fusion selon la revendication 1, dans laquelle la molécule GLP-1 comprend la GLP-1 avec une ou plusieurs additions ou substitutions d'acides aminés à la terminaison N.

22. Protéine de fusion selon la revendication 1, dans laquelle la molécule GLP-1 comporte une modification chimique du groupe aminé N-terminal.

23. Protéine de fusion selon la revendication 21, dans laquelle la molécule GLP-1 est fusionnée à la protéine transferrine modifiée au niveau de l'extrémité N-terminale.

24. Protéine de fusion selon la revendication 1, dans laquelle la molécule GLP-1 est insérée dans le domaine N ou le domaine C de la mTf au niveau d'un ou de plusieurs des sites dans SEQ ID NO: 3 sélectionnés dans le groupe consistant en Asp33, Asn55, Asn75, Asp90, Gly257, Lys280, His289, Ser298, Ser105, Glu141, Asp166, Gln184, Asp197, Lys217, Thr231 et Cys241.

25. Protéine de fusion selon la revendication 24, dans laquelle la molécule GLP-1 est insérée dans le domaine N de la mTf et est en outre insérée dans la mTf au niveau d'un ou de plusieurs sites dans SEQ ID NO: 3 sélectionnés parmi Asp33, Asn55, Asn75, Asp90, Gly257, Lys280, His289, Ser298, Ser105, Glu141, Asp166, Gln184, Asp197, Lys217, Thr231 et Cys241.

26. Protéine de fusion selon la revendication 1, dans laquelle la mTf est en outre modifiée par une délétion de la Pro C-terminale.

27. Protéine de fusion selon la revendication 26, dans laquelle la mTf est en outre modifiée par une délétion de Arg-Arg en position adjacente à la Pro C-terminale.

28. Protéine de fusion selon la revendication 1, dans laquelle la mTf est en outre modifiée par une élimination de la liaison disulfure entre Cys402 et Cys674 de SEQ ID NO: 3.

29. Protéine de fusion selon la revendication 26 ou 27, dans laquelle la mTf est en outre modifiée par une mutation de Cys402 et Cys674 de SEQ ID NO: 3 en résidus Gly.

30. Protéine de fusion selon la revendication 1, dans laquelle la molécule GLP-1 est insérée dans une ou plusieurs des boucles dans SEQ ID NO: 3 sélectionnées dans le groupe consistant en N₁(286-292), N₂(162-170), C₁(489-495) et C₂(623-628).

31. Protéine de fusion selon la revendication 1, dans laquelle la protéine mTf comprend un seul domaine N-terminal.

32. Protéine de fusion selon l'une quelconque des revendications précédentes, dans laquelle la molécule GLP-1 est GLP-1 (7-37) ou GLP-1 (7-36).

33. Protéine de fusion selon la revendication 32, dans laquelle la molécule GLP-1 est GLP-1 (7-37) consistant en SEQ ID NO: 6 ou GLP-1 (7-36) consistant en les acides aminés 1-30 de SEQ ID NO: 6.

34. Protéine de fusion selon la revendication 32, dans laquelle l'Ala à la deuxième position dans SEQ ID NO: 6 a été mutée.

35. Protéine de fusion selon la revendication 34, dans laquelle l'Ala à la deuxième position dans SEQ ID NO: 6 a été mutée en Gly, Ser ou Val.

36. Molécule d'acide nucléique codant pour une protéine de fusion selon l'une quelconque des revendications précédentes.

37. Vecteur comprenant une molécule d'acide nucléique selon la revendication 36.

38. Cellule hôte comprenant un vecteur selon la revendication 37.

39. Cellule hôte comprenant une molécule d'acide nucléique selon la revendication 36.

40. Procédé d'expression d'une protéine de fusion mTf comprenant la mise en culture d'une cellule hôte selon la revendication 38 ou 39 dans des conditions qui expriment la protéine de fusion codée.

41. Cellule hôte selon la revendication 38 ou 40, où la cellule est procaryote ou eucaryote.

42. Cellule hôte selon la revendication 41, où la cellule est une cellule de levure.

43. Animal transgénique non humain comprenant une molécule d'acide nucléique selon la revendication 36.

44. Composition pharmaceutique comprenant la protéine de fusion selon l'une quelconque des revendications 1 à 35 et un véhicule.

45. Procédé de production d'une protéine de fusion mTf comprenant l'isolement d'une protéine de fusion à partir d'un animal transgénique selon la revendication 43.

46. Protéine de fusion selon l'une quelconque des revendications 1 à 35 destinée à être utilisée dans un procédé de traitement médical.

47. Protéine de fusion selon la revendication 46 destinée au traitement d'un taux de glucose élevé par comparaison à un sujet sain.

48. Protéine de fusion selon la revendication 47, où le taux de glucose élevé est associé au diabète.

49. Protéine de fusion selon la revendication 48, où le diabète est le diabète de type II.

50. Protéine de fusion selon la revendication 46 destinée au traitement de l'insuffisance cardiaque congestive.

51. Protéine de fusion selon la revendication 46 destinée au traitement de l'obésité.

52. Protéine de fusion selon la revendication 46 destinée à la régulation du taux de glucose chez un sujet.


### SEQUENCE LISTING

<110> PRIOR, Christopher P. LAI, Char-Huei SADEGHI, Homayoun TURNER, Andrew J.
<120> MODIFIED TRANSFERRIN FUSION PROTEINS
<130> 54710-5001-01-WO
<150> US 60/406,977
   <151> 2002-08-30
<150> US 10/378,094
   <151> 2003-03-04
<160> 90
<170> PatentIn version 3.2
<210> 1
   <211> 2318
   <212> DNA
   <213> Homo sapiens
<220>
   <221> CDS
   <222> (51) .. (2147)
   <223> GenBank Acc. No. NM_001063, transferrin gene and protein
<220>
   <221> sig_peptide
   <222> (51)..(107)
<400> 1
   gcacagaagc gagtccgact gtgctcgctg ctcagcgccg cacccggaag atg agg       56
                                                          Met Arg
                                                          1
   ctc gcc gtg gga gcc ctg ctg gtc tgc gcc gtc ctg ggg ctg tgt ctg     104
   Leu Ala Val Gly Ala Leu Leu Val Cys Ala Val Leu Gly Leu Cys Leu
           5                   10                  15
   gct gtc cct gat aaa act gtg aga tgg tgt gca gtg tcg gag cat gag     152
   Ala Val Pro Asp Lys Thr Val Arg Trp Cys Ala Val Ser Glu His Glu
       20                  25                  30
   gcc act aag tgc cag agt ttc cgc gac cat atg aaa agc gtc att cca     200
   Ala Thr Lys Cys Gln Ser Phe Arg Asp His Met Lys Ser Val Ile Pro
   35                  40                  45                  50
   tcc gat ggt ccc agt gtt gct tgt gtg aag aaa gcc tcc tac ctt gat     248
   Ser Asp Gly Pro Ser Val Ala Cys Val Lys Lys Ala Ser Tyr Leu Asp
                   55                  60                  65
   tgc atc agg gcc att gcg gca aac gaa gcg gat gct gtg aca ctg gat     296
   Cys Ile Arg Ala Ile Ala Ala Asn Glu Ala Asp Ala Val Thr Leu Asp
               70                  75                  80
   gca ggt ttg gtg tat gat gct tac ctg gct ccc aat aac ctg aag cct     344
   Ala Gly Leu Val Tyr Asp Ala Tyr Leu Ala Pro Asn Asn Leu Lys Pro
           85                  90                  95
   gtg gtg gca gag ttc tat ggg tca aaa gag gat cca cag act ttc tat      392
   Val Val Ala Glu Phe Tyr Gly Ser Lys Glu Asp Pro Gln Thr Phe Tyr
       100                 105                 110
   tat gct gtt gct gtg gtg aag aag gat agt ggc ttc cag atg aac cag      440
   Tyr Ala Val Ala Val Val Lys Lys Asp Ser Gly Phe Gln Met Asn Gln
   115                 120                 125                 130
   ctt cga ggc aag aag tcc tgc cac acg ggt cta ggc agg tcc gct ggg      488
   Leu Arg Gly Lys Lys Ser Cys His Thr Gly Leu Gly Arg Ser Ala Gly
                   135                 140                 145
   tgg aac atc ccc ata ggc tta ctt tac tgt gac tta cct gag cca cgt      536
   Trp Asn Ile Pro Ile Gly Leu Leu Tyr Cys Asp Leu Pro Glu Pro Arg
               150                 155                 160
   aaa cct ctt gag aaa gca gtg gcc aat ttc ttc tcg ggc agc tgt gcc      584
   Lys Pro Leu Glu Lys Ala Val Ala Asn Phe Phe Ser Gly Ser Cys Ala
           165                 170                 175
   cct tgt gcg gat ggg acg gac ttc ccc cag ctg tgt caa ctg tgt cca      632
   Pro Cys Ala Asp Gly Thr Asp Phe Pro Gln Leu Cys Gln Leu Cys Pro
       180                 185                 190
   ggg tgt ggc tgc tcc acc ctt aac caa tac ttc ggc tac tcg gga gcc      680
   Gly Cys Gly Cys Ser Thr Leu Asn Gln Tyr Phe Gly Tyr Ser Gly Ala
   195                 200                 205                 210
   ttc aag tgt ctg aag gat ggt gct ggg gat gtg gcc ttt gtc aag cac      728
   Phe Lys Cys Leu Lys Asp Gly Ala Gly Asp Val Ala Phe Val Lys His
                   215                 220                 225
   tcg act ata ttt gag aac ttg gca aac aag gct gac agg gac cag tat      776
   Ser Thr Ile Phe Glu Asn Leu Ala Asn Lys Ala Asp Arg Asp Gln Tyr
               230                 235                 240
   gag ctg ctt tgc ctg gac aac acc cgg aag ccg gta gat gaa tac aag      824
   Glu Leu Leu Cys Leu Asp Asn Thr Arg Lys Pro Val Asp Glu Tyr Lys
           245                 250                 255
   gac tgc cac ttg gcc cag gtc cct tct cat acc gtc gtg gcc cga agt      872
   Asp Cys His Leu Ala Gln Val Pro Ser His Thr Val Val Ala Arg Ser
       260                 265                 270
   atg ggc ggc aag gag gac ttg atc tgg gag ctt ctc aac cag gcc cag      920
   Met Gly Gly Lys Glu Asp Leu Ile Trp Glu Leu Leu Asn Gln Ala Gln
   275                 280                 285                 290
   gaa cat ttt ggc aaa gac aaa tca aaa gaa ttc caa cta ttc agc tct      968
   Glu His Phe Gly Lys Asp Lys Ser Lys Glu Phe Gln Leu Phe Ser Ser
                   295                 300                 305
   cct cat ggg aag gac ctg ctg ttt aag gac tct gcc cac ggg ttt tta     1016
   Pro His Gly Lys Asp Leu Leu Phe Lys Asp Ser Ala His Gly Phe Leu
               310                 315                 320
   aaa gtc ccc ccc agg atg gat gcc aag atg tac ctg ggc tat gag tat     1064
   Lys Val Pro Pro Arg Met Asp Ala Lys Met Tyr Leu Gly Tyr Glu Tyr
             325                 330                 335
   gtc act gcc atc cgg aat cta cgg gaa ggc aca tgc cca gaa gcc cca      1112
   Val Thr Ala Ile Arg Asn Leu Arg Glu Gly Thr Cys Pro Glu Ala Pro
       340                 345                 350
   aca gat gaa tgc aag cct gtg aag tgg tgt gcg ctg agc cac cac gag      1160
   Thr Asp Glu Cys Lys Pro Val Lys Trp Cys Ala Leu Ser His His Glu
   355                 360                 365                 370
   agg ctc aag tgt gat gag tgg agt gtt aac agt gta ggg aaa ata gag      1208
   Arg Leu Lys Cys Asp Glu Trp Ser Val Asn Ser Val Gly Lys Ile Glu
                   375                 380                 385
   tgt gta tca gca gag acc acc gaa gac tgc atc gcc aag atc atg aat      1256
   Cys Val Ser Ala Glu Thr Thr Glu Asp Cys Ile Ala Lys Ile Met Asn
               390                 395                 400
   gga gaa gct gat gcc atg agc ttg gat gga ggg ttt gtc tac ata gcg      1304
   Gly Glu Ala Asp Ala Met Ser Leu Asp Gly Gly Phe Val Tyr Ile Ala
           405                 410                 415
   ggc aag tgt ggt ctg gtg cct gtc ttg gca gaa aac tac aat aag agc      1352
   Gly Lys Cys Gly Leu Val Pro Val Leu Ala Glu Asn Tyr Asn Lys Ser
       420                 425                 430
   gat aat tgt gag gat aca cca gag gca ggg tat ttt gct gta gca gtg      1400
   Asp Asn Cys Glu Asp Thr Pro Glu Ala Gly Tyr Phe Ala Val Ala Val
   435                 440                 445                 450
   gtg aag aaa tca gct tct gac ctc acc tgg gac aat ctg aaa ggc aag      1448
   Val Lys Lys Ser Ala Ser Asp Leu Thr Trp Asp Asn Leu Lys Gly Lys
                   455                 460                 465
   aag tcc tgc cat acg gca gtt ggc aga acc gct ggc tgg aac atc ccc      1496
   Lys Ser Cys His Thr Ala Val Gly Arg Thr Ala Gly Trp Asn Ile Pro
               470                 475                 480
   atg ggc ctg ctc tac aat aag atc aac cac tgc aga ttt gat gaa ttt      1544
   Met Gly Leu Leu Tyr Asn Lys Ile Asn His Cys Arg Phe Asp Glu Phe
           485                 490                 495
   ttc agt gaa ggt tgt gcc cct ggg tct aag aaa gac tcc agt ctc tgt      1592
   Phe Ser Glu Gly Cys Ala Pro Gly Ser Lys Lys Asp Ser Ser Leu Cys
       500                 505                 510
   aag ctg tgt atg ggc tca ggc cta aac ctg tgt gaa ccc aac aac aaa      1640
   Lys Leu Cys Met Gly Ser Gly Leu Asn Leu Cys Glu Pro Asn Asn Lys
   515                 520                 525                 530
   gag gga tac tac ggc tac aca ggc gct ttc agg tgt ctg gtt gag aag      1688
   Glu Gly Tyr Tyr Gly Tyr Thr Gly Ala Phe Arg Cys Leu Val Glu Lys
                   535                 540                 545
   gga gat gtg gcc ttt gtg aaa cac cag act gtc cca cag aac act ggg      1736
   Gly Asp Val Ala Phe Val Lys His Gln Thr Val Pro Gln Asn Thr Gly
               550                 555                 560
   gga aaa aac cct gat cca tgg gct aag aat ctg aat gaa aaa gac tat      1784
   Gly Lys Asn Pro Asp Pro Trp Ala Lys Asn Leu Asn Glu Lys Asp Tyr
           565                 570                 575
   gag ttg ctg tgc ctt gat ggt acc agg aaa cct gtg gag gag tat gcg      1832
   Glu Leu Leu Cys Leu Asp Gly Thr Arg Lys Pro Val Glu Glu Tyr Ala
       580                 585                 590
   aac tgc cac ctg gcc aga gcc ccg aat cac gct gtg gtc aca cgg aaa      1880
   Asn Cys His Leu Ala Arg Ala Pro Asn His Ala Val Val Thr Arg Lys
   595                 600                 605                 610
   gat aag gaa gct tgc gtc cac aag ata tta cgt caa cag cag cac cta      1928
   Asp Lys Glu Ala Cys Val His Lys Ile Leu Arg Gln Gln Gln His Leu
                   615                 620                 625
   ttt gga agc aac gta act gac tgc tcg ggc aac ttt tgt ttg ttc cgg      1976
   Phe Gly Ser Asn Val Thr Asp Cys Ser Gly Asn Phe Cys Leu Phe Arg
               630                 635                 640
   tcg gaa acc aag gac ctt ctg ttc aga gat gac aca gta tgt ttg gcc      2024
   Ser Glu Thr Lys Asp Leu Leu Phe Arg Asp Asp Thr Val Cys Leu Ala
           645                 650                 655
   aaa ctt cat gac aga aac aca tat gaa aaa tac tta gga gaa gaa tat      2072
   Lys Leu His Asp Arg Asn Thr Tyr Glu Lys Tyr Leu Gly Glu Glu Tyr
       660                 665                 670
   gtc aag gct gtt ggt aac ctg aga aaa tgc tcc acc tca tca ctc ctg      2120
   Val Lys Ala Val Gly Asn Leu Arg Lys Cys Ser Thr Ser Ser Leu Leu
   675                 680                 685                 690
   gaa gcc tgc act ttc cgt aga cct taa aatctcagag gtagggctgc            2167
   Glu Ala Cys Thr Phe Arg Arg Pro
                   695
   caccaaggtg aagatgggaa cgcagatgat ccatgagttt gccctggttt cactggccca    2227
   agtggtttgt gctaaccacg tctgtcttca cagctctgtg ttgccatgtg tgctgaacaa    2287
   aaaataaaaa ttattattga ttttatattt c                                   2318
<210> 2
   <211> 698
   <212> PRT
   <213> Homo sapiens
<400> 2
   Met Arg Leu Ala Val Gly Ala Leu Leu Val Cys Ala Val Leu Gly Leu
   1               5                   10                  15
   Cys Leu Ala Val Pro Asp Lys Thr Val Arg Trp Cys Ala Val Ser Glu
               20                  25                  30
   His Glu Ala Thr Lys Cys Gln Ser Phe Arg Asp His Met Lys Ser Val
           35                  40                  45
   Ile Pro Ser Asp Gly Pro Ser Val Ala Cys Val Lys Lys Ala Ser Tyr
       50                  55                  60
   Leu Asp Cys Ile Arg Ala Ile Ala Ala Asn Glu Ala Asp Ala Val Thr
   65                  70                  75                  80
   Leu Asp Ala Gly Leu Val Tyr Asp Ala Tyr Leu Ala Pro Asn Asn Leu
   85 90 95
   Lys Pro Val Val Ala Glu Phe Tyr Gly Ser Lys Glu Asp Pro Gln Thr
   100 105 110
   Phe Tyr Tyr Ala Val Ala Val Val Lys Lys Asp Ser Gly Phe Gln Met
   115 120 125
   Asn Gln Leu Arg Gly Lys Lys Ser Cys His Thr Gly Leu Gly Arg Ser
   130 135 140
   Ala Gly Trp Asn Ile Pro Ile Gly Leu Leu Tyr Cys Asp Leu Pro Glu
   145 150 155 160
   Pro Arg Lys Pro Leu Glu Lys Ala Val Ala Asn Phe Phe Ser Gly Ser
   165 170 175
   Cys Ala Pro Cys Ala Asp Gly Thr Asp Phe Pro Gln Leu Cys Gln Leu
   180 185 190
   Cys Pro Gly Cys Gly Cys Ser Thr Leu Asn Gln Tyr Phe Gly Tyr Ser
   195 200 205
   Gly Ala Phe Lys Cys Leu Lys Asp Gly Ala Gly Asp Val Ala Phe Val
   210 215 220
   Lys His Ser Thr Ile Phe Glu Asn Leu Ala Asn Lys Ala Asp Arg Asp
   225 230 235 240
   Gln Tyr Glu Leu Leu Cys Leu Asp Asn Thr Arg Lys Pro Val Asp Glu
   245 250 255
   Tyr Lys Asp Cys His Leu Ala Gln Val Pro Ser His Thr Val Val Ala
   260 265 270
   Arg Ser Met Gly Gly Lys Glu Asp Leu Ile Trp Glu Leu Leu Asn Gln
   275 280 285
   Ala Gln Glu His Phe Gly Lys Asp Lys Ser Lys Glu Phe Gln Leu Phe
   290 295 300
   Ser Ser Pro His Gly Lys Asp Leu Leu Phe Lys Asp Ser Ala His Gly
   305 310 315 320
   Phe Leu Lys Val Pro Pro Arg Met Asp Ala Lys Met Tyr Leu Gly Tyr
   325 330 335
   Glu Tyr Val Thr Ala Ile Arg Asn Leu Arg Glu Gly Thr Cys Pro Glu
   340 345 350
   Ala Pro Thr Asp Glu Cys Lys Pro Val Lys Trp Cys Ala Leu Ser His
   355 360 365
   His Glu Arg Leu Lys Cys Asp Glu Trp Ser Val Asn Ser Val Gly Lys
   370 375 380
   Ile Glu Cys Val Ser Ala Glu Thr Thr Glu Asp Cys Ile Ala Lys Ile
   385 390 395 400
   Met Asn Gly Glu Ala Asp Ala Met Ser Leu Asp Gly Gly Phe Val Tyr
                   405                 410                 415
   Ile Ala Gly Lys Cys Gly Leu Val Pro Val Leu Ala Glu Asn Tyr Asn
               420                 425                 430
   Lys Ser Asp Asn Cys Glu Asp Thr Pro Glu Ala Gly Tyr Phe Ala Val
           435                 440                 445
   Ala Val Val Lys Lys Ser Ala Ser Asp Leu Thr Trp Asp Asn Leu Lys
       450                 455                 460
   Gly Lys Lys Ser Cys His Thr Ala Val Gly Arg Thr Ala Gly Trp Asn
   465                 470                 475                 480
   Ile Pro Met Gly Leu Leu Tyr Asn Lys Ile Asn His Cys Arg Phe Asp
                   485                 490                 495
   Glu Phe Phe Ser Glu Gly Cys Ala Pro Gly Ser Lys Lys Asp Ser Ser
               500                 505                 510
   Leu Cys Lys Leu Cys Met Gly Ser Gly Leu Asn Leu Cys Glu Pro Asn
           515                 520                 525
   Asn Lys Glu Gly Tyr Tyr Gly Tyr Thr Gly Ala Phe Arg Cys Leu Val
       530                 535                 540
   Glu Lys Gly Asp Val Ala Phe Val Lys His Gln Thr Val Pro Gln Asn
   545                 550                 555                 560
   Thr Gly Gly Lys Asn Pro Asp Pro Trp Ala Lys Asn Leu Asn Glu Lys
                   565                 570                 575
   Asp Tyr Glu Leu Leu Cys Leu Asp Gly Thr Arg Lys Pro Val Glu Glu
               580                 585                 590
   Tyr Ala Asn Cys His Leu Ala Arg Ala Pro Asn His Ala Val Val Thr
           595                 600                 605
   Arg Lys Asp Lys Glu Ala Cys Val His Lys Ile Leu Arg Gln Gln Gln
       610                 615                 620
   His Leu Phe Gly Ser Asn Val Thr Asp Cys Ser Gly Asn Phe Cys Leu
   625                 630                 635                 640
   Phe Arg Ser Glu Thr Lys Asp Leu Leu Phe Arg Asp Asp Thr Val Cys
                   645                 650                 655
   Leu Ala Lys Leu His Asp Arg Asn Thr Tyr Glu Lys Tyr Leu Gly Glu
               660                 665                 670
   Glu Tyr Val Lys Ala Val Gly Asn Leu Arg Lys Cys Ser Thr Ser Ser
           675                 680                 685
   Leu Leu Glu Ala Cys Thr Phe Arg Arg Pro
       690                 695
<210> 3
   <211> 679
   <212> PRT
   <213> Homo sapiens
<220>
   <221> MISC_FEATURE
   <223> Mature Transferrin Protein
<400> 3
   Val Pro Asp Lys Thr Val Arg Trp Cys Ala Val Ser Glu His Glu Ala
   1               5                   10                  15
   Thr Lys Cys Gln Ser Phe Arg Asp His Met Lys Ser Val Ile Pro Ser
               20                  25                  30
   Asp Gly Pro Ser Val Ala Cys Val Lys Lys Ala Ser Tyr Leu Asp Cys
           35                  40                  45
   Ile Arg Ala Ile Ala Ala Asn Glu Ala Asp Ala Val Thr Leu Asp Ala
       50                  55                  60
   Gly Leu Val Tyr Asp Ala Tyr Leu Ala Pro Asn Asn Leu Lys Pro Val
   65                  70                  75                  80
   Val Ala Glu Phe Tyr Gly Ser Lys Glu Asp Pro Gln Thr Phe Tyr Tyr
                   85                  90                  95
   Ala Val Ala Val Val Lys Lys Asp Ser Gly Phe Gln Met Asn Gln Leu
               100                 105                 110
   Arg Gly Lys Lys Ser Cys His Thr Gly Leu Gly Arg Ser Ala Gly Trp
           115                 120                 125
   Asn Ile Pro Ile Gly Leu Leu Tyr Cys Asp Leu Pro Glu Pro Arg Lys
       130                 135                 140
   Pro Leu Glu Lys Ala Val Ala Asn Phe Phe Ser Gly Ser Cys Ala Pro
   145                 150                 155                 160
   Cys Ala Asp Gly Thr Asp Phe Pro Gln Leu Cys Gln Leu Cys Pro Gly
                   165                 170                 175
   Cys Gly Cys Ser Thr Leu Asn Gln Tyr Phe Gly Tyr Ser Gly Ala Phe
               180                 185                 190
   Lys Cys Leu Lys Asp Gly Ala Gly Asp Val Ala Phe Val Lys His Ser
           195                 200                 205
   Thr Ile Phe Glu Asn Leu Ala Asn Lys Ala Asp Arg Asp Gln Tyr Glu
       210                 215                 220
   Leu Leu Cys Leu Asp Asn Thr Arg Lys Pro Val Asp Glu Tyr Lys Asp
   225                 230                 235                 240
   Cys His Leu Ala Gln Val Pro Ser His Thr Val Val Ala Arg Ser Met
                   245                 250                 255
   Gly Gly Lys Glu Asp Leu Ile Trp Glu Leu Leu Asn Gln Ala Gln Glu
               260                 265                 270
   His Phe Gly Lys Asp Lys Ser Lys Glu Phe Gln Leu Phe Ser Ser Pro
           275                 280                 285
   His Gly Lys Asp Leu Leu Phe Lys Asp Ser Ala His Gly Phe Leu Lys
   290 295 300
   Val Pro Pro Arg Met Asp Ala Lys Met Tyr Leu Gly Tyr Glu Tyr Val
   305 310 315 320
   Thr Ala Ile Arg Asn Leu Arg Glu Gly Thr Cys Pro Glu Ala Pro Thr
   325 330 335
   Asp Glu Cys Lys Pro Val Lys Trp Cys Ala Leu Ser His His Glu Arg
   340 345 350
   Leu Lys Cys Asp Glu Trp Ser Val Asn Ser Val Gly Lys Ile Glu Cys
   355 360 365
   Val Ser Ala Glu Thr Thr Glu Asp Cys Ile Ala Lys Ile Met Asn Gly
   370 375 380
   Glu Ala Asp Ala Met Ser Leu Asp Gly Gly Phe Val Tyr Ile Ala Gly
   385 390 395 400
   Lys Cys Gly Leu Val Pro Val Leu Ala Glu Asn Tyr Asn Lys Ser Asp
   405 410 415
   Asn Cys Glu Asp Thr Pro Glu Ala Gly Tyr Phe Ala Val Ala Val Val
   420 425 430
   Lys Lys Ser Ala Ser Asp Leu Thr Trp Asp Asn Leu Lys Gly Lys Lys
   435 440 445
   Ser Cys His Thr Ala Val Gly Arg Thr Ala Gly Trp Asn Ile Pro Met
   450 455 460
   Gly Leu Leu Tyr Asn Lys Ile Asn His Cys Arg Phe Asp Glu Phe Phe
   465 470 475 480
   Ser Glu Gly Cys Ala Pro Gly Ser Lys Lys Asp Ser Ser Leu Cys Lys
   485 490 495
   Leu Cys Met Gly Ser Gly Leu Asn Leu Cys Glu Pro Asn Asn Lys Glu
   500 505 510
   Gly Tyr Tyr Gly Tyr Thr Gly Ala Phe Arg Cys Leu Val Glu Lys Gly
   515 520 525
   Asp Val Ala Phe Val Lys His Gln Thr Val Pro Gln Asn Thr Gly Gly
   530 535 540
   Lys Asn Pro Asp Pro Trp Ala Lys Asn Leu Asn Glu Lys Asp Tyr Glu
   545 550 555 560
   Leu Leu Cys Leu Asp Gly Thr Arg Lys Pro Val Glu Glu Tyr Ala Asn
   565 570 575
   Cys His Leu Ala Arg Ala Pro Asn His Ala Val Val Thr Arg Lys Asp
   580 585 590
   Lys Glu Ala Cys Val His Lys Ile Leu Arg Gln Gln Gln His Leu Phe
   595 600 605
   Gly Ser Asn Val Thr Asp Cys Ser Gly Asn Phe Cys Leu Phe Arg Ser
   610 615 620
   Glu Thr Lys Asp Leu Leu Phe Arg Asp Asp Thr Val Cys Leu Ala Lys
   625 630 635 640
   Leu His Asp Arg Asn Thr Tyr Glu Lys Tyr Leu Gly Glu Glu Tyr Val
   645 650 655
   Lys Ala Val Gly Asn Leu Arg Lys Cys Ser Thr Ser Ser Leu Leu Glu
   660 665 670
   Ala Cys Thr Phe Arg Arg Pro
   675
<210> 4
   <211> 53
   <212> PRT
   <213> Homo sapiens
<220>
   <221> misc_feature
   <223> Amino acids 1-53 of synaptotagmin I
<400> 4
   Met Val Ser Glu Ser His His Glu Ala Leu Ala Ala Pro Pro Val Thr
   1 5 10 15
   Thr Val Ala Thr Val Leu Pro Ser Asn Ala Thr Glu Pro Ala Ser Pro
   20 25 30
   Gly Glu Gly Lys Glu Asp Ala Phe Ser Lys Leu Lys Glu Lys Phe Met
   35 40 45
   Asn Glu Leu His Lys
   50
<210> 5
   <211> 12
   <212> PRT
   <213> Artificial sequence
<220>
   <223> Neutrophil lactoferrin splice variant
<400> 5
   Glu Asp Cys Ile Ala Leu Lys Gly Glu Ala Asp Ala
   1 5 10
<210> 6
   <211> 31
   <212> PRT
   <213> Homo sapiens
<220>
   <221> MISC_FEATURE
   <223> Glucagon-Like Peptide
<220>
   <221> misc_feature
   <222> (31) .. (31)
   <223> Xaa can be any naturally occurring amino acid
<220>
   <221> MISC_FEATURE
   <222> (37) .. (37)
   <223> Xaa can be Gly in GLP-1 or NH2 in GLP-2
<400> 6
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg Xaa
               20                  25                  30
<210> 7
   <211> 28
   <212> PRT
   <213> Artificial sequence
<220>
   <223> GLP-1 molecule having insulinotropic activity
<400> 7
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys
               20                  25
<210> 8
   <211> 29
   <212> PRT
   <213> Artificial sequence
<220>
   <223> GLP-1 molecule having insulinotropic activity
<400> 8
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly
               20                  25
<210> 9
   <211> 29
   <212> PRT
   <213> Artificial sequence
<220>
   <223> GLP-1 analog
<220>
   <221> MISC_FEATURE
   <222> (1)..(1)
   <223> Xaa can be Ala, Gly, Val, Thr, Ile, or alpha-methyl-Ala
<220>
   <221> MISC_FEATURE
   <222> (14) .. (14)
   <223> Xaa can be Glu, Gln, Ala, Thr, Ser, or Gly
<220>
   <221> MISC_FEATURE
   <222> (20) .. (20)
   <223> Xaa can be Glu, Gln, Ala, Thr, Ser or Gly
<400> 9
   Xaa Glu Gly Thr Phe Thr Ser Asp Val Ser Ser Tyr Leu Xaa Gly Gln
   1               5                   10                  15
   Ala Ala Lys Xaa Phe Ile Ala Trp Leu Val Lys Gly Arg
               20                  25
<210> 10
   <211> 10
   <212> PRT
   <213> Artificial sequence
<220>
   <223> EPO domain
<400> 10
   Cys Arg Ile Gly Pro Ile Thr Trp Val Cys
   1               5                   10
<210> 11
   <211> 20
   <212> PRT
   <213> Artificial sequence
<220>
   <223> EMP1 peptide
<400> 11
   Gly Gly Thr Tyr Ser Cys His Phe Gly Pro Leu Thr Trp Val Cys Lys
   1               5                   10                  15
   Pro Gln Gly Gly
               20
<210> 12
   <211> 13
   <212> PRT
   <213> Artificial sequence
<220>
   <223> EMP20 peptide
<400> 12
   Tyr Ser Cys His Phe Gly Pro Leu Thr Trp Val Cys Lys
   1 5 10
<210> 13
   <211> 47
   <212> PRT
   <213> Homo sapiens
<220>
   <221> misc_feature
   <223> N1 subdomain of transferrin
<400> 13
   Asp Lys Ser Lys Glu Phe Gln Leu Phe Ser Ser Pro His Gly Lys Asp
   1 5 10 15
   Leu Leu Phe Lys Asp Ser Ala His Gly Phe Leu Lys Val Pro Pro Arg
   20 25 30
   Met Asp Ala Lys Met Tyr Leu Gly Tyr Glu Tyr Val Thr Ala Ile
   35 40 45
<210> 14
   <211> 45
   <212> PRT
   <213> Homo sapiens
<220>
   <221> misc_feature
   <223> N2 subdomain of Transferrin
<400> 14
   Pro Glu Pro Arg Lys Pro Leu Glu Lys Ala Val Ala Asn Phe Phe Ser
   1 5 10 15
   Gly Ser Cys Ala Pro Cys Ala Asp Gly Thr Asp Phe Pro Gln Leu Cys
   20 25 30
   Gln Leu Cys Pro Gly Cys Gly Cys Ser Thr Leu Asn Gln
   35 40 45
<210> 15
   <211> 42
   <212> PRT
   <213> Homo sapiens
<220>
   <221> misc_feature
   <223> C1 subdomain of transferrin
<400> 15
   Asn His Cys Arg Phe Asp Glu Phe Phe Ser Glu Gly Cys Ala Pro Gly
   1               5                   10                  15
   Ser Lys Lys Asp Ser Ser Leu Cys Lys Leu Cys Met Gly Ser Gly Leu
               20                  25                  30
   Asn Leu Cys Glu Pro Asn Asn Lys Glu Gly
           35                  40
<210> 16
   <211> 49
   <212> PRT
   <213> Homo sapiens
<220>
   <221> misc_feature
   <223> C2 subdomain of transferrin
<400> 16
   Asn Val Thr Asp Cys Ser Gly Asn Phe Cys Leu Phe Arg Ser Glu Thr
   1               5                   10                  15
   Lys Asp Leu Leu Phe Arg Asp Asp Thr Val Cys Leu Ala Lys Leu His
               20                  25                  30
   Asp Arg Asn Thr Tyr Glu Lys Tyr Leu Gly Glu Glu Tyr Val Lys Ala
           35                  40                  45
   Val
<210> 17
   <211> 36
   <212> PRT
   <213> Human immunodeficiency virus
<220>
   <223> HIV T-20 antifusogenic peptide
<400> 17
   Tyr Thr Ser Leu Ile His Ser Leu Ile Glu Glu Ser Gln Asn Gln Gln
   1               5                   10                  15
   Glu Lys Asn Glu Gln Glu Leu Leu Glu Leu Asp Lys Trp Ala Ser Leu
               20                  25                  30
   Trp Asn Trp Phe
           35
<210> 18
   <211> 30
   <212> DNA
   <213> Artificial sequence
<220>
   <223> Primer P0038
<400> 18
   ctagagaaaa ggtacactag cttaatacac 30
<210> 19
   <211> 46
   <212> DNA
   <213> Artificial sequence
<220>
   <223> Primer P0039
<400> 19
   tgcgattctt caattaagga gtgtattaag ctagtgtacc ttttct 46
<210> 20
   <211> 40
   <212> DNA
   <213> Artificial sequence
<220>
   <223> Primer P0040
<400> 20
   tccttaattg aagaatcgca aaaccagcaa gaaaagaatg 40
<210> 21
   <211> 40
   <212> DNA
   <213> Artificial sequence
<220>
   <223> Primer P0041
<400> 21
   taattccaat aattcttgtt cattcttttc ttgctggttt 40
<210> 22
   <211> 54
   <212> DNA
   <213> Artificial sequence
<220>
   <223> Primer P0042
<400> 22
   aacaagaatt attggaatta gataaatggg caagtttgtg gaattggttt gtac 54
<210> 23
   <211> 30
   <212> DNA
   <213> Artificial sequence
<220>
   <223> Primer P0043
<400> 23
   aaaccaattc cacaaacttg cccatttatc 30
<210> 24
   <211> 25
   <212> DNA
   <213> Artificial sequence
<220>
   <223> Primer P0044
<400> 24
   tcgaccttac actagcttaa tacac 25
<210> 25
   <211> 41
   <212> DNA
   <213> Artificial sequence
<220>
   <223> Primer P0045
<400> 25
   tgcgattctt caattaagga gtgtattaag ctagtgtaag g 41
<210> 26
   <211> 40
   <212> DNA
   <213> Artificial sequence
<220>
   <223> Primer P0040
<400> 26
   tccttaattg aagaatcgca aaaccagcaa gaaaagaatg 40
<210> 27
   <211> 40
   <212> DNA
   <213> Artificial sequence
<220>
   <223> Primer P0041
<400> 27
   taattccaat aattcttgtt cattcttttc ttgctggttt 40
<210> 28
   <211> 55
   <212> DNA
   <213> Artificial sequence
<220>
   <223> Primer P0046
<400> 28
   aacaagaatt attggaatta gataaatggg caagtttgtg gaattggttt taata 55
<210> 29
   <211> 39
   <212> DNA
   <213> Artificial sequence
<220>
   <223> Primer P0047
<400> 29
   agcttattaa aaccaattcc acaaacttgc ccatttatc 39
<210> 30
   <211> 175
   <212> DNA
   <213> Artificial sequence
<220>
   <223> pREX0032 insert
<400> 30
   caaggctgtt ggtaacctga gaaaatgctc cacctcatca ctcctggaag cctgcacttt 60
   ctacactagc ttaatacact ccttaattga agaatcgcaa aaccagcaag aaaagaatga 120
   acaagaatta ttggaattag ataaatgggc aagtttgtgg aattggtttt aataa 175
<210> 31
   <211> 184
   <212> DNA
   <213> Artificial sequence
<220>
   <223> pREX0017 insert
<400> 31
   caaggctgtt ggtaacctga gaaaatgctc cacctcatca ctcctggaag cctgcacttt 60
   ccgtcgacct tacactagct taatacactc cttaattgaa gaatcgcaaa accagcaaga 120
   aaagaatgaa caagaattat tggaattaga taaatgggca agtttgtgga attggtttta 180
   ataa 184
<210> 32
   <211> 50
   <212> DNA
   <213> Artificial sequence
<220>
   <223> Primer P0060
<400> 32
   tcatcactcc tggaagcctg cactttctac actagcttaa tacactcctt 50
<210> 33
   <211> 50
   <212> DNA
   <213> Artificial sequence
<220>
   <223> Primer P0061
<400> 33
   aaggagtgta ttaagctagt gtagaaagtg caggcttcca ggagtgatga 50
<210> 34
   <211> 41
   <212> DNA
   <213> Artificial sequence
<220>
   <223> Primer P0064
<400> 34
   ttgtctacat agcgggcaag ggtggtctgg tgcctgtctt g 41
<210> 35
   <211> 41
   <212> DNA
   <213> Artificial sequence
<220>
   <223> Primer P0065
<400> 35
   caagacaggc accagaccac ccttgcccgc tatgtagaca a 41
<210> 36
   <211> 45
   <212> DNA
   <213> Artificial sequence
<220>
   <223> Primer P0068
<400> 36
   tccacctcat cactcctgga agccggtact ttccgtcgac cttaa 45
<210> 37
   <211> 48
   <212> DNA
   <213> Artificial sequence
<220>
   <223> Primer P0069
<400> 37
   cttattaagg tcgacggaaa gtaccggctt ccaggagtga tgaggtgg 48
<210> 38
   <211> 50
   <212> DNA
   <213> Artificial sequence
<220>
   <223> pREX0017 plasmid starting at 1501
<400> 38
   tagcgggcaa gtgtggtctg gtgcctgtct tggcagaaaa ctacaataag 50
<210> 39
   <211> 50
   <212> DNA
   <213> Artificial sequence
<220>
   <223> pREX0034 plasmid starting at 1501
<400> 39
   tagcgggcaa gggtggtctg gtgcctgtct tggcagaaaa ctacaataag 50
<210> 40
   <211> 50
   <212> DNA
   <213> Artificial sequence
<220>
   <223> pREX0017 plasmid starting at 2301
<400> 40
   tgctccacct catcactcct ggaagcctgc actttccgtc gaccttacac 50
<210> 41
   <211> 50
   <212> DNA
   <213> Artificial sequence
<220>
   <223> pREX0034 plasmid starting at 2301
<400> 41
   tgctccacct catcactcct ggaagccggt actttccgtc gaccttacac 50
<210> 42
   <211> 48
   <212> DNA
   <213> Artificial sequence
<220>
   <223> Primer P0066
<400> 42
   tccacctcat cactcctgga agccggcact ttctacacta gcttaata 48
<210> 43
   <211> 48
   <212> DNA
   <213> Artificial sequence
<220>
   <223> Primer P0067
<400> 43
   gtgtattaag ctagtgtaga aagtaccggc ttccaggagt gatgaggt 48
<210> 44
   <211> 41
   <212> DNA
   <213> Artificial sequence
<220>
   <223> pREX0033 plasmid starting at 2301
<400> 44
   tgctccacct catcactcct ggaagccggt actttctaca c 41
<210> 45
   <211> 60
   <212> DNA
   <213> Artificial sequence
<220>
   <223> synthetic oligonucleotide encoding peptide with EPO activity
<400> 45
   ggtggtactt actcttgtca ttttggtcca ttgacttggg tttgtaagcc acaaggtggt 60
<210> 46
   <211> 140
   <212> DNA
   <213> Artificial sequence
<220>
   <223> His289-Gly290 insert PCR product
<400> 46
   agacaaatca aaagaatttc aactattcag ctctcctcat ggtggtactt actcttgtca 60
   ttttggtcca ttgacttggg tttgtaagcc acaaggtggt gggaaggacc tgctgtttaa 120
   ggactctgcc cacgggtttt 140
<210> 47
   <211> 210
   <212> DNA
   <213> Artificial sequence
<220>
   <223> Glu625-Thr626 insert PCR product
<400> 47
   cctatttgga agcaacgtaa ctgactgctc gggcaacttt tgtttgttcc ggtcggaagg 60
   tggtacttac tcttgtcatt ttggtccatt gacttgggtt tgtaagccac aaggtggtac 120
   caaggacctt ctgttcagag atgacacagt atgtttggcc aaacttcatg acagaaacac 180
   atatgaaaaa tacttaggag aagaatatgt 210
<210> 48
   <211> 30
   <212> PRT
   <213> Artificial sequence
<220>
   <223> glucagon-like peptide-1
<400> 48
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg
               20                  25                  30
<210> 49
   <211> 90
   <212> DNA
   <213> Artificial sequence
<220>
   <223> sequence encoding glucagon-like peptide-1
<400> 49
   catgctgaag gtacttttac ttctgatgtt tcttcttatt tggaaggtca agctgctaaa    60
   gaatttattg cttggttggt taaaggtaga                                     90
<210> 50
   <211> 118
   <212> DNA
   <213> Artificial sequence
<220>
   <223> Top Strand Synthetic Oligonucleotide
<220>
   <221> misc_feature
   <222> (7) .. (112)
   <223> Top Strand Primer P0056
<400> 50
   aggtctctag agaaaaggca tgctgaaggt acttttactt ctgatgtttc ttcttatttg    60
   gaaggtcaag ctgctaaaga atttattgct tggttggtta aaggtagggt acctgata     118
<210> 51
   <211> 118
   <212> DNA
   <213> Artificial sequence
<220>
   <223> Bottom Strand Synthetic Oligonucleotide
<220>
   <221> misc_feature
   <222> (9) .. (108)
   <223> Bottom Strand Primer P0057
<400> 51
   tatcaggtac cctaccttta accaaccaag caataaattc tttagcagct tgaccttcca 60
   aataagaaga aacatcagaa gtaaaagtac cttcagcatg ccttttctct agagacct 118
<210> 52
   <211> 6
   <212> PRT
   <213> Artificial sequence
<220>
   <223> N-terminal flanking peptide encoded by pREX00052
<400> 52
   Arg Ser Leu Glu Lys Arg
   1 5
<210> 53
   <211> 21
   <212> DNA
   <213> Artificial sequence
<220>
   <223> Primer P0070
<400> 53
   gctatgacca acaagtgtct c 21
<210> 54
   <211> 22
   <212> DNA
   <213> Artificial sequence
<220>
   <223> Primer P0071
<400> 54
   cgcacctgtg gcgccggtga tg 22
<210> 55
   <211> 60
   <212> DNA
   <213> Artificial sequence
<220>
   <223> Sequence for fusion of IFN Beta-1 to mTf
<220>
   <221> misc_feature
   <222> (18) .. (48)
   <223> Primer P0082 sequence
<400>. 55
   ctgcttactc taggtctcta gagaaaacag ggtacctccg aaacgtacct gataaaactg 60
<210> 56
   <211> 60
   <212> DNA
   <213> Artificial sequence
<220>
   <223> Sequence for fusion of IFN Beta-1 to mTf
<220>
   <221> misc_feature
   <222> (17) .. (39)
   <223> Primer P0083 sequence
<400> 56
   cagttttatc aggtacgttt cggaggtacc ctgttttctc tagagaccta gagtaagcag 60
<210> 57
   <211> 8
   <212> PRT
   <213> Artificial sequence
<220>
   <223> MFa-1 sequence
<400> 57
   Ala Tyr Ser Arg Ser Leu Glu Lys
   1 5
<210> 58
   <211> 6
   <212> PRT
   <213> Artificial sequence
<220>
   <223> IFN-B-1 sequence
<400> 58
   Thr Gly Tyr Leu Arg Asn
   1 5
<210> 59
   <211> 5
   <212> PRT
   <213> Artificial sequence
<220>
   <223> mTf sequence
<400> 59
   Val Pro Asp Lys Thr
   1               5
<210> 60
   <211> 43
   <212> DNA
   <213> Artificial sequence
<220>
   <223> Primer P0084
<400> 60
   ctctaggtct ctagagaaaa ggagctacaa cttgcttgga ttc 43
<210> 61
   <211> 25
   <212> DNA
   <213> Artificial sequence
<220>
   <223> Primer P0085
<400> 61
   gtctgaatgt cccatggagg ctttg 25
<210> 62
   <211> 36
   <212> DNA
   <213> Artificial sequence
<220>
   <223> Primer P0086
<400> 62
   actttccgtc gacctagcta caacttgctt ggattc 36
<210> 63
   <211> 50
   <212> DNA
   <213> Artificial sequence
<220>
   <223> Primer P0087
<400> 63
   tgaatgtcca atggaggctt tgattatttc gaattaagaa tactaaatac 50
<210> 64
   <211> 31
   <212> PRT
   <213> Artificial sequence
<220> i
   <223> GLP-1(7-37) amino acid sequence
<400> 64
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg Gly
               20                  25                  30
<210> 65
   <211> 757
   <212> DNA
   <213> Homo sapiens
<220>
   <221> CDS
   <222> (1)..(564)
   <223> GenBank No. NM_002176, interferon-beta 1
<210> 66
   <211> 187
   <212> PRT
   <213> Homo sapiens
<400> 66
   Met Thr Asn Lys Cys Leu Leu Gln Ile Ala Leu Leu Leu Cys Phe Ser
   1               5                   10                  15
   Thr Thr Ala Leu Ser Met Ser Tyr Asn Leu Leu Gly Phe Leu Gln Arg
               20                  25                  30
   Ser Ser Asn Phe Gln Cys Gln Lys Leu Leu Trp Gln Leu Asn Gly Arg
           35                  40                  45
   Leu Glu Tyr Cys Leu Lys Asp Arg Met Asn Phe Asp Ile Pro Glu Glu
       50                  55                  60
   Ile Lys Gln Leu Gln Gln Phe Gln Lys Glu Asp Ala Ala Leu Thr Ile
   65                  70                  75                  80
   Tyr Glu Met Leu Gln Asn Ile Phe Ala Ile Phe Arg Gln Asp Ser Ser
                   85                  90                  95
   Ser Thr Gly Trp Asn Glu Thr Ile Val Glu Asn Leu Leu Ala Asn Val
               100                 105                 110
   Tyr His Gln Ile Asn His Leu Lys Thr Val Leu Glu Glu Lys Leu Glu
           115                 120                 125
   Lys Glu Asp Phe Thr Arg Gly Lys Leu Met Ser Ser Leu His Leu Lys
       130                 135                 140
   Arg Tyr Tyr Gly Arg Ile Leu His Tyr Leu Lys Ala Lys Glu Tyr Ser
   145                 150                 155                 160
   His Cys Ala Trp Thr Ile Val Arg Val Glu Ile Leu Arg Asn Phe Tyr
                   165                 170                 175
   Phe Ile Asn Arg Leu Thr Gly Tyr Leu Arg Asn
               180                 185
<210> 67
   <211> 28
   <212> DNA
   <213> Artificial Sequence
<220>
   <223> Description of Artificial Sequence: XbaI/KpnI region of pREX0052
<400> 67
   aggtctctag agaagagggt acctgata 28
<210> 68
   <211> 30
   <212> DNA
   <213> Artificial Sequence
<220>
   <223> Description of Artificial Sequence: SalI/HindIII region of pREX0052
<400> 68
   actttccgtc gaccttaata agcttaattc 30
<210> 69
   <211> 9
   <212> PRT
   <213> Artificial Sequence
<220>
   <223> Description of Artificial Sequence: Amino acid sequence encoded by XbaI/KpnI region of pREX0052
<400> 69
   Arg Ser Leu Glu Lys Arg Val Pro Asp
   1               5
<210> 70
   <211> 5
   <212> PRT
   <213> Artificial Sequence
<220>
   <223> Description of Artificial Sequence: Amino acid sequence encoded by SalI/HindIII region of pREX0052
<400> 70
   Thr Phe Arg Arg Pro
   1               5
<210> 71
   <211> 450
   <212> DNA
   <213> Homo sapiens
<220>
   <221> CDS
   <222> (45) .. (377)
   <223> GenBank Accession No. NM_000207, human insulin
<400> 71
gctgcatcag aagaggccat caagcacatc actgtccttc tgcc atg gcc ctg tgg   56
   atg cgc ctc ctg ccc ctg ctg gcg ctg ctg gcc ctc tgg gga cct gac   104
   cca gcc gca gcc ttt gtg aac caa cac ctg tgc ggc tca cac ctg gtg   152
   gaa gct ctc tac cta gtg tgc ggg gaa cga ggc ttc ttc tac aca ccc   200
   aag acc cgc cgg gag gca gag gac ctg cag gtg ggg cag gtg gag ctg   248
   ggc ggg ggc cct ggt gca ggc agc ctg cag ccc ttg gcc ctg gag ggg 296
   tcc ctg cag aag cgt ggc att gtg gaa caa tgc tgt acc agc atc tgc 344
   tcc ctc tac cag ctg gag aac tac tgc aac tag acgcagcccg caggcagccc 397
   cccacccgcc gcctcctgca ccgagagaga tggaataaag cccttgaacc agc 450
<210> 72
   <211> 110
   <212> PRT
   <213> Homo sapiens
<400> 72
<210> 73
   <211> 24
   <212> DNA
   <213> Artificial Sequence
<220>
   <223> Description of Artificial Sequence: 5' primer for cloning insulin sequence
<400> 73
   tttgtgaacc aacacctgtg cggc 24
<210> 74
   <211> 34
   <212> DNA
   <213> Artificial Sequence
<220>
   <223> Description of Artificial Sequence: 3' primer for cloning insulin sequence
<400> 74
   gttgcagtag ttctccagct ggtagaggga gcag 34
<210> 75
   <211> 289
   <212> DNA
   <213> Artificial Sequence
<220>
   <223> Description of Artificial Sequence: Plasmid pREX0052 N-insulin
<220>
   <221> CDS
   <222> (1)..(288)
<400> 75
   gct tac tct agg tct cta gat aag agg ttt gtg aac caa cac ctg tgc    48
   Ala Tyr Ser Arg Ser Leu Asp Lys Arg Phe Val Asn Gln His Leu Cys
    1               5                  10                  15
   ggc tca cac ctg gtg gaa gct ctc tac cta gtg tgc ggg gaa cga ggc    96
   Gly Ser His Leu Val Glu Ala Leu Tyr Leu Val Cys Gly Glu Arg Gly
               20                  25                  30
   ttc ttc tac aca ccc aag acc cgc cgg gag gca gag gac ctg cag gtg   144
   Phe Phe Tyr Thr Pro Lys Thr Arg Arg Glu Ala Glu Asp Leu Gln Val
           35                  40                  45
   ggg cag gtg gag ctg ggc ggg ggc cct ggt gca ggc agc ctg cag ccc   192
   Gly Gln Val Glu Leu Gly Gly Gly Pro Gly Ala Gly Ser Leu Gln Pro
       50                  55                  60
   ttg gcc ctg gag ggg tcc ctg cag aag cgt ggc att gtg gaa caa tgc   240
   Leu Ala Leu Glu Gly Ser Leu Gln Lys Arg Gly Ile Val Glu Gln Cys
   65                  70                  75                  80
   tgt acc agc atc tgc tcc ctc tac cag ctg gag aac tac tgc aac gta c 289
   Cys Thr Ser Ile Cys Ser Leu Tyr Gln Leu Glu Asn Tyr Cys Asn Val
                   85                  90                  95
<210> 76
   <211> 96
   <212> PRT
   <213> Artificial Sequence
<220>
   <223> Description of Artificial Sequence: Plasmid pREX0052 N-insulin
<400> 76
   Ala Tyr Ser Arg Ser Leu Asp Lys Arg Phe Val Asn Gln His Leu Cys
    1               5                  10                  15
   Gly Ser His Leu Val Glu Ala Leu Tyr Leu Val Cys Gly Glu Arg Gly
   20 25 30
   Phe Phe Tyr Thr Pro Lys Thr Arg Arg Glu Ala Glu Asp Leu Gln Val
   35 40 45
   Gly Gln Val Glu Leu Gly Gly Gly Pro Gly Ala Gly Ser Leu Gln Pro
   50 55 60
   Leu Ala Leu Glu Gly Ser Leu Gln Lys Arg Gly Ile Val Glu Gln Cys
   65 70 75 80
   Cys Thr Ser Ile Cys Ser Leu Tyr Gln Leu Glu Asn Tyr Cys Asn Val
   85 90 95
<210> 77
   <211> 49
   <212> DNA
   <213> Artificial Sequence
<220>
   <223> Description of Artificial Sequence: 5' insulin cloning primer
<400> 77
   gcttactcta ggtctctaga taagaggttt gtgaaccaac acctgtgcg 49
<210> 78
   <211> 22
   <212> DNA
   <213> Artificial Sequence
<220>
   <223> Description of Artificial Sequence: Linker for cloning insulin
<400> 78
   ctggagaact actgcaacgt ac 22
<210> 79
   <211> 18
   <212> DNA
   <213> Artificial Sequence
<220>
   <223> Description of Artificial Sequence: Linker for cloning insulin
<400> 79
   gttgcagtag ttctccag 18
<210> 80
   <211> 290
   <212> DNA
   <213> Artificial Sequence
<220>
   <223> Description of Artificial Sequence: Plasmid pREX0052 C-insulin
<220>
   <221> CDS
   <222> (1)..(276)
<400> 80
   tgc act ttc cgt cga cct ttt gtg aac caa cac ctg tgc ggc tca cac    48
   Cys Thr Phe Arg Arg Pro Phe Val Asn Gln His Leu Cys Gly Ser His
    1               5                  10                  15
   ctg gtg gaa gct ctc tac cta gtg tgc ggg gaa cga ggc ttc ttc tac    96
   Leu Val Glu Ala Leu Tyr Leu Val Cys Gly Glu Arg Gly Phe Phe Tyr
               20                  25                  30
   aca ccc aag acc cgc cgg gag gca gag gac ctg cag gtg ggg cag gtg   144
   Thr Pro Lys Thr Arg Arg Glu Ala Glu Asp Leu Gln Val Gly Gln Val
           35                  40                  45
   gag ctg ggc ggg ggc cct ggt gca ggc agc ctg cag ccc ttg gcc ctg   192
   Glu Leu Gly Gly Gly Pro Gly Ala Gly Ser Leu Gln Pro Leu Ala Leu
       50                  55                  60
   gag ggg tcc ctg cag aag cgt ggc att gtg gaa caa tgc tgt acc agc   240
   Glu Gly Ser Leu Gln Lys Arg Gly Ile Val Glu Gln Cys Cys Thr Ser
   65                  70                  75                  80
   atc tgc tcc ctc tac cag ctg gag aac tac tgc aac taataagctt aatt   290
   Ile Cys Ser Leu Tyr Gln Leu Glu Asn Tyr Cys Asn
                   85                  90
<210> 81
   <211> 92
   <212> PRT
   <213> Artificial Sequence
<220>
   <223> Description of Artificial Sequence: Plasmid pREX0052 C-insulin
<400> 81
   Cys Thr Phe Arg Arg Pro Phe Val Asn Gln His Leu Cys Gly Ser His
    1               5                  10                  15
   Leu Val Glu Ala Leu Tyr Leu Val Cys Gly Glu Arg Gly Phe Phe Tyr
               20                  25                  30
   Thr Pro Lys Thr Arg Arg Glu Ala Glu Asp Leu Gln Val Gly Gln Val
           35                  40                  45
   Glu Leu Gly Gly Gly Pro Gly Ala Gly Ser Leu Gln Pro Leu Ala Leu
       50                  55                  60
   Glu Gly Ser Leu Gln Lys Arg Gly Ile Val Glu Gln Cys Cys Thr Ser
   65                  70                  75                  80
   Ile Cys Ser Leu Tyr Gln Leu Glu Asn Tyr Cys Asn
                   85                  90
<210> 82
   <211> 40
   <212> DNA
   <213> Artificial Sequence
<220>
   <223> Description of Artificial Sequence: 5' insulin cloning primer
<400> 82
   tgcactttcc gtcgaccttt tgtgaaccaa cacctgtgcg 40
<210> 83
   <211> 32
   <212> DNA
   <213> Artificial Sequence
<220>
   <223> Description of Artificial Sequence: 3' insulin cloning primer
<400> 83
   aattaagctt attagttgca gtagttctcc ag 32
<210> 84
   <211> 676
   <212> PRT
   <213> Oryctolagus cuniculus
<400> 84
   Val Thr Glu Lys Thr Val Arg Trp Cys Ala Val Asn Asp His Glu Ala
   1               5                   10                  15
   Ser Lys Cys Ala Asn Phe Arg Asp Ser Met Lys Lys Val Leu Pro Glu
               20                  25                  30
   Asp Gly Pro Arg Ile Ile Cys Val Lys Lys Ala Ser Tyr Leu Asp Cys
           35                  40                  45
   Ile Lys Ala Ile Ala Ala His Glu Ala Asp Ala Val Thr Leu Asp Ala
       50                  55                  60
   Gly Leu Val His Glu Ala Gly Leu Thr Pro Asn Asn Leu Lys Pro Val
   65                  70                  75                  80
   Val Ala Glu Phe Tyr Gly Ser Lys Glu Asn Pro Lys Thr Phe Tyr Tyr
                   85                  90                  95
   Ala Val Ala Leu Val Lys Lys Gly Ser Asn Phe Gln Leu Asn Glu Leu
               100                 105                 110
   Gln Gly Lys Lys Ser Cys His Thr Gly Leu Gly Arg Ser Ala Gly Trp
           115                 120                 125
   Asn Ile Pro Ile Gly Leu Leu Tyr Cys Asp Leu Pro Glu Pro Arg Lys
       130                 135                 140
   Pro Leu Glu Lys Ala Val Ala Ser Phe Phe Ser Gly Ser Cys Val Pro
   145 150 155 160
   Cys Ala Asp Gly Ala Asp Phe Pro Gln Leu Cys Gln Leu Cys Pro Gly
   165 170 175
   Cys Gly Cys Ser Ser Val Gln Pro Tyr Phe Gly Tyr Ser Gly Ala Phe
   180 185 190
   Lys Cys Leu Lys Asp Gly Leu Gly Asp Val Ala Phe Val Lys Gln Glu
   195 200 205
   Thr Ile Phe Glu Asn Leu Pro Ser Lys Asp Glu Arg Asp Gln Tyr Glu
   210 215 220
   Leu Leu Cys Leu Asp Asn Thr Arg Lys Pro Val Asp Glu Tyr Glu Gln
   225 230 235 240
   Cys His Leu Ala Arg Val Pro Ser His Ala Val Val Ala Arg Ser Val
   245 250 255
   Asp Gly Lys Glu Asp Leu Ile Trp Glu Leu Leu Asn Gln Ala Gln Glu
   260 265 270
   His Phe Gly Lys Asp Lys Ser Gly Asp Phe Gln Leu Phe Ser Ser Pro
   275 280 285
   His Gly Lys Asn Leu Leu Phe Lys Asp Ser Ala Tyr Gly Phe Phe Lys
   290 295 300
   Val Pro Pro Arg Met Asp Ala Asn Leu Tyr Leu Gly Tyr Glu Tyr Val
   305 310 315 320
   Thr Ala Val Arg Asn Leu Arg Glu Gly Ile Cys Pro Asp Pro Leu Gln
   325 330 335
   Asp Glu Cys Lys Ala Val Lys Trp Cys Ala Leu Ser His His Glu Arg
   340 345 350
   Leu Lys Cys Asp Glu Trp Ser Val Thr Ser Gly Gly Leu Ile Glu Cys
   355 360 365
   Glu Ser Ala Glu Thr Pro Glu Asp Cys Ile Ala Lys Ile Met Asn Gly
   370 375 380
   Glu Ala Asp Ala Met Ser Leu Asp Gly Gly Tyr Val Tyr Ile Ala Gly
   385 390 395 400
   Gln Cys Gly Leu Val Pro Val Leu Ala Glu Asn Tyr Glu Ser Thr Asp
   405 410 415
   Cys Lys Lys Ala Pro Glu Glu Gly Tyr Leu Ser Val Ala Val Val Lys
   420 425 430
   Lys Ser Asn Pro Asp Ile Asn Trp Asn Asn Leu Glu Gly Lys Lys Ser
   435 440 445
   Cys His Thr Ala Val Asp Arg Thr Ala Gly Trp Asn Ile Pro Met Gly
   450 455 460
   Leu Leu Tyr Asn Arg Ile Asn His Cys Arg Phe Asp Glu Phe Phe Arg
   465 470 475 480
   Gln Gly Cys Ala Pro Gly Ser Gln Lys Asn Ser Ser Leu Cys Glu Leu
   485 490 495
   Cys Ile Gly Pro Ser Val Cys Ala Pro Asn Asn Arg Glu Gly Tyr Tyr
   500 505 510
   Gly Tyr Thr Gly Ala Phe Arg Cys Leu Val Glu Lys Gly Asp Val Ala
   515 520 525
   Phe Val Lys Ser Gln Thr Val Leu Gln Asn Thr Gly Gly Arg Asn Ser
   530 535 540
   Glu Pro Trp Ala Lys Asp Leu Lys Glu Glu Asp Phe Glu Leu Leu Cys
   545 550 555 560
   Leu Asp Gly Thr Arg Lys Pro Val Ser Glu Ala His Asn Cys His Leu
   565 570 575
   Ala Lys Ala Pro Asn His Ala Val Val Ser Arg Lys Asp Lys Ala Ala
   580 585 590
   Cys Val Lys Gln Lys Leu Leu Asp Leu Gln Val Glu Tyr Gly Asn Thr
   595 600 605
   Val Ala Asp Cys Ser Ser Lys Phe Cys Met Phe His Ser Lys Thr Lys
   610 615 620
   Asp Leu Leu Phe Arg Asp Asp Thr Lys Cys Leu Val Asp Leu Arg Gly
   625 630 635 640
   Lys Asn Thr Tyr Glu Lys Tyr Leu Gly Ala Asp Tyr Ile Lys Ala Val
   645 650 655
   Ser Asn Leu Arg Lys Cys Ser Thr Ser Arg Leu Leu Glu Ala Cys Thr
   660 665 670
   Phe His Lys His
   675
<210> 85
   <211> 676
   <212> PRT
   <213> Rattus norvegicus
<400> 85
   Val Pro Asp Lys Thr Val Lys Trp Cys Ala Val Ser Glu His Glu Asn
   1 5 10 15
   Thr Lys Cys Ile Ser Phe Arg Asp His Met Lys Thr Val Leu Pro Ala
   20 25 30
   Asp Gly Pro Arg Leu Pro Cys Val Lys Lys Thr Ser Tyr Gln Asp Cys
   35 40 45
   Ile Lys Ala Ile Ser Gly Gly Glu Ala Asp Ala Ile Thr Leu Asp Gly
   50 55 60
   Gly Trp Val Tyr Asp Ala Gly Leu Thr Pro Asn Asn Leu Lys Pro Val
   65 70 75 80
   Ala Ala Glu Phe Tyr Gly Ser Leu Glu His Arg Gln Thr His Tyr Leu
   85 90 95
   Ala Val Ala Val Val Lys Lys Gly Thr Asp Phe Gln Leu Asn Gln Leu
   100 105 110
   Gln Gly Lys Lys Ser Cys His Thr Gly Leu Gly Arg Ser Ala Gly Trp
   115 120 125
   Ile Ile Pro Ile Gly Leu Leu Phe Cys Asn Leu Pro Glu Pro Arg Lys
   130 135 140
   Pro Leu Glu Lys Ala Val Ala Ser Phe Phe Ser Gly Ser Cys Val Pro
   145 150 155 160
   Cys Ala Asp Pro Val Ala Phe Pro Gln Leu Cys Gln Leu Cys Pro Gly
   165 170 175
   Cys Gly Cys Ser Pro Thr Gln Pro Phe Phe Gly Tyr Val Gly Ala Phe
   180 185 190
   Lys Cys Leu Arg Asp Gly Gly Gly Asp Val Ala Phe Val Lys His Thr
   195 200 205
   Thr Ile Phe Glu Val Leu Pro Gln Lys Ala Asp Arg Asp Gln Tyr Glu
   210 215 220
   Leu Leu Cys Leu Asp Asn Thr Arg Lys Pro Val Asp Gln Tyr Glu Asp
   225 230 235 240
   Cys Tyr Leu Ala Arg Ile Pro Ser His Ala Val Val Ala Arg Asn Gly
   245 250 255
   Asp Gly Lys Glu Asp Leu Ile Trp Glu Ile Leu Lys Val Ala Gln Glu
   260 265 270
   His Phe Gly Lys Gly Lys Ser Lys Asp Phe Gln Leu Phe Gly Ser Pro
   275 280 285
   Leu Gly Lys Asp Leu Leu Phe Lys Asp Ser Arg Phe Gly Leu Leu Arg
   290 295 300
   Ala Pro Lys Asp Gly Leu Gln Ala Val Pro Arg Pro Gln Leu Cys His
   305 310 315 320
   Cys His Ser Lys Ser Ala Gly Ser Cys Pro Asp Ala Ile Asp Ser Ala
   325 330 335
   Pro Val Lys Trp Cys Ala Leu Ser His Gln Glu Arg Ala Lys Cys Asp
   340 345 350
   Glu Trp Ser Val Thr Gly Asn Gly Gln Ile Glu Cys Glu Ser Ala Glu
   355 360 365
   Ser Thr Glu Asp Cys Ile Asp Lys Ile Val Asn Gly Glu Ala Asp Ala
   370 375 380
   Met Ser Leu Asp Gly Gly His Ala Tyr Ile Ala Gly Gln Cys Gly Leu
   385 390 395 400
   Val Pro Val Met Ala Glu Asn Tyr Asp Ile Ser Ser Cys Thr Asn Pro
   405 410 415
   Gln Ser Asp Val Phe Pro Lys Gly Tyr Tyr Ala Val Ala Val Val Lys
   420 425 430
   Ala Ser Asp Ser Ser Ile Asn Trp Asn Asn Leu Lys Gly Lys Lys Ser
   435 440 445
   Cys His Thr Gly Val Asp Arg Thr Ala Gly Trp Asn Ile Pro Met Gly
   450 455 460
   Leu Leu Phe Ser Arg Ile Asn His Cys Lys Phe Asp Glu Phe Phe Ser
   465 470 475 480
   Gln Gly Cys Ala Pro Gly Tyr Lys Lys Asn Ser Thr Leu Cys Asp Leu
   485 490 495
   Cys Ile Gly Pro Ala Lys Cys Ala Pro Asn Asn Arg Glu Gly Tyr Asn
   500 505 510
   Gly Tyr Thr Gly Ala Phe Gln Cys Leu Val Glu Lys Gly Asp Val Ala
   515 520 525
   Phe Val Lys His Gln Thr Val Leu Glu Asn Thr Asn Gly Lys Asn Thr
   530 535 540
   Ala Ala Trp Ala Lys Asp Leu Lys Gln Glu Asp Phe Gln Leu Leu Cys
   545 550 555 560
   Pro Asp Gly Thr Lys Lys Pro Val Thr Glu Phe Ala Thr Cys His Leu
   565 570 575
   Ala Gln Ala Pro Asn His Val Val Val Ser Arg Lys Glu Lys Ala Ala
   580 585 590
   Arg Val Ser Thr Val Leu Thr Ala Gln Lys Asp Leu Phe Trp Lys Gly
   595 600 605
   Asp Lys Asp Cys Thr Gly Asn Phe Cys Leu Phe Arg Ser Ser Thr Lys
   610 615 620
   Asp Leu Leu Phe Arg Asp Asp Thr Lys Cys Leu Thr Lys Leu Pro Glu
   625 630 635 640
   Gly Thr Thr Tyr Glu Glu Tyr Leu Gly Ala Glu Tyr Leu Gln Ala Val
   645 650 655
   Gly Asn Ile Arg Lys Cys Ser Thr Ser Arg Leu Leu Glu Ala Cys Thr
   660 665 670
   Phe His Lys Ser
   675
<210> 86
   <211> 677
   <212> PRT
   <213> Mus musculus
<400> 86
   Val Pro Asp Lys Thr Val Lys Trp Cys Ala Val Ser Glu His Glu Asn
   1 5 10 15
   Thr Lys Cys Ile Ser Phe Arg Asp His Met Lys Thr Val Leu Pro Pro
   20 25 30
   Asp Gly Pro Arg Leu Ala Cys Val Lys Lys Thr Ser Tyr Pro Asp Cys
   35 40 45
   Ile Lys Ala Ile Ser Ala Ser Glu Ala Asp Ala Met Thr Leu Asp Gly
   50 55 60
   Gly Trp Val Tyr Asp Ala Gly Leu Thr Pro Asn Asn Leu Lys Pro Val
   65 70 75 80
   Ala Ala Glu Phe Tyr Gly Ser Val Glu His Pro Gln Thr Tyr Tyr Tyr
   85 90 95
   Ala Val Ala Val Val Lys Lys Gly Thr Asp Phe Gln Leu Asn Gln Leu
   100 105 110
   Glu Gly Lys Lys Ser Cys His Thr Gly Leu Gly Arg Ser Ala Gly Trp
   115 120 125
   Val Ile Pro Ile Gly Leu Leu Phe Cys Lys Leu Ser Glu Pro Arg Ser
   130 135 140
   Pro Leu Glu Lys Ala Val Ser Ser Phe Phe Ser Gly Ser Cys Val Pro
   145 150 155 160
   Cys Ala Asp Pro Val Ala Phe Pro Lys Leu Cys Gln Leu Cys Pro Gly
   165 170 175
   Cys Gly Cys Ser Ser Thr Gln Pro Phe Phe Gly Tyr Val Gly Ala Phe
   180 185 190
   Lys Cys Leu Lys Asp Gly Gly Gly Asp Val Ala Phe Val Lys His Thr
   195 200 205
   Thr Ile Phe Glu Val Leu Pro Glu Lys Ala Asp Arg Asp Gln Tyr Glu
   210 215 220
   Leu Leu Cys Leu Asp Asn Thr Arg Lys Pro Val Asp Gln Tyr Glu Asp
   225 230 235 240
   Cys Tyr Leu Ala Arg Ile Pro Ser His Ala Val Val Ala Arg Lys Asn
   245 250 255
   Asn Gly Lys Glu Asp Leu Ile Trp Glu Ile Leu Lys Val Ala Gln Glu
   260 265 270
   His Phe Gly Lys Gly Lys Ser Lys Asp Phe Gln Leu Phe Ser Ser Pro
   275 280 285
   Leu Gly Lys Asp Leu Leu Phe Lys Asp Ser Ala Phe Gly Leu Leu Arg
   290 295 300
   Val Pro Pro Arg Met Asp Tyr Arg Leu Tyr Leu Gly His Asn Tyr Val
   305 310 315 320
   Thr Ala Ile Arg Asn Gln Gln Glu Gly Val Cys Pro Glu Gly Ser Ile
                   325                 330                 335
   Asp Asn Ser Pro Val Lys Trp Cys Ala Leu Ser His Leu Glu Arg Thr
               340                 345                 350
   Lys Cys Asp Glu Trp Ser Ile Ile Ser Glu Gly Lys Ile Glu Cys Glu
           355                 360                 365
   Ser Ala Glu Thr Thr Glu Asp Cys Ile Glu Lys Ile Val Asn Gly Glu
       370                 375                 380
   Ala Asp Ala Met Thr Leu Asp Gly Gly His Ala Tyr Ile Ala Gly Gln
   385                 390                 395                 400
   Cys Gly Leu Val Pro Val Met Ala Glu Tyr Tyr Glu Ser Ser Asn Cys
                   405                 410                 415
   Ala Ile Pro Ser Gln Gln Gly Ile Phe Pro Lys Gly Tyr Tyr Ala Val
               420                 425                 430
   Ala Val Val Lys Ala Ser Asp Thr Ser Ile Thr Trp Asn Asn Leu Lys
           435                 440                 445
   Gly Lys Lys Ser Cys His Thr Gly Val Asp Arg Thr Ala Gly Trp Asn
       450                 455                 460
   Ile Pro Met Gly Met Leu Tyr Asn Arg Ile Asn His Cys Lys Phe Asp
   465                 470                 475                 480
   Glu Phe Phe Ser Gln Gly Cys Ala Pro Gly Tyr Glu Lys Asn Ser Thr
                   485                 490                 495
   Leu Cys Asp Leu Cys Ile Gly Pro Leu Lys Cys Ala Pro Asn Asn Lys
               500                 505                 510
   Glu Glu Tyr Asn Gly Tyr Thr Gly Ala Phe Arg Cys Leu Val Glu Lys
           515                 520                 525
   Gly Asp Val Ala Phe Val Lys His Gln Thr Val Leu Asp Asn Thr Glu
       530                 535                 540
   Gly Lys Asn Pro Ala Glu Trp Ala Lys Asn Leu Lys Gln Glu Asp Phe
   545                 550                 555                 560
   Glu Leu Leu Cys Pro Asp Gly Thr Arg Lys Pro Val Lys Asp Phe Ala
                   565                 570                 575
   Ser Cys His Leu Ala Gln Ala Pro Asn His Val Val Val Ser Arg Lys
               580                 585                 590
   Glu Lys Ala Ala Arg Val Lys Ala Val Leu Thr Ser Gln Glu Thr Leu
           595                 600                 605
   Phe Gly Gly Ser Asp Cys Thr Gly Asn Phe Cys Leu Phe Lys Ser Thr
       610                 615                 620
   Thr Lys Asp Leu Leu Phe Arg Asp Asp Thr Lys Cys Phe Val Lys Leu
   625                 630                 635                 640
   Pro Glu Gly Thr Thr Pro Glu Lys Tyr Leu Gly Ala Glu Tyr Met Gln
   645 650 655
   Ser Val Gly Asn Met Arg Lys Cys Ser Thr Ser Arg Leu Leu Glu Ala
   660 665 670
   Cys Thr Phe His Lys
   675
<210> 87
   <211> 688
   <212> PRT
   <213> Equus caballus
<400> 87
   Ala Glu Gln Thr Val Arg Trp Cys Thr Val Ser Asn His Glu Val Ser
   1 5 10 15
   Lys Cys Ala Ser Phe Arg Asp Ser Met Lys Ser Ile Val Pro Ala Pro
   20 25 30
   Pro Leu Val Ala Cys Val Lys Arg Thr Ser Tyr Leu Glu Cys Ile Lys
   35 40 45
   Ala Ile Ala Asp Asn Glu Ala Asp Ala Val Thr Leu Asp Ala Gly Leu
   50 55 60
   Val Phe Glu Ala Gly Leu Ser Pro Tyr Asn Leu Lys Pro Val Val Ala
   65 70 75 80
   Glu Phe Tyr Gly Ser Lys Thr Glu Pro Gln Thr His Tyr Tyr Ala Val
   85 90 95
   Ala Val Val Lys Lys Asn Ser Asn Phe Gln Leu Asn Gln Leu Gln Gly
   100 105 110
   Lys Lys Ser Cys His Thr Gly Leu Gly Arg Ser Ala Gly Trp Asn Ile
   115 120 125
   Pro Ile Gly Leu Leu Tyr Trp Gln Leu Pro Glu Pro Arg Glu Ser Leu
   130 135 140
   Gln Lys Ala Val Ser Asn Phe Phe Ala Gly Ser Cys Val Pro Cys Ala
   145 150 155 160
   Asp Arg Thr Ala Val Pro Asn Leu Cys Gln Leu Cys Val Gly Lys Gly
   165 170 175
   Thr Asp Lys Cys Ala Cys Ser Asn His Glu Pro Tyr Phe Gly Tyr Ser
   180 185 190
   Gly Ala Phe Lys Cys Leu Ala Asp Gly Ala Gly Asp Val Ala Phe Val
   195 200 205
   Lys His Ser Thr Val Leu Glu Asn Leu Pro Gln Glu Ala Asp Arg Asp
   210 215 220
   Glu Tyr Gln Leu Leu Cys Arg Asp Asn Thr Arg Lys Ser Val Asp Glu
   225 230 235 240
   Tyr Lys Asp Cys Tyr Leu Ala Ser Ile Pro Ser His Ala Val Val Ala
   245 250 255
   Arg Ser Val Asp Gly Lys Glu Asp Leu Ile Trp Gly Leu Leu Asn Gln
   260 265 270
   Ala Gln Glu His Phe Gly Thr Glu Lys Ser Lys Asp Phe His Leu Phe
   275 280 285
   Ser Ser Pro His Gly Lys Asp Leu Leu Phe Lys Asp Ser Ala Leu Gly
   290 295 300
   Phe Leu Arg Ile Pro Pro Ala Met Asp Thr Trp Leu Tyr Leu Gly Tyr
   305 310 315 320
   Glu Tyr Val Thr Ala Ile Arg Asn Leu Arg Glu Asp Ile Arg Pro Glu
   325 330 335
   Val Pro Lys Asp Glu Cys Lys Lys Val Lys Trp Cys Ala Ile Gly His
   340 345 350
   His Glu Lys Val Lys Cys Asp Glu Trp Ser Val Asn Ser Gly Gly Asn
   355 360 365
   Ile Glu Cys Glu Ser Ala Gln Ser Thr Glu Asp Cys Ile Ala Lys Ile
   370 375 380
   Val Lys Gly Glu Ala Asp Ala Met Ser Leu Asp Gly Gly Phe Ile Tyr
   385 390 395 400
   Ile Ala Gly Lys Cys Gly Leu Val Pro Val Leu Ala Glu Asn Tyr Glu
   405 410 415
   Thr Arg Ser Gly Ser Ala Cys Val Asp Thr Pro Glu Glu Gly Tyr His
   420 425 430
   Ala Val Ala Val Val Lys Ser Ser Ser Asp Pro Asp Leu Thr Trp Asn
   435 440 445
   Ser Leu Lys Gly Lys Lys Ser Cys His Thr Gly Val Asp Arg Thr Ala
   450 455 460
   Gly Trp Asn Ile Pro Met Gly Leu Leu Tyr Ser Glu Ile Lys His Cys
   465 470 475 480
   Glu Phe Asp Lys Phe Phe Arg Glu Gly Cys Ala Pro Gly Tyr Arg Arg
   485 490 495
   Asn Ser Thr Leu Cys Asn Leu Cys Ile Gly Ser Ala Ser Gly Pro Gly
   500 505 510
   Arg Glu Cys Glu Pro Asn Asn His Glu Arg Tyr Tyr Gly Tyr Thr Gly
   515 520 525
   Ala Phe Arg Cys Leu Val Glu Lys Gly Asp Val Ala Phe Val Lys His
   530 535 540
   Gln Thr Val Glu Gln Asn Thr Asp Gly Arg Asn Pro Asp Asp Trp Ala
   545 550 555 560
   Lys Asp Leu Lys Ser Glu Asn Phe Lys Leu Leu Cys Pro Asp Gly Thr
   565 570 575
   Arg Lys Ser Val Thr Glu Phe Lys Ser Cys Tyr Leu Ala Arg Ala Pro
               580                 585                 590
   Asn His Ala Val Val Ser Arg Lys Glu Lys Ala Ala Cys Val Cys Gln
           595                 600                 605
   Glu Leu His Asn Gln Gln Ala Ser Tyr Gly Lys Asn Gly Ser His Cys
       610                 615                 620
   Pro Asp Lys Phe Cys Leu Phe Gln Ser Ala Thr Lys Asp Leu Leu Phe
   625                 630                 635                 640
   Arg Asp Asp Thr Gln Cys Leu Ala Asn Leu Gln Pro Thr Thr Thr Tyr
                   645                 650                 655
   Lys Thr Tyr Leu Gly Glu Lys Tyr Leu Thr Ala Val Ala Asn Leu Arg
               660                 665                 670
   Gln Cys Ser Thr Ser Arg Leu Leu Glu Ala Cys Thr Phe His Arg Val
           675                 680                 685
<210> 88
   <211> 685
   <212> PRT
   <213> Bos taurus
<400> 88
   Asp Pro Glu Arg Thr Val Arg Trp Cys Thr Ile Ser Thr His Glu Ala
   1               5                   10                  15
   Asn Lys Cys Ala Ser Phe Arg Glu Asn Val Leu Arg Ile Leu Glu Ser
               20                  25                  30
   Gly Pro Phe Val Ser Cys Val Lys Lys Thr Ser His Met Asp Cys Ile
           35                  40                  45
   Lys Ala Ile Ser Asn Asn Glu Ala Asp Ala Val Thr Leu Asp Gly Gly
       50                  55                  60
   Leu Val Tyr Glu Ala Gly Leu Lys Pro Asn Asn Leu Lys Pro Val Val
   65                  70                  75                  80
   Ala Glu Phe His Gly Thr Lys Asp Asn Pro Gln Thr His Tyr Tyr Ala
                   85                  90                  95
   Val Ala Val Val Lys Lys Asp Thr Asp Phe Lys Leu Asn Glu Leu Arg
               100                 105                 110
   Gly Lys Lys Ser Cys His Thr Gly Leu Gly Arg Ser Ala Gly Trp Asn
           115                 120                 125
   Ile Pro Met Ala Lys Leu Tyr Lys Glu Leu Pro Asp Pro Gln Glu Ser
       130                 135                 140
   Ile Gln Arg Ala Ala Ala Asn Phe Phe Ser Ala Ser Cys Val Pro Cys
   145                 150                 155                 160
   Ala Asp Gln Ser Ser Phe Pro Lys Leu Cys Gln Leu Cys Ala Gly Lys
                   165                 170                 175
   Gly Thr Asp Lys Cys Ala Cys Ser Asn His Glu Pro Tyr Phe Gly Tyr
               180                 185                 190
   Ser Gly Ala Phe Lys Cys Leu Met Glu Gly Ala Gly Asp Val Ala Phe
           195                 200                 205
   Val Lys His Ser Thr Val Phe Asp Asn Leu Pro Asn Pro Glu Asp Arg
       210                 215                 220
   Lys Asn Tyr Glu Leu Leu Cys Gly Asp Asn Thr Arg Lys Ser Val Asp
   225                 230                 235                 240
   Asp Tyr Gln Glu Cys Tyr Leu Ala Met Val Pro Ser His Ala Val Val
                   245                 250                 255
   Ala Arg Thr Val Gly Gly Lys Glu Asp Val Ile Trp Glu Leu Leu Asn
               260                 265                 270
   His Ala Gln Glu His Phe Gly Lys Asp Lys Pro Asp Asn Phe Gln Leu
           275                 280                 285
   Phe Gln Ser Pro His Gly Lys Asp Leu Leu Phe Lys Asp Ser Ala Asp
       290                 295                 300
   Gly Phe Leu Lys Ile Pro Ser Lys Met Asp Phe Glu Leu Tyr Leu Gly
   305                 310                 315                 320
   Tyr Glu Tyr Val Thr Ala Leu Gln Asn Leu Arg Glu Ser Lys Pro Pro
                   325                 330                 335
   Asp Ser Ser Lys Asp Glu Cys Met Val Lys Trp Cys Ala Ile Gly His
               340                 345                 350
   Gln Glu Arg Thr Lys Cys Asp Arg Trp Ser Gly Phe Ser Gly Gly Ala
           355                 360                 365
   Ile Glu Cys Glu Thr Ala Glu Asn Thr Glu Glu Cys Ile Ala Lys Ile
       370                 375                 380
   Met Lys Gly Glu Ala Asp Ala Met Ser Leu Asp Gly Gly Tyr Leu Tyr
   385                 390                 395                 400
   Ile Ala Gly Lys Cys Gly Leu Val Pro Val Leu Ala Glu Asn Tyr Lys
                   405                 410                 415
   Thr Glu Gly Glu Ser Cys Lys Asn Thr Pro Glu Lys Gly Tyr Leu Ala
               420                 425                 430
   Val Ala Val Val Lys Thr Ser Asp Ala Asn Ile Asn Trp Asn Asn Leu
           435                 440                 445
   Lys Asp Lys Lys Ser Cys His Thr Ala Val Asp Arg Thr Ala Gly Trp
       450                 455                 460
   Asn Ile Pro Met Gly Leu Leu Tyr Ser Lys Ile Asn Asn Cys Lys Phe
   465                 470                 475                 480
   Asp Glu Phe Phe Ser Ala Gly Cys Ala Pro Gly Ser Pro Arg Asn Ser
                   485                 490                 495
   Ser Leu Cys Ala Leu Cys Ile Gly Ser Glu Lys Gly Thr Gly Lys Glu
   500 505 510
   Cys Val Pro Asn Ser Asn Glu Arg Tyr Tyr Gly Tyr Thr Gly Ala Phe
   515 520 525
   Arg Cys Leu Val Glu Lys Gly Asp Val Ala Phe Val Lys Asp Gln Thr
   530 535 540
   Val Ile Gln Asn Thr Asp Gly Asn Asn Asn Glu Ala Trp Ala Lys Asn
   545 550 555 560
   Leu Lys Lys Glu Asn Phe Glu Val Leu Cys Lys Asp Gly Thr Arg Lys
   565 570 575
   Pro Val Thr Asp Ala Glu Asn Cys His Leu Ala Arg Gly Pro Asn His
   580 585 590
   Ala Val Val Ser Arg Lys Asp Lys Ala Thr Cys Val Glu Lys Ile Leu
   595 600 605
   Asn Lys Gln Gln Asp Asp Phe Gly Lys Ser Val Thr Asp Cys Thr Ser
   610 615 620
   Asn Phe Cys Leu Phe Gln Ser Asn Ser Lys Asp Leu Leu Phe Arg Asp
   625 630 635 640
   Asp Thr Lys Cys Leu Ala Ser Ile Ala Lys Lys Thr Tyr Asp Ser Tyr
   645 650 655
   Leu Gly Asp Asp Tyr Val Arg Ala Met Thr Asn Leu Arg Gln Cys Ser
   660 665 670
   Thr Ser Lys Leu Leu Glu Ala Cys Thr Phe His Lys Pro
   675 680 685
<210> 89
   <211> 696
   <212> PRT
   <213> Sus scrofa
<220>
   <221> misc_feature
   <222> (308)..(308)
   <223> Xaa can be any naturally occurring amino acid
<400> 89
   Val Ala Gln Lys Thr Val Arg Trp Cys Thr Ile Ser Asn Gln Glu Ala
   1               5                   10                  15
   Asn Lys Cys Ser Ser Phe Arg Glu Asn Met Ser Lys Ala Val Lys Asn
               20                  25                  30
   Gly Pro Leu Val Ser Cys Val Lys Lys Ser Ser Tyr Leu Asp Cys Ile
           35                  40                  45
   Lys Ala Ile Arg Asp Lys Glu Ala Asp Ala Val Thr Leu Asp Ala Gly
       50                  55                  60
   Leu Val Phe Glu Ala Gly Leu Ala Pro Tyr Asn Leu Lys Pro Val Val
   65 70 75 80
   Ala Glu Phe Tyr Gly Gln Lys Asp Asn Pro Gln Thr His Tyr Tyr Ala
   85 90 95
   Val Ala Val Val Lys Lys Gly Ser Asn Phe Gln Trp Asn Gln Leu Gln
   100 105 110
   Gly Lys Arg Ser Cys His Thr Gly Leu Gly Arg Ser Ala Gly Trp Ile
   115 120 125
   Ile Pro Met Gly Leu Leu Tyr Asp Gln Leu Pro Glu Pro Arg Lys Pro
   130 135 140
   Ile Glu Lys Ala Val Ala Ser Phe Phe Ser Ser Ser Cys Val Pro Cys
   145 150 155 160
   Ala Asp Pro Val Asn Phe Pro Lys Leu Cys Gln Gln Cys Ala Gly Lys
   165 170 175
   Gly Ala Glu Lys Cys Ala Cys Ser Asn His Glu Pro Tyr Phe Gly Tyr
   180 185 190
   Ala Gly Ala Phe Asn Cys Leu Lys Glu Asp Ala Gly Asp Val Ala Phe
   195 200 205
   Val Lys His Ser Thr Val Leu Glu Asn Leu Pro Asp Lys Ala Asp Arg
   210 215 220
   Asp Gln Tyr Glu Leu Leu Cys Arg Asp Asn Thr Arg Arg Pro Val Asp
   225 230 235 240
   Asp Tyr Glu Asn Cys Tyr Leu Ala Gln Val Pro Ser His Ala Val Val
   245 250 255
   Ala Arg Ser Val Asp Gly Gln Glu Asp Ser Ile Trp Glu Leu Leu Asn
   260 265 270
   Gln Ala Gln Glu His Phe Gly Arg Asp Lys Ser Pro Asp Phe Gln Leu
   275 280 285
   Phe Ser Ser Ser His Gly Lys Asp Leu Leu Phe Lys Asp Ser Ala Asn
   290 295 300
   Gly Phe Leu Xaa Ile Pro Ser Lys Met Asp Ser Ser Leu Tyr Leu Gly
   305 310 315 320
   Tyr Gln Tyr Val Thr Ala Leu Arg Asn Leu Arg Glu Glu Ile Ser Pro
   325 330 335
   Asp Ser Ser Lys Asn Glu Cys Lys Lys Val Arg Trp Cys Ala Ile Gly
   340 345 350
   His Glu Glu Thr Gln Lys Cys Asp Ala Trp Ser Ile Asn Ser Gly Gly
   355 360 365
   Lys Ile Glu Cys Val Ser Ala Glu Asn Thr Glu Asp Cys Ile Ala Lys
   370 375 380
   Ile Val Lys Gly Glu Ala Asp Ala Met Ser Leu Asp Gly Gly Tyr Ile
   385 390 395 400
   Tyr Ile Ala Gly Lys Cys Gly Leu Val Pro Val Leu Ala Glu Asn Tyr
   405 410 415
   Lys Thr Glu Gly Glu Asn Cys Val Asn Thr Pro Glu Lys Gly Tyr Leu
   420 425 430
   Ala Val Ala Val Val Lys Lys Ser Ser Gly Pro Asp Leu Asn Trp Asn
   435 440 445
   Asn Leu Lys Gly Lys Lys Ser Cys His Thr Ala Val Asp Arg Thr Ala
   450 455 460
   Gly Trp Asn Ile Pro Met Gly Leu Leu Tyr Asn Lys Ile Asn Ser Cys
   465 470 475 480
   Lys Phe Asp Gln Phe Phe Gly Glu Gly Cys Ala Pro Gly Ser Gln Arg
   485 490 495
   Asn Ser Ser Leu Cys Ala Leu Cys Ile Gly Ser Glu Arg Ala Pro Gly
   500 505 510
   Arg Glu Cys Leu Ala Asn Asn His Glu Arg Tyr Tyr Gly Tyr Thr Gly
   515 520 525
   Ala Phe Arg Cys Leu Val Glu Lys Gly Asp Val Ala Phe Val Lys Asp
   530 535 540
   Gln Val Val Gln Gln Asn Thr Asp Gly Lys Asn Lys Asp Asp Trp Ala
   545 550 555 560
   Lys Asp Leu Lys Gln Met Asp Phe Glu Leu Leu Cys Gln Asn Gly Ala
   565 570 575
   Arg Glu Pro Val Asp Asn Ala Glu Asn Cys His Leu Ala Arg Ala Pro
   580 585 590
   Asn His Ala Val Val Ala Arg Asp Asp Lys Val Thr Cys Val Ala Glu
   595 600 605
   Glu Leu Leu Lys Gln Gln Ala Gln Phe Gly Arg His Val Thr Asp Cys
   610 615 620
   Ser Ser Ser Phe Cys Met Phe Lys Ser Asn Thr Lys Asp Leu Leu Phe
   625 630 635 640
   Arg Asp Asp Thr Gln Cys Leu Ala Arg Val Gly Lys Thr Thr Tyr Glu
   645 650 655
   Ser Tyr Leu Gly Ala Asp Tyr Ile Thr Ala Val Ala Asn Leu Arg Lys
   660 665 670
   Cys Ser Thr Ser Lys Leu Leu Glu Ala Cys Thr Phe His Ser Ala Lys
   675 680 685
   Asn Pro Arg Val Glu Thr Thr Thr
   690 695
<210> 90
   <211> 686
   <212> PRT
   <213> Gallus gallus
<400> 90
   Ala Pro Pro Lys Ser Val Ile Arg Trp Cys Thr Ile Ser Ser Pro Glu
   1 5 10 15
   Glu Lys Lys Cys Asn Asn Leu Arg Asp Leu Thr Gln Gln Glu Arg Ile
   20 25 30
   Ser Leu Thr Cys Val Gln Lys Ala Thr Tyr Leu Asp Cys Ile Lys Ala
   35 40 45
   Ile Ala Asn Asn Glu Ala Asp Ala Ile Ser Leu Asp Gly Gly Gln Ala
   50 55 60
   Phe Glu Ala Gly Leu Ala Pro Tyr Lys Leu Lys Pro Ile Ala Ala Glu
   65 70 75 80
   Val Tyr Glu His Thr Glu Gly Ser Thr Thr Ser Tyr Tyr Ala Val Ala
   85 90 95
   Val Val Lys Lys Gly Thr Glu Phe Thr Val Asn Asp Leu Gln Gly Lys
   100 105 110
   Thr Ser Cys His Thr Gly Leu Gly Arg Ser Ala Gly Trp Asn Ile Pro
   115 120 125
   Ile Gly Thr Leu Leu His Arg Gly Ala Ile Glu Trp Glu Gly Ile Glu
   130 135 140
   Ser Gly Ser Val Glu Gln Ala Val Ala Lys Phe Phe Ser Ala Ser Cys
   145 150 155 160
   Val Pro Gly Ala Thr Ile Glu Gln Lys Leu Cys Arg Gln Cys Lys Gly
   165 170 175
   Asp Pro Lys Thr Lys Cys Ala Arg Asn Ala Pro Tyr Ser Gly Tyr Ser
   180 185 190
   Gly Ala Phe His Cys Leu Lys Asp Gly Lys Gly Asp Val Ala Phe Val
   195 200 205
   Lys His Thr Thr Val Asn Glu Asn Ala Pro Asp Gln Lys Asp Glu Tyr
   210 215 220
   Glu Leu Leu Cys Leu Asp Gly Ser Arg Gln Pro Val Asp Asn Tyr Lys
   225 230 235 240
   Thr Cys Asn Trp Ala Arg Val Ala Ala His Ala Val Val Ala Arg Asp
   245 250 255
   Asp Asn Lys Val Glu Asp Ile Trp Ser Phe Leu Ser Lys Ala Gln Ser
   260 265 270
   Asp Phe Gly Val Asp Thr Lys Ser Asp Phe His Leu Phe Gly Pro Pro
   275 280 285
   Gly Lys Lys Asp Pro Val Leu Lys Asp Leu Leu Phe Lys Asp Ser Ala
   290 295 300
   Ile Met Leu Lys Arg Val Pro Ser Leu Met Asp Ser Gln Leu Tyr Leu
   305                 310                 315                 320
   Gly Phe Glu Tyr Tyr Ser Ala Ile Gln Ser Met Arg Lys Asp Gln Leu
                   325                 330                 335
   Thr Pro Ser Pro Arg Glu Asn Arg Ile Gln Trp Cys Ala Val Gly Lys
               340                 345                 350
   Asp Glu Lys Ser Lys Cys Asp Arg Trp Ser Val Val Ser Asn Gly Asp
           355                 360                 365
   Val Glu Cys Thr Val Val Asp Glu Thr Lys Asp Cys Ile Ile Lys Ile
       370                 375                 380
   Met Lys Gly Glu Ala Asp Ala Val Ala Leu Asp Gly Gly Leu Val Tyr
   385                 390                 395                 400
   Thr Ala Gly Val Cys Gly Leu Val Pro Val Met Ala Glu Arg Tyr Asp
                   405                 410                 415
   Asp Glu Ser Gln Cys Ser Lys Thr Asp Glu Arg Pro Ala Ser Tyr Phe
               420                 425                 430
   Ala Val Ala Val Ala Arg Lys Asp Ser Asn Val Asn Trp Asn Asn Leu
           435                 440                 445
   Lys Gly Lys Lys Ser Cys His Thr Ala Val Gly Arg Thr Ala Gly Trp
       450                 455                 460
   Val Ile Pro Met Gly Leu Ile His Asn Arg Thr Gly Thr Cys Asn Phe
   465                 470                 475                 480
   Asp Glu Tyr Phe Ser Glu Gly Cys Ala Pro Gly Ser Pro Pro Asn Ser
                   485                 490                 495
   Arg Leu Cys Gln Leu Cys Gln Gly Ser Gly Gly Ile Pro Pro Glu Lys
               500                 505                 510
   Cys Val Ala Ser Ser His Glu Lys Tyr Phe Gly Tyr Thr Gly Ala Leu
           515                 520                 525
   Arg Cys Leu Val Glu Lys Gly Asp Val Ala Phe Ile Gln His Ser Thr
       530                 535                 540
   Val Glu Glu Asn Thr Gly Gly Lys Asn Lys Ala Asp Trp Ala Lys Asn
   545                 550                 555                 560
   Leu Gln Met Asp Asp Phe Glu Leu Leu Cys Thr Asp Gly Arg Arg Ala
                   565                 570                 575
   Asn Val Met Asp Tyr Arg Glu Cys Asn Leu Ala Glu Val Pro Thr His
               580                 585                 590
   Ala Val Val Val Arg Pro Glu Lys Ala Asn Lys Ile Arg Asp Leu Leu
           595                 600                 605
   Glu Arg Gln Glu Lys Arg Phe Gly Val Asn Gly Ser Glu Lys Ser Lys
       610                 615                 620
   Phe Met Met Phe Glu Ser Gln Asn Lys Asp Leu Leu Phe Lys Asp Leu
   625                 630                 635                 640
   Thr Lys Cys Leu Phe Lys Val Arg Glu Gly Thr Thr Tyr Lys Glu Phe
                   645                 650                 655
   Leu Gly Asp Lys Phe Tyr Thr Val Ile Ser Ser Leu Lys Thr Cys Asn
               660                 665                 670
   Pro Ser Asp Ile Leu Gln Met Cys Ser Phe Leu Glu Gly Lys
           675                 680                 685